# EUROPEAN PATENT SPECIFICATION

(11) **EP 2 592 945 B1**
(45) Date of publication and mention of the grant of the patent: **17.01.2018**
(21) Application number: 11735748.3
(22) Date of filing: 14.07.2011
(51) Int. Cl.: A23J 3/16, A23L 33/17

(54) **BLENDED SOY PROTEIN PRODUCTS WITH A COMBINED PUFA CONTENT HAVING ALTERED CHARACTERISTICS**
GEMISCHTE SOJAPROTEINPRODUKTE MIT EINEM KOMBINIERTEN PUFA-GEHALT MIT GEÄNDERTEN EIGENSCHAFTEN
PRODUITS PROTÉIQUES MÉLANGÉS DE SOJA AYANT DES CARACTÉRISTIQUES MODIFIÉES

(30) Priority: 14.07.2010 US 364045 P
(43) Date of publication of application: 22.05.2013
(73) Proprietor: E. I. du Pont de Nemours and Company, Wilmington, DE 19805 (US)
(72) Inventor: KERR, Phillip, S., Wildwood Missouri 63005 (US); SHAH, Naina, K., Manchester Missouri 63021 (US); IRWIN, Anthony, J., Shrewsbury Missouri 63119 (US); STAERK, Daniel, U., Kirkwood Missouri 63122 (US); DEAK, Nicolas, University Missouri 63130 (US)
(74) Representative: Dzieglewska, Hanna Eva
(86) International application number: PCT/US2011/044025
(87) International publication number: WO 2012/009548

(56) References cited:
- WO-A2-2009/086047
- US-A- 4 309 344
- US-A1- 2003 104 108
- KISHORE K ET AL: "Isolation and characterization of microsomal omega-6-desaturase gene (fad2-1) from soybean", INDIAN JOURNAL OF EXPERIMENTAL BIOLOGY, vol. 45, no. 4, April 2007 (2007-04), pages 390-397, XP002668469, ISSN: 0019-5189
- DAMUDE HOWARD G ET AL: "Identification of bifunctional Delta 12/omega 3 fatty acid desaturases for improving the ratio of omega 3 to omega 6 fatty acids in microbes and plants", PROCEEDINGS OF THE NATIONAL ACADEMY OF SCIENCES OF USA, NATIONAL ACADEMY OF SCIENCE, WASHINGTON, DC; US, vol. 103, no. 25, 1 June 2006 (2006-06-01) , pages 9446-9451, XP009099270, ISSN: 0027-8424, DOI: 10.1073/PNAS.0511079103
- Salisbury P & Green A: "Breeding for functional foods", 13th Austrialian Research Assembly on Brassica-Conference Proceedings , 2003, XP002668470, Retrieved from the Internet: URL:http://www.australianoilseeds.com/__da ta/assets/pdf_file/0003/4539/Breeding_for_ functional_foods.pdf [retrieved on 2012-01-31] & "2003 Research Papers from 'High Performance Crops for Australia'", , Retrieved from the Internet: URL:http://www.csiro.au/proprietaryDocumen ts/P_pubs_2003.pdf [retrieved on 2012-01-31]
- GUDBRANDSEN ODDRUN A ET AL: "Dietary soya protein concentrate enriched with isoflavones reduced fatty liver, increased hepatic fatty acid oxidation and decreased the hepatic mRNA level of VLDL receptor in obese Zucker rats", BRITISH JOURNAL OF NUTRITION, vol. 96, no. 2, August 2006 (2006-08), pages 249-257, XP002668471, ISSN: 0007-1145
- MATTHAN NIRUPA R ET AL: "Effect of soy protein from differently processed products on cardiovascular disease risk factors and vascular endothelial function in hypercholesterolemic subjects", AMERICAN JOURNAL OF CLINICAL NUTRITION, vol. 85, no. 4, April 2007 (2007-04), pages 960-966, XP002668472, ISSN: 0002-9165

### SEQUENCE LISTING

<110> E. I. du Pont de Nemours and Company
   Kerr,S. Phillip
   Shah, Naina
   Irwin, J. Anthony
   Staerk, Daniel U.
   Deak Nicolas
<120> SOY PROTEIN PRODUCTS HAVING ALTERED CHARACTERISTICS
<130> BB1786 PCT
<150> 60/364045
   <151> 2010-07-14
<160> 26
<170> PatentIn version 3.5
<210> 1
   <211> 7993
   <212> DNA
   <213> Recombinant DNA Fragment PHP21676A
<220>
   <221> misc_feature
   <222> (5182)..(5182)
   <223> n is a, c, g, or t
<400> 1
   cgcgccaagc ttggatccgc gccaagcttg gatcctagaa ctagaaacgt gatgccactt      60
   gttattgaag tcgattacag catctattct gttttactat ttataacttt gccatttctg     120
   acttttgaaa actatctctg gatttcggta tcgctttgtg aagatcgagc aaaagagacg     180
   ttttgtggac gcaatggtcc aaatccgttc tacatgaaca aattggtcac aatttccact     240
   aaaagtaaat aaatggcaag ttaaaaaagg aatatgcatt ttactgattg cctaggtgag     300
   ctccaagaga agttgaatct acacgtctac caaccgctaa aaaaagaaaa acattgatat     360
   gtaacctgat tccattagct tttgacttct tcaacagatt ctctacttag atttctaaca     420
   gaaatattat tactagcaca tcattttcag tctcactaca gcaaaaaatc caacggcaca     480
   atacagacaa caggagatat cagactacag agatagatag atgctactgc atgtagtaag     540
   ttaaataaaa ggaaaataaa atgtcttgct accaaaacta ctacagacta tgatgctcac     600
   cacaggccaa atcctgcaac taggacagca ttatcttata tatattgtac aaaacaagca     660
   tcaaggaaca tttggtctag gcaatcagta cctcgttcta ccatcaccct cagttatcac     720
   atccttgaag gatccattac tgggaatcat cggcaacaca tgctcctgat ggggcacaat     780
   gacatcaaga aggtaggggc caggggtgtc caacattctc tgaattgccg ctctaagctc     840
   ttccttcttc gtcactcgcg ctgccggtat cccacaagca tcagcaaact tgagcatgtt     900
   tgggaatatc tcgctctcgc tagacggatc tccaagatag gtgtgagctc tattggactt     960
   gtagaaccta tcctccaact gaaccaccat acccaaatgc tgattgttca acaacaatat 1020
   cttaactggg agattctcca ctcttatagt ggccaactcc tgaacattca tgatgaaact 1080
   accatcccca tcaatgtcaa ccacaacagc cccagggtta gcaacagcag caccaatagc 1140
   cgcaggcaat ccaaaaccca tggctccaag accccctgag gtcaaccact gcctcggtct 1200
   cttgtacttg taaaactgcg cagcccacat ttgatgctgc ccaaccccag tactaacaat 1260
   agcatctcca ttagtcaact catcaagaac ctcgatagca tgctgcggag aaatcgcgtc 1320
   ctggaatgtc ttgtaaccca atggaaactt gtgtttctgc acattaatct cttctctcca 1380
   acctccaaga tcaaacttac cctccactcc tttctcctcc aaaatcatat taattccctt 1440
   caaggccaac ttcaaatccg cgcaaaccga cacgtgcgcc tgcttgttct tcccaatctc 1500
   ggcagaatca atatcaatgt gaacaatctt agccctacta gcaaaagcct caagcttccc 1560
   agtaacacgg tcatcaaacc ttaccccaaa ggcaagcaac aaatcactat tgtcaacagc 1620
   atagttagca taaacagtac catgcatacc cagcatctga agggaatatt catcaccaat 1680
   aggaaaagtt ccaagaccca ttaaagtgct agcaacggga ataccagtga gttcaacaaa 1740
   gcgcctcaat tcagcactgg aattcaaact gccaccgccg acgtagagaa cgggcttttg 1800
   ggcctccatg atgagtctga caatgtgttc caattgggcc tcggcggggg gcctgggcag 1860
   cctggcgagg taaccgggga ggttaacggg ctcgtcccaa ttaggcacgg cgagttgctg 1920
   ctgaacgtct ttgggaatgt cgatgaggac cggaccgggg cggccggagg tggcgacgaa 1980
   gaaagcctcg gcgacgacgc gggggatgtc gtcgacgtcg aggatgaggt agttgtgctt 2040
   cgtgatggat ctgctcacct ccacgatcgg ggtttcttgg aaggcgtcgg tgccgatcat 2100
   ccggcgggcg acctggccgg tgatggcgac gactgggacg ctgtccatta aagcgtcggc 2160
   gaggccgctc acgaggttgg tggcgccggg gccggaggtg gcaatgcaga cgccggggag 2220
   gccggaggaa cgcgcgtagc cttcggcggc gaagacgccg ccctgctcgt ggcgcgggag 2280
   cacgttgcgg atggcggcgg agcgcgtgag cgcctggtgg atctccatcg acgcaccgcc 2340
   ggggtacgcg aacaccgtcg tcacgccctg cctctccagc gcctccacaa ggatgtccgc 2400
   gcccttgcga ggttcgccgg aggcgaaccg tgacacgaag ggctccgtgg tcggcgcttc 2460
   cttggtgaag ggcgccgccg tggggggttt ggagatggaa catttgattt tgagagcgtg 2520
   gttgggtttg gtgagggttt gatgagagag agggagggtg gatctagtaa tgcgtttggg 2580
   gaaggtgggg tgtgaagagg aagaagagaa tcgggtggtt ctggaagcgg tggccgccat 2640
   tgtgttgtgt ggcatggtta tacttcaaaa actgcacaac aagcctagag ttagtaccta 2700
   aacagtaaat ttacaacaga gagcaaagac acatgcaaaa atttcagcca taaaaaaagt 2760
   tataatagaa tttaaagcaa aagtttcatt ttttaaacat atatacaaac aaactggatt 2820
   tgaaggaagg gattaattcc cctgctcaaa gtttgaattc ctattgtgac ctatactcga 2880
   ataaaattga agcctaagga atgtatgaga aacaagaaaa caaaacaaaa ctacagacaa 2940
   acaagtacaa ttacaaaatt cgctaaaatt ctgtaatcac caaaccccat ctcagtcagc 3000
   acaaggccca aggtttattt tgaaataaaa aaaaagtgat tttatttctc ataagctaaa 3060
   agaaagaaag gcaattatga aatgatttcg actagatctg aaagtccaac gcgtattccg 3120
   cagatattaa agaaagagta gagtttcaca tggatcctag atggacccag ttgaggaaaa 3180
   agcaaggcaa agcaaaccag aagtgcaaga tccgaaattg aaccacggaa tctaggattt 3240
   ggtagaggga gaagaaaagt accttgagag gtagaagaga agagaagagc agagagatat 3300
   atgaacgagt gtgtcttggt ctcaactctg aagcgatacg agtttagagg ggagcattga 3360
   gttccaattt atagggaaac cgggtggcag ggtgagttta atgacggaaa agcccctaag 3420
   taacgagatt ggattgtggg ttagattcaa ccgtttgcat ccgcggctta gattggggaa 3480
   gtcagagtga atctcaaccg ttgactgagt tgaaaattga atgtagcaac caattgagcc 3540
   aaccccagcc tttgcccttt gattttgatt tgtttgttgc atacttttta tttgtcttct 3600
   ggttctgact ctctttctct cgtttcaatg ccaggttgcc tactcccaca ccactcacaa 3660
   gaagattcta ctgttagtat taaatatttt ttaatgtatt aaatgatgaa tgcttttgta 3720
   aacagaacaa gactatgtct aataagtgtc ttgcaacatt ttttaagaaa ttaaaaaaaa 3780
   tatatttatt atcaaaatca aatgtatgaa aaatcatgaa taatataatt ttatacattt 3840
   ttttaaaaaa tcttttaatt tcttaattaa tatcttaaaa ataatgatta atatttaacc 3900
   caaaataatt agtatgattg gtaaggaaga tatccatgtt atgtttggat gtgagtttga 3960
   tctagagcaa agcttactag agtcgaccga tccgtcgacg gcgcggatcc tcgaagagaa 4020
   gggttaataa cacatttttt aacattttta acacaaattt tagttattta aaaatttatt 4080
   aaaaaattta aaataagaag aggaactctt taaataaatc taacttacaa aatttatgat 4140
   ttttaataag ttttcaccaa taaaaaatgt cataaaaata tgttaaaaag tatattatca 4200
   atattctctt tatgataaat aaaaagaaaa aaaaaataaa agttaagtga aaatgagatt 4260
   gaagtgactt taggtgtgta taaatatatc aaccccgcca acaatttatt taatccaaat 4320
   atattgaagt atattattcc atagccttta tttatttata tatttattat ataaaagctt 4380
   tatttgttct aggttgttca tgaaatattt ttttggtttt atctccgttg taagaaaatc 4440
   atgtgctttg tgtcgccact cactattgca gctttttcat gcattggtca gattgacggt 4500
   tgattgtatt tttgtttttt atggttttgt gttatgactt aagtcttcat ctctttatct 4560
   cttcatcagg tttgatggtt acctaatatg gtccatgggt acatgcatgg ttaaattagg 4620
   tggccaactt tgttgtgaac gatagaattt tttttatatt aagtaaacta tttttatatt 4680
   atgaaataat aataaaaaaa atattttatc attattaaca aaatcatatt agttaatttg 4740
   ttaactctat aataaaagaa atactgtaac attcacatta catggtaaca tctttccacc 4800
   ctttcatttg ttttttgttt gatgactttt tttcttgttt aaatttattt cccttctttt 4860
   aaatttggaa tacattatca tcatatataa actaaaatac taaaaacagg attacacaaa 4920
   tgataaataa taacacaaat atttataaat ctagctgcaa tatatttaaa ctagctatat 4980
   cgatattgta aaataaaact agctgcattg atactgataa aaaaatatca tgtgctttct 5040
   ggactgatga tgcagtatac ttttgacatt gcctttattt tatttttcag aaaagctttc 5100
   ttagttctgg gttcttcatt atttgtttcc catctccatt gtgaattgaa tcatttgctt 5160
   cgtgtcacaa atacaattta gntaggtaca tgcattggtc agattcacgg tttattatgt 5220
   catgacttaa gttcatggta gtacattacc tgccacgcat gcattatatt ggttagattt 5280
   gataggcaaa tttggttgtc aacaatataa atataaataa tgtttttata ttacgaaata 5340
   acagtgatca aaacaaacag ttttatcttt attaacaaga ttttgttttt gtttgatgac 5400
   gttttttaat gtttacgctt tcccccttct tttgaattta gaacacttta tcatcataaa 5460
   atcaaatact aaaaaaatta catatttcat aaataataac acaaatattt ttaaaaaatc 5520
   tgaaataata atgaacaata ttacatatta tcacgaaaat tcattaataa aaatattata 5580
   taaataaaat gtaatagtag ttatatgtag gaaaaaagta ctgcacgcat aatatataca 5640
   aaaagattaa aatgaactat tataaataat aacactaaat taatggtgaa tcatatcaaa 5700
   ataatgaaaa agtaaataaa atttgtaatt aacttctata tgtattacac acacaaataa 5760
   taaataatag taaaaaaaat tatgataaat atttaccatc tcataagata tttaaaataa 5820
   tgataaaaat atagattatt ttttatgcaa ctagctagcc aaaaagagaa cacgggtata 5880
   tataaaaaga gtacctttaa attctactgt acttccttta ttcctgacgt ttttatatca 5940
   agtggacata cgtgaagatt ttaattatca gtctaaatat ttcattagca cttaatactt 6000
   ttctgtttta ttcctatcct ataagtagtc ccgattctcc caacattgct tattcacaca 6060
   actaactaag aaagtcttcc atagcccccc aagcggccgg agctggtcat ctcgctcatc 6120
   gtcgagtcgg cggccggagc tggtcatctc gctcatcgtc gagtcggcgg ccgccggtcc 6180
   tctctctttc cgtggcatgg caatctattg ggctgtccag ggttgcatcc ttactggtgt 6240
   ttgggtcatt gcccatgagt gtggtcacca tgcattcagt gactaccagc tgcttgatga 6300
   tattgttggc cttatcctcc actccgctct cctagtcccg tacttttcat ggaaatacag 6360
   ccatcgccgt caccactcca acactggttc tcttgagcgg gatgaagtat ttgtgccaaa 6420
   gcagaagtcc tgtatcaagt ggtactctaa ataccttaac aatcctccag gcagagtcct 6480
   cactcttgct gtcaccctca cacttggttg gcccttgtac ttggctttaa atgtttctgg 6540
   aaggccttat gatagatttg cttgccacta tgacccatat ggtcccattt actctgatcg 6600
   tgaacgactt caaatatata tatcagatgc aggagtactt gcaggactta ctctctctac 6660
   cgtgttgcaa ccctgaaagg gttggtttgg ctgctatgtg tttatggggt gcctttgctc 6720
   attgtgaacg gttttcttgt gactatcaca tatttgcagc acacacactt tgccttgcct 6780
   cattacgatt catcagaatg ggactggctg aagggagctt tggcaactat ggacagagat 6840
   tatgggattc tgaacaaggt gtttcatcac ataactgata ctcatgtggc tcaccatctc 6900
   ttctctacaa tgccacatta ccatgcaatg gaggcaacca atgcaatcaa gccaatattg 6960
   ggtgagtact accaatttga tgacacacca ttttacaagg cactgtggag agaagcgaga 7020
   gagtgcctct atgtggagcc agatgaagga acatccgaga agggctcctc caccgtttaa 7080
   gattgcagaa atcagagctt caataccaaa acattgctgg gtcaagaatc catggagatc 7140
   cctcagttat gttctcaggg atgtgcttgt aattgctgca ttggtggctg cagcaattca 7200
   cttcgacaac tggcttctct ggctaatcta ttgccccatt caaggcacaa tgttctgggc 7260
   tctctttgtt cttggacatg attgtggcca tggaagcttt tcagatagcc ctttgctgaa 7320
   tagcctggtg ggacacatct tgcattcctc aattcttgtg ccataccatg gatggagaat 7380
   tagccacaga actcaccatc aaaaccatgg acacattgag aaggatgagt catgggttcc 7440
   attaacagag aagatttaca agaatctaga cagcatgaca agactcatta gattcactgt 7500
   gccatttcca ttgtttgtgt atccaattta tttgttttca agaagccccg gaaaggaagg 7560
   ctctcacttc aatccctaca gcaatctgtt cccacccagt gagagaaaag gaatagcaat 7620
   atcaacactg tgttgggcta ccatgttttc tctgcttatc tatctctcat tcataactag 7680
   tccacttcta gtgctcaagc tctatgggcg gccgccgact cgacgatgag cgagatgacc 7740
   agctccggcc gccgactcga cgatgagcga gatgaccagc tccggccgcg acacaagtgt 7800
   gagagtacta aataaatgct ttggttgtac gaaatcatta cactaaataa aataatcaaa 7860
   gcttatatat gccttccgct aaggccgaat gcaaagaaat tggttctttc tcgttatctt 7920
   ttgccacttt tactagtacg tattaattac tacttaatca tctttgttta cggctcatta 7980
   tatccgtcga cgg 7993
<210> 2
   <211> 1533
   <212> DNA
   <213> 1533 kb recombinant fragment
<400> 2
   cggtcctctc tctttccgtg gcatggcaat ctattgggct gtccagggtt gcatccttac      60
   tggtgtttgg gtcattgccc atgagtgtgg tcaccatgca ttcagtgact accagctgct     120
   tgatgatatt gttggcctta tcctccactc cgctctccta gtcccgtact tttcatggaa     180
   atacagccat cgccgtcacc actccaacac tggttctctt gagcgggatg aagtatttgt     240
   gccaaagcag aagtcctgta tcaagtggta ctctaaatac cttaacaatc ctccaggcag     300
   agtcctcact cttgctgtca ccctcacact tggttggccc ttgtacttgg ctttaaatgt     360
   ttctggaagg ccttatgata gatttgcttg ccactatgac ccatatggtc ccatttactc     420
   tgatcgtgaa cgacttcaaa tatatatatc agatgcagga gtacttgcag gacttactct     480
   ctctaccgtg ttgcaaccct gaaagggttg gtttggctgc tatgtgttta tggggtgcct     540
   ttgctcattg tgaacggttt tcttgtgact atcacatatt tgcagcacac acactttgcc     600
   ttgcctcatt acgattcatc agaatgggac tggctgaagg gagctttggc aactatggac     660
   agagattatg ggattctgaa caaggtgttt catcacataa ctgatactca tgtggctcac     720
   catctcttct ctacaatgcc acattaccat gcaatggagg caaccaatgc aatcaagcca     780
   atattgggtg agtactacca atttgatgac acaccatttt acaaggcact gtggagagaa     840
   gcgagagagt gcctctatgt ggagccagat gaaggaacat ccgagaaggg ctcctccacc     900
   gtttaagatt gcagaaatca gagcttcaat accaaaacat tgctgggtca agaatccatg     960
   gagatccctc agttatgttc tcagggatgt gcttgtaatt gctgcattgg tggctgcagc    1020
   aattcacttc gacaactggc ttctctggct aatctattgc cccattcaag gcacaatgtt    1080
   ctgggctctc tttgttcttg gacatgattg tggccatgga agcttttcag atagcccttt    1140
   gctgaatagc ctggtgggac acatcttgca ttcctcaatt cttgtgccat accatggatg    1200
   gagaattagc cacagaactc accatcaaaa ccatggacac attgagaagg atgagtcatg    1260
   ggttccatta acagagaaga tttacaagaa tctagacagc atgacaagac tcattagatt    1320
   cactgtgcca tttccattgt ttgtgtatcc aatttatttg ttttcaagaa gccccggaaa    1380
   ggaaggctct cacttcaatc cctacagcaa tctgttccca cccagtgaga gaaaaggaat    1440
   agcaatatca acactgtgtt gggctaccat gttttctctg cttatctatc tctcattcat    1500
   aactagtcca cttctagtgc tcaagctcta tgg                                 1533
<210> 3
   <211> 28
   <212> DNA
   <213> primer BM35
<400> 3
   gcggccgccg gtcctctctc tttccgtg 28
<210> 4
   <211> 31
   <212> DNA
   <213> primer BM39
<400> 4
   taaacggtgg aggagccctt ctcggatgtt c 31
<210> 5
   <211> 33
   <212> DNA
   <213> primer BM40
<400> 5
   gaacatccga gaagggctcc tccaccgttt aag 33
<210> 6
   <211> 28
   <212> DNA
   <213> primer BM41
<400> 6
   gcggccgccc atagagcttg agcactag 28
<210> 7
   <211> 890
   <212> DNA
   <213> Glycine max KSFAD2 hybrid
<400> 7
   cggtcctctc tctttccgtg gcatggcaat ctattgggct gtccagggtt gcatccttac      60
   tggtgtttgg gtcattgccc atgagtgtgg tcaccatgca ttcagtgact accagctgct     120
   tgatgatatt gttggcctta tcctccactc cgctctccta gtcccgtact tttcatggaa     180
   atacagccat cgccgtcacc actccaacac tggttctctt gagcgggatg aagtatttgt     240
   gccaaagcag aagtcctgta tcaagtggta ctctaaatac cttaacaatc ctccaggcag     300
   agtcctcact cttgctgtca ccctcacact tggttggccc ttgtacttgg ctttaaatgt     360
   ttctggaagg ccttatgata gatttgcttg ccactatgac ccatatggtc ccatttactc     420
   tgatcgtgaa cgacttcaaa tatatatatc agatgcagga gtacttgcag gacttactct     480
   ctctaccgtg ttgcaaccct gaaagggttg gtttggctgc tatgtgttta tggggtgcct     540
   ttgctcattg tgaacggttt tcttgtgact atcacatatt tgcagcacac acactttgcc     600
   ttgcctcatt acgattcatc agaatgggac tggctgaagg gagctttggc aactatggac     660
   agagattatg ggattctgaa caaggtgttt catcacataa ctgatactca tgtggctcac     720
   catctcttct ctacaatgcc acattaccat gcaatggagg caaccaatgc aatcaagcca     780
   atattgggtg agtactacca atttgatgac acaccatttt acaaggcact gtggagagaa     840
   gcgagagagt gcctctatgt ggagccagat gaaggaacat ccgagaaggg                890
<210> 8
   <211> 28
   <212> DNA
   <213> primer KS1
<400> 8
   gcggccgccg gtcctctctc tttccgtg 28
<210> 9
   <211> 30
   <212> DNA
   <213> primer KS2
<400> 9
   tagagagagt aagtcctgca agtactcctg 30
<210> 10
   <211> 30
   <212> DNA
   <213> primer KS3
<400> 10
   caggagtact tgcaggactt actctctcta 30
<210> 11
   <211> 29
   <212> DNA
   <213> primer KS4
<400> 11
   gcggccggcc ccttctcgga tgttccttc 29
<210> 12
   <211> 2460
   <212> DNA
   <213> gene expression-silencing cassette from pKS133 which comprises Kti3 promoter and terminator sequences bordering a string of nucleotides that promote formation of a stem structure surrounding a unique Not1 restriction site
<220>
   <221> misc_feature
   <222> (1186)..(1186)
   <223> n is a, c, g, or t
<400> 12
   ccaagcttgg atcctcgaag agaagggtta ataacacatt ttttaacatt tttaacacaa     60
   attttagtta tttaaaaatt tattaaaaaa tttaaaataa gaagaggaac tctttaaata    120
   aatctaactt acaaaattta tgatttttaa taagttttca ccaataaaaa atgtcataaa    180
   aatatgttaa aaagtatatt atcaatattc tctttatgat aaataaaaag aaaaaaaaaa    240
   taaaagttaa gtgaaaatga gattgaagtg actttaggtg tgtataaata tatcaacccc    300
   gccaacaatt tatttaatcc aaatatattg aagtatatta ttccatagcc tttatttatt    360
   tatatattta ttatataaaa gctttatttg ttctaggttg ttcatgaaat atttttttgg    420
   ttttatctcc gttgtaagaa aatcatgtgc tttgtgtcgc cactcactat tgcagctttt    480
   tcatgcattg gtcagattga cggttgattg tatttttgtt ttttatggtt ttgtgttatg    540
   acttaagtct tcatctcttt atctcttcat caggtttgat ggttacctaa tatggtccat    600
   gggtacatgc atggttaaat taggtggcca actttgttgt gaacgataga atttttttta    660
   tattaagtaa actattttta tattatgaaa taataataaa aaaaatattt tatcattatt    720
   aacaaaatca tattagttaa tttgttaact ctataataaa agaaatactg taacattcac    780
   attacatggt aacatctttc caccctttca tttgtttttt gtttgatgac tttttttctt    840
   gtttaaattt atttcccttc ttttaaattt ggaatacatt atcatcatat ataaactaaa    900
   atactaaaaa caggattaca caaatgataa ataataacac aaatatttat aaatctagct    960
   gcaatatatt taaactagct atatcgatat tgtaaaataa aactagctgc attgatactg   1020
   ataaaaaaat atcatgtgct ttctggactg atgatgcagt atacttttga cattgccttt   1080
   attttatttt tcagaaaagc tttcttagtt ctgggttctt cattatttgt ttcccatctc   1140
   cattgtgaat tgaatcattt gcttcgtgtc acaaatacaa tttagntagg tacatgcatt   1200
   ggtcagattc acggtttatt atgtcatgac ttaagttcat ggtagtacat tacctgccac   1260
   gcatgcatta tattggttag atttgatagg caaatttggt tgtcaacaat ataaatataa   1320
   ataatgtttt tatattacga aataacagtg atcaaaacaa acagttttat ctttattaac   1380
   aagattttgt ttttgtttga tgacgttttt taatgtttac gctttccccc ttcttttgaa   1440
   tttagaacac tttatcatca taaaatcaaa tactaaaaaa attacatatt tcataaataa   1500
   taacacaaat atttttaaaa aatctgaaat aataatgaac aatattacat attatcacga   1560
   aaattcatta ataaaaatat tatataaata aaatgtaata gtagttatat gtaggaaaaa   1620
   agtactgcac gcataatata tacaaaaaga ttaaaatgaa ctattataaa taataacact   1680
   aaattaatgg tgaatcatat caaaataatg aaaaagtaaa taaaatttgt aattaacttc   1740
   tatatgtatt acacacacaa ataataaata atagtaaaaa aaattatgat aaatatttac   1800
   catctcataa gatatttaaa ataatgataa aaatatagat tattttttat gcaactagct   1860
   agccaaaaag agaacacggg tatatataaa aagagtacct ttaaattcta ctgtacttcc   1920
   tttattcctg acgtttttat atcaagtgga catacgtgaa gattttaatt atcagtctaa   1980
   atatttcatt agcacttaat acttttctgt tttattccta tcctataagt agtcccgatt   2040
   ctcccaacat tgcttattca cacaactaac taagaaagtc ttccatagcc ccccaagcgg   2100
   ccggagctgg tcatctcgct catcgtcgag tcggcggccg gagctggtca tctcgctcat   2160
   cgtcgagtcg gcggccgccg actcgacgat gagcgagatg accagctccg gccgccgact   2220
   cgacgatgag cgagatgacc agctccggcc gcgacacaag tgtgagagta ctaaataaat   2280
   gctttggttg tacgaaatca ttacactaaa taaaataatc aaagcttata tatgccttcc   2340
   gctaaggccg aatgcaaaga aattggttct ttctcgttat cttttgccac ttttactagt   2400
   acgtattaat tactacttaa tcatctttgt ttacggctca ttatatccgt cgacggcgcg   2460
<210> 13
   <211> 8966
   <212> DNA
   <213> vector pKS210
<220>
   <221> misc_feature
   <222> (1177)..(1177)
   <223> n is a, c, g, or t
<400> 13
   gatcctcgaa gagaagggtt aataacacat tttttaacat ttttaacaca aattttagtt     60
   atttaaaaat ttattaaaaa atttaaaata agaagaggaa ctctttaaat aaatctaact    120
   tacaaaattt atgattttta ataagttttc accaataaaa aatgtcataa aaatatgtta    180
   aaaagtatat tatcaatatt ctctttatga taaataaaaa gaaaaaaaaa ataaaagtta 240
   agtgaaaatg agattgaagt gactttaggt gtgtataaat atatcaaccc cgccaacaat 300
   ttatttaatc caaatatatt gaagtatatt attccatagc ctttatttat ttatatattt 360
   attatataaa agctttattt gttctaggtt gttcatgaaa tatttttttg gttttatctc 420
   cgttgtaaga aaatcatgtg ctttgtgtcg ccactcacta ttgcagcttt ttcatgcatt 480
   ggtcagattg acggttgatt gtatttttgt tttttatggt tttgtgttat gacttaagtc 540
   ttcatctctt tatctcttca tcaggtttga tggttaccta atatggtcca tgggtacatg 600
   catggttaaa ttaggtggcc aactttgttg tgaacgatag aatttttttt atattaagta 660
   aactattttt atattatgaa ataataataa aaaaaatatt ttatcattat taacaaaatc 720
   atattagtta atttgttaac tctataataa aagaaatact gtaacattca cattacatgg 780
   taacatcttt ccaccctttc atttgttttt tgtttgatga ctttttttct tgtttaaatt 840
   tatttccctt cttttaaatt tggaatacat tatcatcata tataaactaa aatactaaaa 900
   acaggattac acaaatgata aataataaca caaatattta taaatctagc tgcaatatat 960
   ttaaactagc tatatcgata ttgtaaaata aaactagctg cattgatact gataaaaaaa 1020
   tatcatgtgc tttctggact gatgatgcag tatacttttg acattgcctt tattttattt 1080
   ttcagaaaag ctttcttagt tctgggttct tcattatttg tttcccatct ccattgtgaa 1140
   ttgaatcatt tgcttcgtgt cacaaataca atttagntag gtacatgcat tggtcagatt 1200
   cacggtttat tatgtcatga cttaagttca tggtagtaca ttacctgcca cgcatgcatt 1260
   atattggtta gatttgatag gcaaatttgg ttgtcaacaa tataaatata aataatgttt 1320
   ttatattacg aaataacagt gatcaaaaca aacagtttta tctttattaa caagattttg 1380
   tttttgtttg atgacgtttt ttaatgttta cgctttcccc cttcttttga atttagaaca 1440
   ctttatcatc ataaaatcaa atactaaaaa aattacatat ttcataaata ataacacaaa 1500
   tatttttaaa aaatctgaaa taataatgaa caatattaca tattatcacg aaaattcatt 1560
   aataaaaata ttatataaat aaaatgtaat agtagttata tgtaggaaaa aagtactgca 1620
   cgcataatat atacaaaaag attaaaatga actattataa ataataacac taaattaatg 1680
   gtgaatcata tcaaaataat gaaaaagtaa ataaaatttg taattaactt ctatatgtat 1740
   tacacacaca aataataaat aatagtaaaa aaaattatga taaatattta ccatctcata 1800
   agatatttaa aataatgata aaaatataga ttatttttta tgcaactagc tagccaaaaa 1860
   gagaacacgg gtatatataa aaagagtacc tttaaattct actgtacttc ctttattcct 1920
   gacgttttta tatcaagtgg acatacgtga agattttaat tatcagtcta aatatttcat 1980
   tagcacttaa tacttttctg ttttattcct atcctataag tagtcccgat tctcccaaca 2040
   ttgcttattc acacaactaa ctaagaaagt cttccatagc cccccaagcg gccggagctg 2100
   gtcatctcgc tcatcgtcga gtcggcggcc ggagctggtc atctcgctca tcgtcgagtc 2160
   ggcggccgcc gactcgacga tgagcgagat gaccagctcc ggccgccgac tcgacgatga 2220
   gcgagatgac cagctccggc cgcgacacaa gtgtgagagt actaaataaa tgctttggtt 2280
   gtacgaaatc attacactaa ataaaataat caaagcttat atatgccttc cgctaaggcc 2340
   gaatgcaaag aaattggttc tttctcgtta tcttttgcca cttttactag tacgtattaa 2400
   ttactactta atcatctttg tttacggctc attatatccg tcgacggcgc gcccgatcat 2460
   ccggatatag ttcctccttt cagcaaaaaa cccctcaaga cccgtttaga ggccccaagg 2520
   ggttatgcta gttattgctc agcggtggca gcagccaact cagcttcctt tcgggctttg 2580
   ttagcagccg gatcgatcca agctgtacct cactattcct ttgccctcgg acgagtgctg 2640
   gggcgtcggt ttccactatc ggcgagtact tctacacagc catcggtcca gacggccgcg 2700
   cttctgcggg cgatttgtgt acgcccgaca gtcccggctc cggatcggac gattgcgtcg 2760
   catcgaccct gcgcccaagc tgcatcatcg aaattgccgt caaccaagct ctgatagagt 2820
   tggtcaagac caatgcggag catatacgcc cggagccgcg gcgatcctgc aagctccgga 2880
   tgcctccgct cgaagtagcg cgtctgctgc tccatacaag ccaaccacgg cctccagaag 2940
   aagatgttgg cgacctcgta ttgggaatcc ccgaacatcg cctcgctcca gtcaatgacc 3000
   gctgttatgc ggccattgtc cgtcaggaca ttgttggagc cgaaatccgc gtgcacgagg 3060
   tgccggactt cggggcagtc ctcggcccaa agcatcagct catcgagagc ctgcgcgacg 3120
   gacgcactga cggtgtcgtc catcacagtt tgccagtgat acacatgggg atcagcaatc 3180
   gcgcatatga aatcacgcca tgtagtgtat tgaccgattc cttgcggtcc gaatgggccg 3240
   aacccgctcg tctggctaag atcggccgca gcgatcgcat ccatagcctc cgcgaccggc 3300
   tgcagaacag cgggcagttc ggtttcaggc aggtcttgca acgtgacacc ctgtgcacgg 3360
   cgggagatgc aataggtcag gctctcgctg aattccccaa tgtcaagcac ttccggaatc 3420
   gggagcgcgg ccgatgcaaa gtgccgataa acataacgat ctttgtagaa accatcggcg 3480
   cagctattta cccgcaggac atatccacgc cctcctacat cgaagctgaa agcacgagat 3540
   tcttcgccct ccgagagctg catcaggtcg gagacgctgt cgaacttttc gatcagaaac 3600
   ttctcgacag acgtcgcggt gagttcaggc ttttccatgg gtatatctcc ttcttaaagt 3660
   taaacaaaat tatttctaga gggaaaccgt tgtggtctcc ctatagtgag tcgtattaat 3720
   ttcgcgggat cgagatctga tcaacctgca ttaatgaatc ggccaacgcg cggggagagg 3780
   cggtttgcgt attgggcgct cttccgcttc ctcgctcact gactcgctgc gctcggtcgt 3840
   tcggctgcgg cgagcggtat cagctcactc aaaggcggta atacggttat ccacagaatc 3900
   aggggataac gcaggaaaga acatgtgagc aaaaggccag caaaaggcca ggaaccgtaa 3960
   aaaggccgcg ttgctggcgt ttttccatag gctccgcccc cctgacgagc atcacaaaaa 4020
   tcgacgctca agtcagaggt ggcgaaaccc gacaggacta taaagatacc aggcgtttcc 4080
   ccctggaagc tccctcgtgc gctctcctgt tccgaccctg ccgcttaccg gatacctgtc 4140
   cgcctttctc ccttcgggaa gcgtggcgct ttctcaatgc tcacgctgta ggtatctcag 4200
   ttcggtgtag gtcgttcgct ccaagctggg ctgtgtgcac gaaccccccg ttcagcccga 4260
   ccgctgcgcc ttatccggta actatcgtct tgagtccaac ccggtaagac acgacttatc 4320
   gccactggca gcagccactg gtaacaggat tagcagagcg aggtatgtag gcggtgctac 4380
   agagttcttg aagtggtggc ctaactacgg ctacactaga aggacagtat ttggtatctg 4440
   cgctctgctg aagccagtta ccttcggaaa aagagttggt agctcttgat ccggcaaaca 4500
   aaccaccgct ggtagcggtg gtttttttgt ttgcaagcag cagattacgc gcagaaaaaa 4560
   aggatctcaa gaagatcctt tgatcttttc tacggggtct gacgctcagt ggaacgaaaa 4620
   ctcacgttaa gggattttgg tcatgacatt aacctataaa aataggcgta tcacgaggcc 4680
   ctttcgtctc gcgcgtttcg gtgatgacgg tgaaaacctc tgacacatgc agctcccgga 4740
   gacggtcaca gcttgtctgt aagcggatgc cgggagcaga caagcccgtc agggcgcgtc 4800
   agcgggtgtt ggcgggtgtc ggggctggct taactatgcg gcatcagagc agattgtact 4860
   gagagtgcac catatggaca tattgtcgtt agaacgcggc tacaattaat acataacctt 4920
   atgtatcata cacatacgat ttaggtgaca ctatagaacg gcgcgccaag cttggatccg 4980
   cgccaagctt ggatcctaga actagaaacg tgatgccact tgttattgaa gtcgattaca 5040
   gcatctattc tgttttacta tttataactt tgccatttct gacttttgaa aactatctct 5100
   ggatttcggt atcgctttgt gaagatcgag caaaagagac gttttgtgga cgcaatggtc 5160
   caaatccgtt ctacatgaac aaattggtca caatttccac taaaagtaaa taaatggcaa 5220
   gttaaaaaag gaatatgcat tttactgatt gcctaggtga gctccaagag aagttgaatc 5280
   tacacgtcta ccaaccgcta aaaaaagaaa aacattgata tgtaacctga ttccattagc 5340
   ttttgacttc ttcaacagat tctctactta gatttctaac agaaatatta ttactagcac 5400
   atcattttca gtctcactac agcaaaaaat ccaacggcac aatacagaca acaggagata 5460
   tcagactaca gagatagata gatgctactg catgtagtaa gttaaataaa aggaaaataa 5520
   aatgtcttgc taccaaaact actacagact atgatgctca ccacaggcca aatcctgcaa 5580
   ctaggacagc attatcttat atatattgta caaaacaagc atcaaggaac atttggtcta 5640
   ggcaatcagt acctcgttct accatcaccc tcagttatca catccttgaa ggatccatta 5700
   ctgggaatca tcggcaacac atgctcctga tggggcacaa tgacatcaag aaggtagggg 5760
   ccaggggtgt ccaacattct ctgaattgcc gctctaagct cttccttctt cgtcactcgc 5820
   gctgccggta tcccacaagc atcagcaaac ttgagcatgt ttgggaatat ctcgctctcg 5880
   ctagacggat ctccaagata ggtgtgagct ctattggact tgtagaacct atcctccaac 5940
   tgaaccacca tacccaaatg ctgattgttc aacaacaata tcttaactgg gagattctcc 6000
   actcttatag tggccaactc ctgaacattc atgatgaaac taccatcccc atcaatgtca 6060
   accacaacag ccccagggtt agcaacagca gcaccaatag ccgcaggcaa tccaaaaccc 6120
   atggctccaa gaccccctga ggtcaaccac tgcctcggtc tcttgtactt gtaaaactgc 6180
   gcagcccaca tttgatgctg cccaacccca gtactaacaa tagcatctcc attagtcaac 6240
   tcatcaagaa cctcgatagc atgctgcgga gaaatcgcgt cctggaatgt cttgtaaccc 6300
   aatggaaact tgtgtttctg cacattaatc tcttctctcc aacctccaag atcaaactta 6360
   ccctccactc ctttctcctc caaaatcata ttaattccct tcaaggccaa cttcaaatcc 6420
   gcgcaaaccg acacgtgcgc ctgcttgttc ttcccaatct cggcagaatc aatatcaatg 6480
   tgaacaatct tagccctact agcaaaagcc tcaagcttcc cagtaacacg gtcatcaaac 6540
   cttaccccaa aggcaagcaa caaatcacta ttgtcaacag catagttagc ataaacagta 6600
   ccatgcatac ccagcatctg aagggaatat tcatcaccaa taggaaaagt tccaagaccc 6660
   attaaagtgc tagcaacggg aataccagtg agttcaacaa agcgcctcaa ttcagcactg 6720
   gaattcaaac tgccaccgcc gacgtagaga acgggctttt gggcctccat gatgagtctg 6780
   acaatgtgtt ccaattgggc ctcggcgggg ggcctgggca gcctggcgag gtaaccgggg 6840
   aggttaacgg gctcgtccca attaggcacg gcgagttgct gctgaacgtc tttgggaatg 6900
   tcgatgagga ccggaccggg gcggccggag gtggcgacga agaaagcctc ggcgacgacg 6960
   cgggggatgt cgtcgacgtc gaggatgagg tagttgtgct tcgtgatgga tctgctcacc 7020
   tccacgatcg gggtttcttg gaaggcgtcg gtgccgatca tccggcgggc gacctggccg 7080
   gtgatggcga cgactgggac gctgtccatt aaagcgtcgg cgaggccgct cacgaggttg 7140
   gtggcgccgg ggccggaggt ggcaatgcag acgccgggga ggccggagga acgcgcgtag 7200
   ccttcggcgg cgaagacgcc gccctgctcg tggcgcggga gcacgttgcg gatggcggcg 7260
   gagcgcgtga gcgcctggtg gatctccatc gacgcaccgc cggggtacgc gaacaccgtc 7320
   gtcacgccct gcctctccag cgcctccaca aggatgtccg cgcccttgcg aggttcgccg 7380
   gaggcgaacc gtgacacgaa gggctccgtg gtcggcgctt ccttggtgaa gggcgccgcc 7440
   gtggggggtt tggagatgga acatttgatt ttgagagcgt ggttgggttt ggtgagggtt 7500
   tgatgagaga gagggagggt ggatctagta atgcgtttgg ggaaggtggg gtgtgaagag 7560
   gaagaagaga atcgggtggt tctggaagcg gtggccgcca ttgtgttgtg tggcatggtt 7620
   atacttcaaa aactgcacaa caagcctaga gttagtacct aaacagtaaa tttacaacag 7680
   agagcaaaga cacatgcaaa aatttcagcc ataaaaaaag ttataataga atttaaagca 7740
   aaagtttcat tttttaaaca tatatacaaa caaactggat ttgaaggaag ggattaattc 7800
   ccctgctcaa agtttgaatt cctattgtga cctatactcg aataaaattg aagcctaagg 7860
   aatgtatgag aaacaagaaa acaaaacaaa actacagaca aacaagtaca attacaaaat 7920
   tcgctaaaat tctgtaatca ccaaacccca tctcagtcag cacaaggccc aaggtttatt 7980
   ttgaaataaa aaaaaagtga ttttatttct cataagctaa aagaaagaaa ggcaattatg   8040
   aaatgatttc gactagatct gaaagtccaa cgcgtattcc gcagatatta aagaaagagt   8100
   agagtttcac atggatccta gatggaccca gttgaggaaa aagcaaggca aagcaaacca   8160
   gaagtgcaag atccgaaatt gaaccacgga atctaggatt tggtagaggg agaagaaaag   8220
   taccttgaga ggtagaagag aagagaagag cagagagata tatgaacgag tgtgtcttgg   8280
   tctcaactct gaagcgatac gagtttagag gggagcattg agttccaatt tatagggaaa   8340
   ccgggtggca ggggtgagtt aatgacggaa aagcccctaa gtaacgagat tggattgtgg   8400
   gttagattca accgtttgca tccgcggctt agattgggga agtcagagtg aatctcaacc   8460
   gttgactgag ttgaaaattg aatgtagcaa ccaattgagc caaccccagc ctttgccctt   8520
   tgattttgat ttgtttgttg catacttttt atttgtcttc tggttctgac tctctttctc   8580
   tcgtttcaat gccaggttgc ctactcccac accactcaca agaagattct actgttagta   8640
   ttaaatattt tttaatgtat taaatgatga atgcttttgt aaacagaaca agactatgtc   8700
   taataagtgt cttgcaacat tttttaagaa attaaaaaaa atatatttat tatcaaaatc   8760
   aaatgtatga aaaatcatga ataatataat tttatacatt tttttaaaaa atcttttaat   8820
   ttcttaatta atatcttaaa aataatgatt aatatttaac ccaaaataat tagtatgatt   8880
   ggtaaggaag atatccatgt tatgtttgga tgtgagtttg atctagagca aagcttacta   8940
   gagtcgaccg atccgtcgac ggcgcg                                        8966
<210> 14
   <211> 6611
   <212> DNA
   <213> Plasmid PHP17731
<220>
   <221> misc_feature
   <222> (4436)..(4436)
   <223> n is a, c, g, or t
<400> 14
   cgcgcctatg cgggaccatc gcagcggacg agaagcggca cgagaacgcg tactcaagaa      60
   tcgtggagaa gcttctggaa gtggacccca ccggggcaat ggtggccata gggaacatga     120
   tggagaagaa gatcacgatg ccggcgcacc ttatgtacga tggggatgac cccaggctat     180
   tcgagcacta ctccgctgtg gcgcagcgca taggcgtgta caccgccaac gactacgcag     240
   acatcttgga tttctcgttg acggtgaaga ttggagaagc ttgaaggatt gatgcctgag     300
   gggaagcggg ccccaggatt tccgtgtgtg ggttgccccc gaggattagg aggttccaag     360
   aacgcgctga tgagcgagcg cgtaagatga agaagcatca tgccgttaag ttcagttgga     420
   ttttcaataa agaattgctt ttgtgagcgg ccgccgactc gacgatgagc gagatgacca     480
   gctccggccg ccgactcgac gatgagcgag atgaccagct ccggccgcga cacaagtgtg     540
   agagtactaa ataaatgctt tggttgtacg aaatcattac actaaataaa ataatcaaag 600
   cttatatatg ccttccgcta aggccgaatg caaagaaatt ggttctttct cgttatcttt 660
   tgccactttt actagtacgt attaattact acttaatcat ctttgtttac ggctcattat 720
   atccgtcgac ggcgcgcccg atcatccgga tatagttcct cctttcagca aaaaacccct 780
   caagacccgt ttagaggccc caaggggtta tgctagttat tgctcagcgg tggcagcagc 840
   caactcagct tcctttcggg ctttgttagc agccggatcg atccaagctg tacctcacta 900
   ttcctttgcc ctcggacgag tgctggggcg tcggtttcca ctatcggcga gtacttctac 960
   acagccatcg gtccagacgg ccgcgcttct gcgggcgatt tgtgtacgcc cgacagtccc 1020
   ggctccggat cggacgattg cgtcgcatcg accctgcgcc caagctgcat catcgaaatt 1080
   gccgtcaacc aagctctgat agagttggtc aagaccaatg cggagcatat acgcccggag 1140
   ccgcggcgat cctgcaagct ccggatgcct ccgctcgaag tagcgcgtct gctgctccat 1200
   acaagccaac cacggcctcc agaagaagat gttggcgacc tcgtattggg aatccccgaa 1260
   catcgcctcg ctccagtcaa tgaccgctgt tatgcggcca ttgtccgtca ggacattgtt 1320
   ggagccgaaa tccgcgtgca cgaggtgccg gacttcgggg cagtcctcgg cccaaagcat 1380
   cagctcatcg agagcctgcg cgacggacgc actgacggtg tcgtccatca cagtttgcca 1440
   gtgatacaca tggggatcag caatcgcgca tatgaaatca cgccatgtag tgtattgacc 1500
   gattccttgc ggtccgaatg ggccgaaccc gctcgtctgg ctaagatcgg ccgcagcgat 1560
   cgcatccata gcctccgcga ccggctgcag aacagcgggc agttcggttt caggcaggtc 1620
   ttgcaacgtg acaccctgtg cacggcggga gatgcaatag gtcaggctct cgctgaattc 1680
   cccaatgtca agcacttccg gaatcgggag cgcggccgat gcaaagtgcc gataaacata 1740
   acgatctttg tagaaaccat cggcgcagct atttacccgc aggacatatc cacgccctcc 1800
   tacatcgaag ctgaaagcac gagattcttc gccctccgag agctgcatca ggtcggagac 1860
   gctgtcgaac ttttcgatca gaaacttctc gacagacgtc gcggtgagtt caggcttttc 1920
   catgggtata tctccttctt aaagttaaac aaaattattt ctagagggaa accgttgtgg 1980
   tctccctata gtgagtcgta ttaatttcgc gggatcgaga tctgatcaac ctgcattaat 2040
   gaatcggcca acgcgcgggg agaggcggtt tgcgtattgg gcgctcttcc gcttcctcgc 2100
   tcactgactc gctgcgctcg gtcgttcggc tgcggcgagc ggtatcagct cactcaaagg 2160
   cggtaatacg gttatccaca gaatcagggg ataacgcagg aaagaacatg tgagcaaaag 2220
   gccagcaaaa ggccaggaac cgtaaaaagg ccgcgttgct ggcgtttttc cataggctcc 2280
   gcccccctga cgagcatcac aaaaatcgac gctcaagtca gaggtggcga aacccgacag 2340
   gactataaag ataccaggcg tttccccctg gaagctccct cgtgcgctct cctgttccga 2400
   ccctgccgct taccggatac ctgtccgcct ttctcccttc gggaagcgtg gcgctttctc 2460
   aatgctcacg ctgtaggtat ctcagttcgg tgtaggtcgt tcgctccaag ctgggctgtg 2520
   tgcacgaacc ccccgttcag cccgaccgct gcgccttatc cggtaactat cgtcttgagt 2580
   ccaacccggt aagacacgac ttatcgccac tggcagcagc cactggtaac aggattagca 2640
   gagcgaggta tgtaggcggt gctacagagt tcttgaagtg gtggcctaac tacggctaca 2700
   ctagaaggac agtatttggt atctgcgctc tgctgaagcc agttaccttc ggaaaaagag 2760
   ttggtagctc ttgatccggc aaacaaacca ccgctggtag cggtggtttt tttgtttgca 2820
   agcagcagat tacgcgcaga aaaaaaggat ctcaagaaga tcctttgatc ttttctacgg 2880
   ggtctgacgc tcagtggaac gaaaactcac gttaagggat tttggtcatg acattaacct 2940
   ataaaaatag gcgtatcacg aggccctttc gtctcgcgcg tttcggtgat gacggtgaaa 3000
   acctctgaca catgcagctc ccggagacgg tcacagcttg tctgtaagcg gatgccggga 3060
   gcagacaagc ccgtcagggc gcgtcagcgg gtgttggcgg gtgtcggggc tggcttaact 3120
   atgcggcatc agagcagatt gtactgagag tgcaccatat ggacatattg tcgttagaac 3180
   gcggctacaa ttaatacata accttatgta tcatacacat acgatttagg tgacactata 3240
   gaacggcgcg ccaagcttgg atcctcgaag agaagggtta ataacacatt ttttaacatt 3300
   tttaacacaa attttagtta tttaaaaatt tattaaaaaa tttaaaataa gaagaggaac 3360
   tctttaaata aatctaactt acaaaattta tgatttttaa taagttttca ccaataaaaa 3420
   atgtcataaa aatatgttaa aaagtatatt atcaatattc tctttatgat aaataaaaag 3480
   aaaaaaaaaa taaaagttaa gtgaaaatga gattgaagtg actttaggtg tgtataaata 3540
   tatcaacccc gccaacaatt tatttaatcc aaatatattg aagtatatta ttccatagcc 3600
   tttatttatt tatatattta ttatataaaa gctttatttg ttctaggttg ttcatgaaat 3660
   atttttttgg ttttatctcc gttgtaagaa aatcatgtgc tttgtgtcgc cactcactat 3720
   tgcagctttt tcatgcattg gtcagattga cggttgattg tatttttgtt ttttatggtt 3780
   ttgtgttatg acttaagtct tcatctcttt atctcttcat caggtttgat ggttacctaa 3840
   tatggtccat gggtacatgc atggttaaat taggtggcca actttgttgt gaacgataga 3900
   atttttttta tattaagtaa actattttta tattatgaaa taataataaa aaaaatattt 3960
   tatcattatt aacaaaatca tattagttaa tttgttaact ctataataaa agaaatactg 4020
   taacattcac attacatggt aacatctttc caccctttca tttgtttttt gtttgatgac 4080
   tttttttctt gtttaaattt atttcccttc ttttaaattt ggaatacatt atcatcatat 4140
   ataaactaaa atactaaaaa caggattaca caaatgataa ataataacac aaatatttat 4200
   aaatctagct gcaatatatt taaactagct atatcgatat tgtaaaataa aactagctgc 4260
   attgatactg ataaaaaaat atcatgtgct ttctggactg atgatgcagt atacttttga 4320
   cattgccttt attttatttt tcagaaaagc tttcttagtt ctgggttctt cattatttgt 4380
   ttcccatctc cattgtgaat tgaatcattt gcttcgtgtc acaaatacaa tttagntagg 4440
   tacatgcatt ggtcagattc acggtttatt atgtcatgac ttaagttcat ggtagtacat 4500
   tacctgccac gcatgcatta tattggttag atttgatagg caaatttggt tgtcaacaat 4560
   ataaatataa ataatgtttt tatattacga aataacagtg atcaaaacaa acagttttat 4620
   ctttattaac aagattttgt ttttgtttga tgacgttttt taatgtttac gctttccccc 4680
   ttcttttgaa tttagaacac tttatcatca taaaatcaaa tactaaaaaa attacatatt 4740
   tcataaataa taacacaaat atttttaaaa aatctgaaat aataatgaac aatattacat 4800
   attatcacga aaattcatta ataaaaatat tatataaata aaatgtaata gtagttatat 4860
   gtaggaaaaa agtactgcac gcataatata tacaaaaaga ttaaaatgaa ctattataaa 4920
   taataacact aaattaatgg tgaatcatat caaaataatg aaaaagtaaa taaaatttgt 4980
   aattaacttc tatatgtatt acacacacaa ataataaata atagtaaaaa aaattatgat 5040
   aaatatttac catctcataa gatatttaaa ataatgataa aaatatagat tattttttat 5100
   gcaactagct agccaaaaag agaacacggg tatatataaa aagagtacct ttaaattcta 5160
   ctgtacttcc tttattcctg acgtttttat atcaagtgga catacgtgaa gattttaatt 5220
   atcagtctaa atatttcatt agcacttaat acttttctgt tttattccta tcctataagt 5280
   agtcccgatt ctcccaacat tgcttattca cacaactaac taagaaagtc ttccatagcc 5340
   ccccaagcgg ccggagctgg tcatctcgct catcgtcgag tcggcggccg gagctggtca 5400
   tctcgctcat cgtcgagtcg gcggccgctg agtgattgct cacgagtgtg gtcaccatgc 5460
   cttcagcaag taccaatggg ttgatgatgt tgtgggtttg acccttcact caacactttt 5520
   agtcccttat ttctcatgga aaataagcca tcgccgccat cactccaaca caggttccct 5580
   tgaccgtgat gaagtgtttg tcccaaaacc aaaatccaaa gttgcatggt tttccaagta 5640
   cttaaacaac cctctaggaa gggctgtttc tcttctcgtc acactcacaa tagggtggcc 5700
   tatgtattta gccttcaatg tctctggtag accctatgat agttttgcaa gccactacca 5760
   cccttatgct cccatatatt ctaaccgtga gaggcttctg atctatgtct ctgatgttgc 5820
   tttgttttct gtgacttact ctctctaccg tgttgcaacc ctgaaagggt tggtttggct 5880
   gctatgtgtt tatggggtgc ctttgctcat tgtgaacggt tttcttgtga ctatcacata 5940
   tttgcagcac acacactttg ccttgcctca ttacgattca tcagaatggg actggctgaa 6000
   gggagctttg gcaactatgg acagagatta agcggccgca tgcctccaga aaagaaagaa 6060
   attttcaagt ccttggaggg atgggcctcg gagtgggtcc taccgctgct gaagcccgtg 6120
   gagcaatgct ggcagccaca aaacttcctc cctgacccct cccttccgca tgaagagttc 6180
   agccatcagg tgaaggagct tcgcgaacgc actaaagagt tacctgatga gtactttgtg 6240
   gtgctggtgg gtgatatggt caccgaggac gcgcttccca cttaccagac catgatcaac 6300
   aaccttgatg gagtgaaaga tgacagcggc acgagcccga gcccgtgggc cgtgtggacc 6360
   cgggcctgga ccgccgagga aaacagacac ggggatctgc tcagaactta tttgtatctc 6420
   tctgggaggg ttgacatggc taaggtcgaa aagaccgtac attacctcat ttcagctggc 6480
   atggaccctg ggacagacaa caacccatat ttggggtttg tgtacacgtc attccaagag 6540
   cgagcaacat ttgtggcgca cgggaacacg gctcggctcg cgaaggaggg cggggatcca 6600
   gtgctggcgc g 6611
<210> 15
   <211> 4097
   <212> DNA
   <213> Recombinant DNA fragment PHP17731A
<220>
   <221> misc_feature
   <222> (1190)..(1190)
   <223> n is a, c, g, or t
<400> 15
   cgcgccaagc ttggatcctc gaagagaagg gttaataaca cattttttaa catttttaac    60
   acaaatttta gttatttaaa aatttattaa aaaatttaaa ataagaagag gaactcttta   120
   aataaatcta acttacaaaa tttatgattt ttaataagtt ttcaccaata aaaaatgtca   180
   taaaaatatg ttaaaaagta tattatcaat attctcttta tgataaataa aaagaaaaaa   240
   aaaataaaag ttaagtgaaa atgagattga agtgacttta ggtgtgtata aatatatcaa   300
   ccccgccaac aatttattta atccaaatat attgaagtat attattccat agcctttatt   360
   tatttatata tttattatat aaaagcttta tttgttctag gttgttcatg aaatattttt   420
   ttggttttat ctccgttgta agaaaatcat gtgctttgtg tcgccactca ctattgcagc   480
   tttttcatgc attggtcaga ttgacggttg attgtatttt tgttttttat ggttttgtgt   540
   tatgacttaa gtcttcatct ctttatctct tcatcaggtt tgatggttac ctaatatggt   600
   ccatgggtac atgcatggtt aaattaggtg gccaactttg ttgtgaacga tagaattttt   660
   tttatattaa gtaaactatt tttatattat gaaataataa taaaaaaaat attttatcat   720
   tattaacaaa atcatattag ttaatttgtt aactctataa taaaagaaat actgtaacat   780
   tcacattaca tggtaacatc tttccaccct ttcatttgtt ttttgtttga tgactttttt   840
   tcttgtttaa atttatttcc cttcttttaa atttggaata cattatcatc atatataaac   900
   taaaatacta aaaacaggat tacacaaatg ataaataata acacaaatat ttataaatct   960
   agctgcaata tatttaaact agctatatcg atattgtaaa ataaaactag ctgcattgat  1020
   actgataaaa aaatatcatg tgctttctgg actgatgatg cagtatactt ttgacattgc  1080
   ctttatttta tttttcagaa aagctttctt agttctgggt tcttcattat ttgtttccca  1140
   tctccattgt gaattgaatc atttgcttcg tgtcacaaat acaatttagn taggtacatg  1200
   cattggtcag attcacggtt tattatgtca tgacttaagt tcatggtagt acattacctg  1260
   ccacgcatgc attatattgg ttagatttga taggcaaatt tggttgtcaa caatataaat 1320
   ataaataatg tttttatatt acgaaataac agtgatcaaa acaaacagtt ttatctttat 1380
   taacaagatt ttgtttttgt ttgatgacgt tttttaatgt ttacgctttc cccttctttt 1440
   tgaatttaga acactttatc atcataaaat caaatactaa aaaaattaca tatttcataa 1500
   ataataacac aaatattttt aaaaaatctg aaataataat gaacaatatt acatattatc 1560
   acgaaaattc attaataaaa atattatata aataaaatgt aatagtagtt atatgtagga 1620
   aaaaagtact gcacgcataa tatatacaaa aagattaaaa tgaactatta taaataataa 1680
   cactaaatta atggtgaatc atatcaaaat aatgaaaaag taaataaaat ttgtaattaa 1740
   cttctatatg tattacacac acaaataata aataatagta aaaaaaatta tgataaatat 1800
   ttaccatctc ataagatatt taaaataatg ataaaaatat agattatttt ttatgcaact 1860
   agctagccaa aaagagaaca cgggtatata taaaaagagt acctttaaat tctactgtac 1920
   ttcctttatt cctgacgttt ttatatcaag tggacatacg tgaagatttt aattatcagt 1980
   ctaaatattt cattagcact taatactttt ctgttttatt cctatcctat aagtagtccc 2040
   gattctccca acattgctta ttcacacaac taactaagaa agtcttccat agccccccaa 2100
   gcggccggag ctggtcatct cgctcatcgt cgagtcggcg gccggagctg gtcatctcgc 2160
   tcatcgtcga gtcggcggcc gctgagtgat tgctcacgag tgtggtcacc atgccttcag 2220
   caagtaccaa tgggttgatg atgttgtggg tttgaccctt cactcaacac ttttagtccc 2280
   ttatttctca tggaaaataa gccatcgccg ccatcactcc aacacaggtt cccttgaccg 2340
   tgatgaagtg tttgtcccaa aaccaaaatc caaagttgca tggttttcca agtacttaaa 2400
   caaccctcta ggaagggctg tttctcttct cgtcacactc acaatagggt ggcctatgta 2460
   tttagccttc aatgtctctg gtagacccta tgatagtttt gcaagccact accaccctta 2520
   tgctcccata tattctaacc gtgagaggct tctgatctat gtctctgatg ttgctttgtt 2580
   ttctgtgact tactctctct accgtgttgc aaccctgaaa gggttggttt ggctgctatg 2640
   tgtttatggg gtgcctttgc tcattgtgaa cggttttctt gtgactatca catatttgca 2700
   gcacacacac tttgccttgc ctcattacga ttcatcagaa tgggactggc tgaagggagc 2760
   tttggcaact atggacagag attaagcggc cgcatgcctc cagaaaagaa agaaattttc 2820
   aagtccttgg agggatgggc ctcggagtgg gtcctaccgc tgctgaagcc cgtggagcaa 2880
   tgctggcagc cacaaaactt cctccctgac ccctcccttc cgcatgaaga gttcagccat 2940
   caggtgaagg agcttcgcga acgcactaaa gagttacctg atgagtactt tgtggtgctg 3000
   gtgggtgata tggtcaccga ggacgcgctt cccacttacc agaccatgat caacaacctt 3060
   gatggagtga aagatgacag cggcacgagc ccgagcccgt gggccgtgtg gacccgggcc 3120
   tggaccgccg aggaaaacag acacggggat ctgctcagaa cttatttgta tctctctggg 3180
   agggttgaca tggctaaggt cgaaaagacc gtacattacc tcatttcagc tggcatggac 3240
   cctgggacag acaacaaccc atatttgggg tttgtgtaca cgtcattcca agagcgagca   3300
   acatttgtgg cgcacgggaa cacggctcgg ctcgcgaagg agggcgggga tccagtgctg   3360
   gcgcgcgcgc ctatgcggga ccatcgcagc ggacgagaag cggcacgaga acgcgtactc   3420
   aagaatcgtg gagaagcttc tggaagtgga ccccaccggg gcaatggtgg ccatagggaa   3480
   catgatggag aagaagatca cgatgccggc gcaccttatg tacgatgggg atgaccccag   3540
   gctattcgag cactactccg ctgtggcgca gcgcataggc gtgtacaccg ccaacgacta   3600
   cgcagacatc ttggatttct cgttgacggt gaagattgga gaagcttgaa ggattgatgc   3660
   ctgaggggaa gcgggcccca ggatttccgt gtgtgggttg cccccgagga ttaggaggtt   3720
   ccaagaacgc gctgatgagc gagcgcgtaa gatgaagaag catcatgccg ttaagttcag   3780
   ttggattttc aataaagaat tgcttttgtg agcggccgcc gactcgacga tgagcgagat   3840
   gaccagctcc ggccgccgac tcgacgatga gcgagatgac cagctccggc cgcgacacaa   3900
   gtgtgagagt actaaataaa tgctttggtt gtacgaaatc attacactaa ataaaataat   3960
   caaagcttat atatgccttc cgctaaggcc gaatgcaaag aaattggttc tttctcgtta   4020
   tcttttgcca cttttactag tacgtattaa ttactactta atcatctttg tttacggctc   4080
   attatatccg tcgacgg                                                  4097
<210> 16
   <211> 3964
   <212> DNA
   <213> ALS selectable marker recombinant DNA fragment
<400> 16
   ggtcgactct agtaagcttt gctctagatc aaactcacat ccaaacataa catggatatc      60
   ttccttacca atcatactaa ttattttggg ttaaatatta atcattattt ttaagatatt     120
   aattaagaaa ttaaaagatt ttttaaaaaa atgtataaaa ttatattatt catgattttt     180
   catacatttg attttgataa taaatatatt ttttttaatt tcttaaaaaa tgttgcaaga     240
   cacttattag acatagtctt gttctgttta caaaagcatt catcatttaa tacattaaaa     300
   aatatttaat actaacagta gaatcttctt gtgagtggtg tgggagtagg caacctggca     360
   ttgaaacgag agaaagagag tcagaaccag aagacaaata aaaagtatgc aacaaacaaa     420
   tcaaaatcaa agggcaaagg ctggggttgg ctcaattggt tgctacattc aattttcaac     480
   tcagtcaacg gttgagattc actctgactt ccccaatcta agccgcggat gcaaacggtt     540
   gaatctaacc cacaatccaa tctcgttact taggggcttt tccgtcatta actcacccct     600
   gccacccggt ttccctataa attggaactc aatgctcccc tctaaactcg tatcgcttca     660
   gagttgagac caagacacac tcgttcatat atctctctgc tcttctcttc tcttctacct     720
   ctcaaggtac ttttcttctc cctctaccaa atcctagatt ccgtggttca atttcggatc     780
   ttgcacttct ggtttgcttt gccttgcttt ttcctcaact gggtccatct aggatccatg     840
   tgaaactcta ctctttcttt aatatctgcg gaatacgcgt tggactttca gatctagtcg 900
   aaatcatttc ataattgcct ttctttcttt tagcttatga gaaataaaat cacttttttt 960
   ttatttcaaa ataaaccttg ggccttgtgc tgactgagat ggggtttggt gattacagaa 1020
   ttttagcgaa ttttgtaatt gtacttgttt gtctgtagtt ttgttttgtt ttcttgtttc 1080
   tcatacattc cttaggcttc aattttattc gagtataggt cacaatagga attcaaactt 1140
   tgagcagggg aattaatccc ttccttcaaa tccagtttgt ttgtatatat gtttaaaaaa 1200
   tgaaactttt gctttaaatt ctattataac tttttttatg gctgaaattt ttgcatgtgt 1260
   ctttgctctc tgttgtaaat ttactgttta ggtactaact ctaggcttgt tgtgcagttt 1320
   ttgaagtata accatgccac acaacacaat ggcggccacc gcttccagaa ccacccgatt 1380
   ctcttcttcc tcttcacacc ccaccttccc caaacgcatt actagatcca ccctccctct 1440
   ctctcatcaa accctcacca aacccaacca cgctctcaaa atcaaatgtt ccatctccaa 1500
   accccccacg gcggcgccct tcaccaagga agcgccgacc acggagccct tcgtgtcacg 1560
   gttcgcctcc ggcgaacctc gcaagggcgc ggacatcctt gtggaggcgc tggagaggca 1620
   gggcgtgacg acggtgttcg cgtaccccgg cggtgcgtcg atggagatcc accaggcgct 1680
   cacgcgctcc gccgccatcc gcaacgtgct cccgcgccac gagcagggcg gcgtcttcgc 1740
   cgccgaaggc tacgcgcgtt cctccggcct ccccggcgtc tgcattgcca cctccggccc 1800
   cggcgccacc aacctcgtga gcggcctcgc cgacgcttta atggacagcg tcccagtcgt 1860
   cgccatcacc ggccaggtcg cccgccggat gatcggcacc gacgccttcc aagaaacccc 1920
   gatcgtggag gtgagcagat ccatcacgaa gcacaactac ctcatcctcg acgtcgacga 1980
   catcccccgc gtcgtcgccg aggctttctt cgtcgccacc tccggccgcc ccggtccggt 2040
   cctcatcgac attcccaaag acgttcagca gcaactcgcc gtgcctaatt gggacgagcc 2100
   cgttaacctc cccggttacc tcgccaggct gcccaggccc cccgccgagg cccaattgga 2160
   acacattgtc agactcatca tggaggccca aaagcccgtt ctctacgtcg gcggtggcag 2220
   tttgaattcc agtgctgaat tgaggcgctt tgttgaactc actggtattc ccgttgctag 2280
   cactttaatg ggtcttggaa cttttcctat tggtgatgaa tattcccttc agatgctggg 2340
   tatgcatggt actgtttatg ctaactatgc tgttgacaat agtgatttgt tgcttgcctt 2400
   tggggtaagg tttgatgacc gtgttactgg gaagcttgag gcttttgcta gtagggctaa 2460
   gattgttcac attgatattg attctgccga gattgggaag aacaagcagg cgcacgtgtc 2520
   ggtttgcgcg gatttgaagt tggccttgaa gggaattaat atgattttgg aggagaaagg 2580
   agtggagggt aagtttgatc ttggaggttg gagagaagag attaatgtgc agaaacacaa 2640
   gtttccattg ggttacaaga cattccagga cgcgatttct ccgcagcatg ctatcgaggt 2700
   tcttgatgag ttgactaatg gagatgctat tgttagtact ggggttgggc agcatcaaat 2760
   gtgggctgcg cagttttaca agtacaagag accgaggcag tggttgacct cagggggtct    2820
   tggagccatg ggttttggat tgcctgcggc tattggtgct gctgttgcta accctggggc   2880
   tgttgtggtt gacattgatg gggatggtag tttcatcatg aatgttcagg agttggccac   2940
   tataagagtg gagaatctcc cagttaagat attgttgttg aacaatcagc atttgggtat   3000
   ggtggttcag ttggaggata ggttctacaa gtccaataga gctcacacct atcttggaga   3060
   tccgtctagc gagagcgaga tattcccaaa catgctcaag tttgctgatg cttgtgggat   3120
   accggcagcg cgagtgacga agaaggaaga gcttagagcg gcaattcaga gaatgttgga   3180
   cacccctggc ccctaccttc ttgatgtcat tgtgccccat caggagcatg tgttgccgat   3240
   gattcccagt aatggatcct tcaaggatgt gataactgag ggtgatggta gaacgaggta   3300
   ctgattgcct agaccaaatg ttccttgatg cttgttttgt acaatatata taagataatg   3360
   ctgtcctagt tgcaggattt ggcctgtggt gagcatcata gtctgtagta gttttggtag   3420
   caagacattt tattttcctt ttatttaact tactacatgc agtagcatct atctatctct   3480
   gtagtctgat atctcctgtt gtctgtattg tgccgttgga ttttttgctg tagtgagact   3540
   gaaaatgatg tgctagtaat aatatttctg ttagaaatct aagtagagaa tctgttgaag   3600
   aagtcaaaag ctaatggaat caggttacat atcaatgttt ttcttttttt agcggttggt   3660
   agacgtgtag attcaacttc tcttggagct cacctaggca atcagtaaaa tgcatattcc   3720
   ttttttaact tgccatttat ttacttttag tggaaattgt gaccaatttg ttcatgtaga   3780
   acggatttgg accattgcgt ccacaaaacg tctcttttgc tcgatcttca caaagcgata   3840
   ccgaaatcca gagatagttt tcaaaagtca gaaatggcaa agttataaat agtaaaacag   3900
   aatagatgct gtaatcgact tcaataacaa gtggcatcac gtttctagtt ctagacccgg   3960
   gtac                                                                3964
<210> 17
   <211> 656
   <212> PRT
   <213> Soybean ALS resistant to herbicides
<400> 17
   Met Pro His Asn Thr Met Ala Ala Thr Ala Ser Arg Thr Thr Arg Phe
   1               5                   10                  15
   Ser Ser Ser Ser Ser His Pro Thr Phe Pro Lys Arg Ile Thr Arg Ser
               20                  25                  30
   Thr Leu Pro Leu Ser His Gln Thr Leu Thr Lys Pro Asn His Ala Leu
           35                  40                  45
   Lys Ile Lys Cys Ser Ile Ser Lys Pro Pro Thr Ala Ala Pro Phe Thr
       50                  55                  60
   Lys Glu Ala Pro Thr Thr Glu Pro Phe Val Ser Arg Phe Ala Ser Gly
   65                  70                  75                  80
   Glu Pro Arg Lys Gly Ala Asp Ile Leu Val Glu Ala Leu Glu Arg Gln
                   85                  90                  95
   Gly Val Thr Thr Val Phe Ala Tyr Pro Gly Gly Ala Ser Met Glu Ile
               100                 105                 110
   His Gln Ala Leu Thr Arg Ser Ala Ala Ile Arg Asn Val Leu Pro Arg
           115                 120                 125
   His Glu Gln Gly Gly Val Phe Ala Ala Glu Gly Tyr Ala Arg Ser Ser
       130                 135                 140
   Gly Leu Pro Gly Val Cys Ile Ala Thr Ser Gly Pro Gly Ala Thr Asn
   145                 150                 155                 160
   Leu Val Ser Gly Leu Ala Asp Ala Leu Met Asp Ser Val Pro Val Val
                   165                 170                 175
   Ala Ile Thr Gly Gln Val Ala Arg Arg Met Ile Gly Thr Asp Ala Phe
               180                 185                 190
   Gln Glu Thr Pro Ile Val Glu Val Ser Arg Ser Ile Thr Lys His Asn
           195                 200                 205
   Tyr Leu Ile Leu Asp Val Asp Asp Ile Pro Arg Val Val Ala Glu Ala
       210                 215                 220
   Phe Phe Val Ala Thr Ser Gly Arg Pro Gly Pro Val Leu Ile Asp Ile
   225                 230                 235                 240
   Pro Lys Asp Val Gln Gln Gln Leu Ala Val Pro Asn Trp Asp Glu Pro
                   245                 250                 255
   Val Asn Leu Pro Gly Tyr Leu Ala Arg Leu Pro Arg Pro Pro Ala Glu
               260                 265                 270
   Ala Gln Leu Glu His Ile Val Arg Leu Ile Met Glu Ala Gln Lys Pro
           275                 280                 285
   Val Leu Tyr Val Gly Gly Gly Ser Leu Asn Ser Ser Ala Glu Leu Arg
       290                 295                 300
   Arg Phe Val Glu Leu Thr Gly Ile Pro Val Ala Ser Thr Leu Met Gly
   305                 310                 315                 320
   Leu Gly Thr Phe Pro Ile Gly Asp Glu Tyr Ser Leu Gln Met Leu Gly
                   325                 330                 335
   Met His Gly Thr Val Tyr Ala Asn Tyr Ala Val Asp Asn Ser Asp Leu
               340                 345                 350
   Leu Leu Ala Phe Gly Val Arg Phe Asp Asp Arg Val Thr Gly Lys Leu
           355                 360                 365
   Glu Ala Phe Ala Ser Arg Ala Lys Ile Val His Ile Asp Ile Asp Ser
       370                 375                 380
   Ala Glu Ile Gly Lys Asn Lys Gln Ala His Val Ser Val Cys Ala Asp
   385                 390                 395                 400
   Leu Lys Leu Ala Leu Lys Gly Ile Asn Met Ile Leu Glu Glu Lys Gly
                   405                 410                 415
   Val Glu Gly Lys Phe Asp Leu Gly Gly Trp Arg Glu Glu Ile Asn Val
               420                 425                 430
   Gln Lys His Lys Phe Pro Leu Gly Tyr Lys Thr Phe Gln Asp Ala Ile
           435                 440                 445
   Ser Pro Gln His Ala Ile Glu Val Leu Asp Glu Leu Thr Asn Gly Asp
       450                 455                 460
   Ala Ile Val Ser Thr Gly Val Gly Gln His Gln Met Trp Ala Ala Gln
   465                 470                 475                 480
   Phe Tyr Lys Tyr Lys Arg Pro Arg Gln Trp Leu Thr Ser Gly Gly Leu
                   485                 490                 495
   Gly Ala Met Gly Phe Gly Leu Pro Ala Ala Ile Gly Ala Ala Val Ala
               500                 505                 510
   Asn Pro Gly Ala Val Val Val Asp Ile Asp Gly Asp Gly Ser Phe Ile
           515                 520                 525
   Met Asn Val Gln Glu Leu Ala Thr Ile Arg Val Glu Asn Leu Pro Val
       530                 535                 540
   Lys Ile Leu Leu Leu Asn Asn Gln His Leu Gly Met Val Val Gln Leu
   545                 550                 555                 560
   Glu Asp Arg Phe Tyr Lys Ser Asn Arg Ala His Thr Tyr Leu Gly Asp
                   565                 570                 575
   Pro Ser Ser Glu Ser Glu Ile Phe Pro Asn Met Leu Lys Phe Ala Asp
   580 585 590
   Ala Cys Gly Ile Pro Ala Ala Arg Val Thr Lys Lys Glu Glu Leu Arg
   595 600 605
   Ala Ala Ile Gln Arg Met Leu Asp Thr Pro Gly Pro Tyr Leu Leu Asp
   610 615 620
   Val Ile Val Pro His Gln Glu His Val Leu Pro Met Ile Pro Ser Asn
   625 630 635 640
   Gly Ser Phe Lys Asp Val Ile Thr Glu Gly Asp Gly Arg Thr Arg Tyr
   645 650 655
<210> 18
   <211> 4
   <212> PRT
   <213> tobacco wild type ALS subsequence B
<400> 18
   Gly Gln Val Pro
   1
<210> 19
   <211> 10
   <212> PRT
   <213> tobacco wild type ALS subsequence F
<220>
   <221> misc_feature
   <222> (4)..(4)
   <223> Xaa can be any naturally occurring amino acid
<400> 19
   Gly Met Val Xaa Gln Trp Glu Asp Arg Phe
   1 5 10
<210> 20
   <211> 5
   <212> PRT
   <213> N-terminal amino acids added to ALS
<400> 20
   Met Pro His Asn Thr
   1 5
<210> 21
   <211> 6547
   <212> DNA
   <213> Plasmid PHP17064
<220>
   <221> misc_feature
   <222> (4842)..(4842)
   <223> n is a, c, g, or t
<220>
   <221> misc_feature
   <222> (5088)..(5088)
   <223> n is a, c, g, or t
<400> 21
   gggcgaattg ggttacccgg accggaattc gggatctgag tctagaaatc cgtcaacatg     60
   gtggagcacg acactctcgt ctactccaag aatatcaaag atacagtctc agaagaccaa    120
   agggctattg agacttttca acaaagggta atatcgggaa acctcctcgg attccattgc    180
   ccagctatct gtcacttcat caaaaggaca gtagaaaagg aaggtggcac ctacaaatgc    240
   catcattgcg ataaaggaaa ggctatcgtt caagatgcct ctgccgacag tggtcccaaa    300
   gatggacccc cacccacgag gagcatcgtg gaaaaagaag acgttccaac cacgtcttca    360
   aagcaagtgg attgatgtga tgatcctatg cgtatggtat gacgtgtgtt caagatgatg    420
   acttcaaacc tacctatgac gtatggtatg aacgtgtgtc gactgatgac ttagatccac    480
   tcgagcggct ataaatacgt acctacgcac cctgcgctac catccctaga gctgcagctt    540
   atttttacaa caattaccaa caacaacaaa caacaaacaa cattacaatt actatttaca    600
   attacagtcg acccgtaccc acacaacaca atggcggcca ccgcttccag aaccacccga    660
   ttctcttctt cctcttcaca ccccaccttc cccaaacgca ttactagatc caccctccct    720
   ctctctcatc aaaccctcac caaacccaac cacgctctca aaatcaaatg ttccatctcc    780
   aaacccccca cggcggcgcc cttcaccaag gaagcgccga ccacggagcc cttcgtgtca    840
   cggttcgcct ccggcgaacc tcgcaagggc gcggacatcc ttgtggaggc gctggagagg    900
   cagggcgtga cgacggtgtt cgcgtacccc ggcggtgcgt cgatggagat ccaccaggcg    960
   ctcacgcgct ccgccgccat ccgcaacgtg ctcccgcgcc acgagcaggg cggcgtcttc   1020
   gccgccgaag gctacgcgcg ttcctccggc ctccccggcg tctgcattgc cacctccggc   1080
   cccggcgcca ccaacctcgt gagcggcctc gccgacgctt taatggacag cgtcccagtc   1140
   gtcgccatca ccggccaggt cgcccgccgg atgatcggca ccgacgcctt ccaagaaacc   1200
   ccgatcgtgg aggtgagcag atccatcacg aagcacaact acctcatcct cgacgtcgac   1260
   gacatccccc gcgtcgtcgc cgaggctttc ttcgtcgcca cctccggccg ccccggtccg   1320
   gtcctcatcg acattcccaa agacgttcag cagcaactcg ccgtgcctaa ttgggacgag   1380
   cccgttaacc tccccggtta cctcgccagg ctgcccaggc cccccgccga ggcccaattg   1440
   gaacacattg tcagactcat catggaggcc caaaagcccg ttctctacgt cggcggtggc   1500
   agtttgaatt ccagtgctga attgaggcgc tttgttgaac tcactggtat tcccgttgct   1560
   agcactttaa tgggtcttgg aacttttcct attggtgatg aatattccct tcagatgctg   1620
   ggtatgcatg gtactgttta tgctaactat gctgttgaca atagtgattt gttgcttgcc   1680
   tttggggtaa ggtttgatga ccgtgttact gggaagcttg aggcttttgc tagtagggct 1740
   aagattgttc acattgatat tgattctgcc gagattggga agaacaagca ggcgcacgtg 1800
   tcggtttgcg cggatttgaa gttggccttg aagggaatta atatgatttt ggaggagaaa 1860
   ggagtggagg gtaagtttga tcttggaggt tggagagaag agattaatgt gcagaaacac 1920
   aagtttccat tgggttacaa gacattccag gacgcgattt ctccgcagca tgctatcgag 1980
   gttcttgatg agttgactaa tggagatgct attgttagta ctggggttgg gcagcatcaa 2040
   atgtgggctg cgcagtttta caagtacaag agaccgaggc agtggttgac ctcagggggt 2100
   cttggagcca tgggttttgg attgcctgcg gctattggtg ctgctgttgc taaccctggg 2160
   gctgttgtgg ttgacattga tggggatggt agtttcatca tgaatgttca ggagttggcc 2220
   actataagag tggagaatct cccagttaag atattgttgt tgaacaatca gcatttgggt 2280
   atggtggttc agttggagga taggttctac aagtccaata gagctcacac ctatcttgga 2340
   gatccgtcta gcgagagcga gatattccca aacatgctca agtttgctga tgcttgtggg 2400
   ataccggcag cgcgagtgac gaagaaggaa gagcttagag cggcaattca gagaatgttg 2460
   gacacccctg gcccctacct tcttgatgtc attgtgcccc atcaggagca tgtgttgccg 2520
   atgattccca gtaatggatc cttcaaggat gtgataactg agggtgatgg tagaacgagg 2580
   tactgattgc ctagaccaaa tgttccttga tgcttgtttt gtacaatata tataagataa 2640
   tgctgtccta gttgcaggat ttggcctgtg gtgagcatca tagtctgtag tagttttggt 2700
   agcaagacat tttattttcc ttttatttaa cttactacat gcagtagcat ctatctatct 2760
   ctgtagtctg atatctcctg ttgtctgtat tgtgccgttg gattttttgc tgtagtgaga 2820
   ctgaaaatga tgtgctagta ataatatttc tgttagaaat ctaagtagag aatctgttga 2880
   agaagtcaaa agctaatgga atcaggttac atatcaatgt ttttcttttt ttagcggttg 2940
   gtagacgtgt agattcaact tctcttggag ctcacctagg caatcagtaa aatgcatatt 3000
   ccttttttaa cttgccattt atttactttt agtggaaatt gtgaccaatt tgttcatgta 3060
   gaacggattt ggaccattgc gtccacaaaa cgtctctttt gctcgatctt cacaaagcga 3120
   taccgaaatc cagagatagt tttcaaaagt cagaaatggc aaagttataa atagtaaaac 3180
   agaatagatg ctgtaatcga cttcaataac aagtggcatc acgtttctag ttctagaccc 3240
   gggtctagag tcgacctgca ggcatgcccg cggatatcga tgggccccgg ccgaagcttc 3300
   ggtccgggtc acccagcttg agtattctat agtgtcacct aaatagcttg gcgtaatcat 3360
   ggtcatagct gtttcctgtg tgaaattgtt atccgctcac aattccacac aacatacgag 3420
   ccggaagcat aaagtgtaaa gcctggggtg cctaatgagt gagctaactc acattaattg 3480
   cgttgcgctc actgcccgct ttccagtcgg gaaacctgtc gtgccagctg cattaatgaa 3540
   tcggccaacg cgcggggaga ggcggtttgc gtattgggcg ctcttccgct tcctcgctca 3600
   ctgactcgct gcgctcggtc gttcggctgc ggcgagcggt atcagctcac tcaaaggcgg 3660
   taatacggtt atccacagaa tcaggggata acgcaggaaa gaacatgtga gcaaaaggcc 3720
   agcaaaaggc caggaaccgt aaaaaggccg cgttgctggc gtttttcgat aggctccgcc 3780
   cccctgacga gcatcacaaa aatcgacgct caagtcagag gtggcgaaac ccgacaggac 3840
   tataaagata ccaggcgttt ccccctggaa gctccctcgt gcgctctcct gttccgaccc 3900
   tgccgcttac cggatacctg tccgcctttc tcccttcggg aagcgtggcg ctttctcata 3960
   gctcacgctg taggtatctc agttcggtgt aggtcgttcg ctccaagctg ggctgtgtgc 4020
   acgaaccccc cgttcagccc gaccgctgcg ccttatccgg taactatcgt cttgagtcca 4080
   acccggtaag acacgactta tcgccactgg cagcagccac tggtaacagg attagcagag 4140
   cgaggtatgt aggcggtgct acagagttct tgaagtggtg gcctaactac ggctacacta 4200
   gaaggacagt atttggtatc tgcgctctgc tgaagccagt taccttcgga aaaagagttg 4260
   gtagctcttg atccggcaaa caaaccaccg ctggtagcgg tggttttttt gtttgcaagc 4320
   agcagattac gcgcagaaaa aaaggatctc aagaagatcc tttgatcttt tctacggggt 4380
   ctgacgctca gtggaacgaa aactcacgtt aagggatttt ggtcatggag ccacgttgtg 4440
   tctcaaaatc tctgatgtta cattgcacaa gataaaaata tatcatcatg aacaataaaa 4500
   ctgtctgctt acataaacag taatacaagg ggtgttatga gccatattca acgggaaacg 4560
   tcttgctcga ggccgcgatt aaattccaac atggatgctg atttatatgc ctataaatgg 4620
   gctcgcgata atgtcggcca atcaggtccg acaatctatc gattgtatgg gaagcccgat 4680
   gcgccagact tgtttctgaa acatggcaaa ggtagccttg ccaatgatgt tacagatgag 4740
   atggtcagac taaactgcct gacggaattt atgcctcttc cgaccatcaa gcattttatc 4800
   cgtactcctg atgatgcatg gttactcacc actgcgatcc cngggaaaac agcattccag 4860
   gtattagaag aatatcctga ttcaggtgaa aatattgttg atgcgctggc agtgttcctg 4920
   cgccggttgc attcgattcc tctttgtaat tgtcctttta acagcgatcg cgtatttcgt 4980
   ctcgctcagg cgcaatcacg aatgaataac ggtttggttg atgcgagtga ttttgatgac 5040
   gagcgtaatg gctggcctgt tgaacaagtc tggaaagaaa tgcataanct tttgccattc 5100
   tcaccggatt cagtcgtcac tcatggtgat ttctcacttg ataaccttat ttttgaccag 5160
   gcgaaattaa taggttgtat tgatcttcga cgagtcggaa tcgcagaccg ataccaggat 5220
   cttgccatcc tatggaactg cctcggtgag ttttctcctt cattacagaa acggcttttt 5280
   caaaaatatg gtattgataa tcctgatatg aataaattgc agtttcattt gatcctcgat 5340
   gagtttttct aatcagaatt ggttaattgg ttgtaacact ggcagagcat tacgctgact 5400
   tgacgggacg gcggctttgt tgaataaatc gaacttttgc tgacttgaag gatcagatca 5460
   cgcatcttcc cgacaacgca gaccgttccg tggcaaagca aaagttcaaa atcaccaact 5520
   ggtccaccta caacaaagct ctcatcaacc gtggctccct cactttctgg ctggatgatg 5580
   gggcgattca ggcctggtat gagtcagcaa caccttcttc acgagccatg acattaacct 5640
   ataaaaatag gcgtatcacg aggccctttc gtctcgcgcg tttcggtgat gacggtgaaa 5700
   acctctgaca catgcagctc ccggagacgg tcacagcttg tctgtaagcg gatgccggga 5760
   gcagacaagc ccgtcagggc gcgtcagcgg gtgttggcgg gtgtcggggc tggcttaact 5820
   atgcggcatc agagcagatt gtactgagag tgcaccatat gcggtgtgaa ataccgcaca 5880
   gatgcgtaag gagaaaatac cgcatcaggc gaaattgtaa acgttaatat tttgttaaaa 5940
   ttcgcgttaa atatttgtta aatcagctca ttttttaacc aataggccga aatcggcaaa 6000
   atcccttata aatcaaaaga atagaccgag atagggttga gtgttgttcc agtttggaac 6060
   aagagtccac tattaaagaa cgtggactcc aacgtcaaag ggcgaaaaac cgtctatcag 6120
   ggcgatggcc cactacgtga accatcaccc aaatcaagtt ttttgcggtc gaggtgccgt 6180
   aaagctctaa atcggaaccc taaagggagc ccccgattta gagcttgacg gggaaagccg 6240
   gcgaacgtgg cgagaaagga agggaagaaa gcgaaaggag cgggcgctag ggcgctggca 6300
   agtgtagcgg tcacgctgcg cgtaaccacc acacccgccg cgcttaatgc gccgctacag 6360
   ggcgcgtcca ttcgccattc aggctgcgca actgttggga agggcgatcg gtgcgggcct 6420
   cttcgctatt acgccagctg gcgaaagggg gatgtgctgc aaggcgatta agttgggtaa 6480
   cgccagggtt ttcccagtca cgacgttgta aaacgacggc cagtgaattg taatacgact 6540
   cactata 6547
<210> 22
   <211> 3191
   <212> DNA
   <213> Recombinant DNA fragment PHP17064A
<400> 22
   ctagaaatcc gtcaacatgg tggagcacga cactctcgtc tactccaaga atatcaaaga     60
   tacagtctca gaagaccaaa gggctattga gacttttcaa caaagggtaa tatcgggaaa    120
   cctcctcgga ttccattgcc cagctatctg tcacttcatc aaaaggacag tagaaaagga    180
   aggtggcacc tacaaatgcc atcattgcga taaaggaaag gctatcgttc aagatgcctc    240
   tgccgacagt ggtcccaaag atggaccccc acccacgagg agcatcgtgg aaaaagaaga    300
   cgttccaacc acgtcttcaa agcaagtgga ttgatgtgat gatcctatgc gtatggtatg    360
   acgtgtgttc aagatgatga cttcaaacct acctatgacg tatggtatga acgtgtgtcg    420
   actgatgact tagatccact cgagcggcta taaatacgta cctacgcacc ctgcgctacc    480
   atccctagag ctgcagctta tttttacaac aattaccaac aacaacaaac aacaaacaac    540
   attacaatta ctatttacaa ttacagtcga cccgtaccca cacaacacaa tggcggccac    600
   cgcttccaga accacccgat tctcttcttc ctcttcacac cccaccttcc ccaaacgcat    660
   tactagatcc accctccctc tctctcatca aaccctcacc aaacccaacc acgctctcaa    720
   aatcaaatgt tccatctcca aaccccccac ggcggcgccc ttcaccaagg aagcgccgac 780
   cacggagccc ttcgtgtcac ggttcgcctc cggcgaacct cgcaagggcg cggacatcct 840
   tgtggaggcg ctggagaggc agggcgtgac gacggtgttc gcgtaccccg gcggtgcgtc 900
   gatggagatc caccaggcgc tcacgcgctc cgccgccatc cgcaacgtgc tcccgcgcca 960
   cgagcagggc ggcgtcttcg ccgccgaagg ctacgcgcgt tcctccggcc tccccggcgt 1020
   ctgcattgcc acctccggcc ccggcgccac caacctcgtg agcggcctcg ccgacgcttt 1080
   aatggacagc gtcccagtcg tcgccatcac cggccaggtc gcccgccgga tgatcggcac 1140
   cgacgccttc caagaaaccc cgatcgtgga ggtgagcaga tccatcacga agcacaacta 1200
   cctcatcctc gacgtcgacg acatcccccg cgtcgtcgcc gaggctttct tcgtcgccac 1260
   ctccggccgc cccggtccgg tcctcatcga cattcccaaa gacgttcagc agcaactcgc 1320
   cgtgcctaat tgggacgagc ccgttaacct ccccggttac ctcgccaggc tgcccaggcc 1380
   ccccgccgag gcccaattgg aacacattgt cagactcatc atggaggccc aaaagcccgt 1440
   tctctacgtc ggcggtggca gtttgaattc cagtgctgaa ttgaggcgct ttgttgaact 1500
   cactggtatt cccgttgcta gcactttaat gggtcttgga acttttccta ttggtgatga 1560
   atattccctt cagatgctgg gtatgcatgg tactgtttat gctaactatg ctgttgacaa 1620
   tagtgatttg ttgcttgcct ttggggtaag gtttgatgac cgtgttactg ggaagcttga 1680
   ggcttttgct agtagggcta agattgttca cattgatatt gattctgccg agattgggaa 1740
   gaacaagcag gcgcacgtgt cggtttgcgc ggatttgaag ttggccttga agggaattaa 1800
   tatgattttg gaggagaaag gagtggaggg taagtttgat cttggaggtt ggagagaaga 1860
   gattaatgtg cagaaacaca agtttccatt gggttacaag acattccagg acgcgatttc 1920
   tccgcagcat gctatcgagg ttcttgatga gttgactaat ggagatgcta ttgttagtac 1980
   tggggttggg cagcatcaaa tgtgggctgc gcagttttac aagtacaaga gaccgaggca 2040
   gtggttgacc tcagggggtc ttggagccat gggttttgga ttgcctgcgg ctattggtgc 2100
   tgctgttgct aaccctgggg ctgttgtggt tgacattgat ggggatggta gtttcatcat 2160
   gaatgttcag gagttggcca ctataagagt ggagaatctc ccagttaaga tattgttgtt 2220
   gaacaatcag catttgggta tggtggttca gttggaggat aggttctaca agtccaatag 2280
   agctcacacc tatcttggag atccgtctag cgagagcgag atattcccaa acatgctcaa 2340
   gtttgctgat gcttgtggga taccggcagc gcgagtgacg aagaaggaag agcttagagc 2400
   ggcaattcag agaatgttgg acacccctgg cccctacctt cttgatgtca ttgtgcccca 2460
   tcaggagcat gtgttgccga tgattcccag taatggatcc ttcaaggatg tgataactga 2520
   gggtgatggt agaacgaggt actgattgcc tagaccaaat gttccttgat gcttgttttg 2580
   tacaatatat ataagataat gctgtcctag ttgcaggatt tggcctgtgg tgagcatcat 2640
   agtctgtagt agttttggta gcaagacatt ttattttcct tttatttaac ttactacatg 2700
   cagtagcatc tatctatctc tgtagtctga tatctcctgt tgtctgtatt gtgccgttgg 2760
   attttttgct gtagtgagac tgaaaatgat gtgctagtaa taatatttct gttagaaatc 2820
   taagtagaga atctgttgaa gaagtcaaaa gctaatggaa tcaggttaca tatcaatgtt 2880
   tttctttttt tagcggttgg tagacgtgta gattcaactt ctcttggagc tcacctaggc 2940
   aatcagtaaa atgcatattc cttttttaac ttgccattta tttactttta gtggaaattg 3000
   tgaccaattt gttcatgtag aacggatttg gaccattgcg tccacaaaac gtctcttttg 3060
   ctcgatcttc acaaagcgat accgaaatcc agagatagtt ttcaaaagtc agaaatggca 3120
   aagttataaa tagtaaaaca gaatagatgc tgtaatcgac ttcaataaca agtggcatca 3180
   cgtttctagt t 3191
<210> 23
   <211> 2924
   <212> DNA
   <213> artificial sequence
<220>
   <223> fragment PHP19340A
<220>
   <221> misc_feature
   <222> (1191)..(1191)
   <223> n is a, c, g, or t
<400> 23
   cgcgccaagc ttggatcctc gaagagaagg gttaataaca cactttttta acatttttaa      60
   cacaaatttt agttatttaa aaatttatta aaaaatttaa aataagaaga ggaactcttt     120
   aaataaatct aacttacaaa atttatgatt tttaataagt tttcaccaat aaaaaatgtc     180
   ataaaaatat gttaaaaagt atattatcaa tattctcttt atgataaata aaaagaaaaa     240
   aaaaataaaa gttaagtgaa aatgagattg aagtgacttt aggtgtgtat aaatatatca     300
   accccgccaa caatttattt aatccaaata tattgaagta tattattcca tagcctttat     360
   ttatttatat atttattata taaaagcttt atttgttcta ggttgttcat gaaatatttt     420
   tttggtttta tctccgttgt aagaaaatca tgtgctttgt gtcgccactc actattgcag     480
   ctttttcatg cattggtcag attgacggtt gattgtattt ttgtttttta tggttttgtg     540
   ttatgactta agtcttcatc tctttatctc ttcatcaggt ttgatggtta cctaatatgg     600
   tccatgggta catgcatggt taaattaggt ggccaacttt gttgtgaacg atagaatttt     660
   ttttatatta agtaaactat ttttatatta tgaaataata ataaaaaaaa tattttatca     720
   ttattaacaa aatcatatta gttaatttgt taactctata ataaaagaaa tactgtaaca     780
   ttcacattac atggtaacat ctttccaccc tttcatttgt tttttgtttg atgacttttt     840
   ttcttgttta aatttatttc ccttctttta aatttggaat acattatcat catatataaa     900
   ctaaaatact aaaaacagga ttacacaaat gataaataat aacacaaata tttataaatc      960
   tagctgcaat atatttaaac tagctatatc gatattgtaa aataaaacta gctgcattga     1020
   tactgataaa aaaatatcat gtgctttctg gactgatgat gcagtatact tttgacattg     1080
   cctttatttt atttttcaga aaagctttct tagttctggg ttcttcatta tttgtttccc     1140
   atctccattg tgaattgaat catttgcttc gtgtcacaaa tacaatttag ntaggtacat     1200
   gcattggtca gattcacggt ttattatgtc atgacttaag ttcatggtag tacattacct     1260
   gccacgcatg cattatattg gttagatttg ataggcaaat ttggttgtca acaatataaa     1320
   tataaataat gtttttatat tacgaaataa cagtgatcaa aacaaacagt tttatcttta     1380
   ttaacaagat tttgtttttg tttgatgacg ttttttaatg tttacgcttt cccccttctt    1440
   ttgaatttag aacactttat catcataaaa tcaaatacta aaaaaattac atatttcata     1500
   aataataaca caaatatttt taaaaaatct gaaataataa tgaacaatat tacatattat     1560
   cacgaaaatt cattaataaa aatattatat aaataaaatg taatagtagt tatatgtagg     1620
   aaaaaagtac tgcacgcata atatatacaa aaagattaaa atgaactatt ataaataata     1680
   acactaaatt aatggtgaat catatcaaaa taatgaaaaa gtaaataaaa tttgtaatta     1740
   acttctatat gtattacaca cacaaataat aaataatagt aaaaaaaatt atgataaata     1800
   tttaccatct cataagatat ttaaaataat gataaaaata tagattattt tttatgcaac     1860
   tagctagcca aaaagagaac acgggtatat ataaaaagag tacctttaaa ttctactgta     1920
   cttcctttat tcctgacgtt tttatatcaa gtggacatac gtgaagattt taattatcag     1980
   tctaaatatt tcattagcac ttaatacttt tctgttttat tcctatccta taagtagtcc     2040
   cgattctccc aacattgctt attcacacaa ctaactaaga aagtcttcca tagcccccca     2100
   agcggccgct gagtgattgc tcacgagtgt ggtcaccatg ccttcagcaa gtaccaatgg     2160
   gttgatgatg ttgtgggttt gacccttcac tcaacacttt tagtccctta tttctcatgg     2220
   aaaataagcc atcgccgcca tcactccaac acaggttccc ttgaccgtga tgaagtgttt     2280
   gtcccaaaac caaaatccaa agttgcatgg ttttccaagt acttaaacaa ccctctagga     2340
   agggctgttt ctcttctcgt cacactcaca atagggtggc ctatgtattt agccttcaat     2400
   gtctctggta gaccctatga tagttttgca agccactacc acccttatgc tcccatatat     2460
   tctaaccgtg agaggcttct gatctatgtc tctgatgttg ctttgttttc tgtgacttac     2520
   tctctctacc gtgttgcaac cctgaaaggg ttggtttggc tgctatgtgt ttatggggtg     2580
   cctttgctca ttgtgaacgg ttttcttgtg actatcacat atttgcagca cacacacttt     2640
   gccttgcctc attacgattc atcagaatgg gactggctga agggagcttt ggcaactatg     2700
   gacagagatt aagcggccgc gacacaagtg tgagagtact aaataaatgc tttggttgta     2760
   cgaaatcatt acactaaata aaataatcaa agcttatata tgccttccgc taaggccgaa     2820
   tgcaaagaaa ttggttcttt ctcgttatct tttgccactt ttactagtac gtattaatta     2880
   ctacttaatc atctttgttt acggctcatt atatccgtcg acgg 2924
<210> 24
   <211> 4511
   <212> DNA
   <213> artificial sequence
<220>
   <223> fragment PHP17752
<400> 24
   cgcgccaagc ttggatcccc cctcgaggtc gacggtatcg ataagcttct gcaggaattc 60
   tgagctagcg aagttcctat tccgaagttc ctattcttca aaaagtatag gaacttcaga 120
   cgtcctcgag tccgtcctgt agaaacccca acccgtgaaa tcaaaaaact cgacggcctg 180
   tgggcattca gtctggatcg cgaaaactgt ggaattgatc cagaattcgc tagcgaagtt 240
   cctattccga agttcctatt ctctagaaag tataggaact tcagatccag aattcggtcc 300
   gggccatcgt ggcctcttgc tcttcaggat gaagagctat gtttcgcgcc aagcttggat 360
   cctagaacta gaaacgtgat gccacttgtt attgaagtcg attacagcat ctattctgtt 420
   ttactattta taactttgcc atttctgact tttgaaaact atctctggat ttcggtatcg 480
   ctttgtgaag atcgagcaaa agagacgttt tgtggacgca atggtccaaa tccgttctac 540
   atgaacaaat tggtcacaat ttccactaaa agtaaataaa tggcaagtta aaaaaggaat 600
   atgcatttta ctgattgcct aggtgagctc caagagaagt tgaatctaca cgtctaccaa 660
   ccgctaaaaa aagaaaaaca ttgatatgta acctgattcc attagctttt gacttcttca 720
   acagattctc tacttagatt tctaacagaa atattattac tagcacatca ttttcagtct 780
   cactacagca aaaaatccaa cggcacaata cagacaacag gagatatcag actacagaga 840
   tagatagatg ctactgcatg tagtaagtta aataaaagga aaataaaatg tcttgctacc 900
   aaaactacta cagactatga tgctcaccac aggccaaatc ctgcaactag gacagcatta 960
   tcttatatat attgtacaaa acaagcatca aggaacattt ggtctaggca atcagtacct 1020
   cgttctacca tcaccctcag ttatcacatc cttgaaggat ccattactgg gaatcatcgg 1080
   caacacatgc tcctgatggg gcacaatgac atcaagaagg taggggccag gggtgtccaa 1140
   cattctctga attgccgctc taagctcttc cttcttcgtc actcgcgctg ccggtatccc 1200
   acaagcatca gcaaacttga gcatgtttgg gaatatctcg ctctcgctag acggatctcc 1260
   aagataggtg tgagctctat tggacttgta gaacctatcc tccaactgaa ccaccatacc 1320
   caaatgctga ttgttcaaca acaatatctt aactgggaga ttctccactc ttatagtggc 1380
   caactcctga acattcatga tgaaactacc atccccatca atgtcaacca caacagcccc 1440
   agggttagca acagcagcac caatagccgc aggcaatcca aaacccatgg ctccaagacc 1500
   ccctgaggtc aaccactgcc tcggtctctt gtacttgtaa aactgcgcag cccacatttg 1560
   atgctgccca accccagtac taacaatagc atctccatta gtcaactcat caagaacctc 1620
   gatagcatgc tgcggagaaa tcgcgtcctg gaatgtcttg taacccaatg gaaacttgtg 1680
   tttctgcaca ttaatctctt ctctccaacc tccaagatca aacttaccct ccactccttt 1740
   ctcctccaaa atcatattaa ttcccttcaa ggccaacttc aaatccgcgc aaaccgacac 1800
   gtgcgcctgc ttgttcttcc caatctcggc agaatcaata tcaatgtgaa caatcttagc 1860
   cctactagca aaagcctcaa gcttcccagt aacacggtca tcaaacctta ccccaaaggc 1920
   aagcaacaaa tcactattgt caacagcata gttagcataa acagtaccat gcatacccag 1980
   catctgaagg gaatattcat caccaatagg aaaagttcca agacccatta aagtgctagc 2040
   aacgggaata ccagtgagtt caacaaagcg cctcaattca gcactggaat tcaaactgcc 2100
   accgccgacg tagagaacgg gcttttgggc ctccatgatg agtctgacaa tgtgttccaa 2160
   ttgggcctcg gcggggggcc tgggcagcct ggcgaggtaa ccggggaggt taacgggctc 2220
   gtcccaatta ggcacggcga gttgctgctg aacgtctttg ggaatgtcga tgaggaccgg 2280
   accggggcgg ccggaggtgg cgacgaagaa agcctcggcg acgacgcggg ggatgtcgtc 2340
   gacgtcgagg atgaggtagt tgtgcttcgt gatggatctg ctcacctcca cgatcggggt 2400
   ttcttggaag gcgtcggtgc cgatcatccg gcgggcgacc tggccggtga tggcgacgac 2460
   tgggacgctg tccattaaag cgtcggcgag gccgctcacg aggttggtgg cgccggggcc 2520
   ggaggtggca atgcagacgc cggggaggcc ggaggaacgc gcgtagcctt cggcggcgaa 2580
   gacgccgccc tgctcgtggc gcgggagcac gttgcggatg gcggcggagc gcgtgagcgc 2640
   ctggtggatc tccatcgacg caccgccggg gtacgcgaac accgtcgtca cgccctgcct 2700
   ctccagcgcc tccacaagga tgtccgcgcc cttgcgaggt tcgccggagg cgaaccgtga 2760
   cacgaagggc tccgtggtcg gcgcttcctt ggtgaagggc gccgccgtgg ggggtttgga 2820
   gatggaacat ttgattttga gagcgtggtt gggtttggtg agggtttgat gagagagagg 2880
   gagggtggat ctagtaatgc gtttggggaa ggtggggtgt gaagaggaag aagagaatcg 2940
   ggtggttctg gaagcggtgg ccgccattgt gttgtgtggc atggttatac ttcaaaaact 3000
   gcacaacaag cctagagtta gtacctaaac agtaaattta caacagagag caaagacaca 3060
   tgcaaaaatt tcagccataa aaaaagttat aatagaattt aaagcaaaag tttcattttt 3120
   taaacatata tacaaacaaa ctggatttga aggaagggat taattcccct gctcaaagtt 3180
   tgaattccta ttgtgaccta tactcgaata aaattgaagc ctaaggaatg tatgagaaac 3240
   aagaaaacaa aacaaaacta cagacaaaca agtacaatta caaaattcgc taaaattctg 3300
   taatcaccaa accccatctc agtcagcaca aggcccaagg tttattttga aataaaaaaa 3360
   aagtgatttt atttctcata agctaaaaga aagaaaggca attatgaaat gatttcgact 3420
   agatctgaaa gtccaacgcg tattccgcag atattaaaga aagagtagag tttcacatgg 3480
   atcctagatg gacccagttg aggaaaaagc aaggcaaagc aaaccagaag tgcaagatcc 3540
   gaaattgaac cacggaatct aggatttggt agagggagaa gaaaagtacc ttgagaggta     3600
   gaagagaaga gaagagcaga gagatatatg aacgagtgtg tcttggtctc aactctgaag     3660
   cgatacgagt ttagagggga gcattgagtt ccaatttata gggaaaccgg gtggcagggg     3720
   tgagttaatg acggaaaagc ccctaagtaa cgagattgga ttgtgggtta gattcaaccg     3780
   tttgcatccg cggcttagat tggggaagtc agagtgaatc tcaaccgttg actgagttga     3840
   aaattgaatg tagcaaccaa ttgagccaac cccagccttt gccctttgat tttgatttgt     3900
   ttgttgcata ctttttattt gtcttctggt tctgactctc tttctctcgt ttcaatgcca     3960
   ggttgcctac tcccacacca ctcacaagaa gattctactg ttagtattaa atatttttta      4020
   atgtattaaa tgatgaatgc ttttgtaaac agaacaagac tatgtctaat aagtgtcttg     4080
   caacattttt taagaaatta aaaaaaatat atttattatc aaaatcaaat gtatgaaaaa     4140
   tcatgaataa tataatttta tacatttttt taaaaaatct tttaatttct taattaatat     4200
   cttaaaaata atgattaata tttaacccaa aataattagt atgattggta aggaagatat     4260
   ccatgttatg tttggatgtg agtttgatct agagcaaagc ttactagagt cgaccgatcc     4320
   gtcgacggcg cgcgcgcctc tagttgaaga cacgttcatg tcttcatcgt aagaagacac     4380
   tcagtagtct tcggccagaa tggcccggac cgaagcttct gcaggaattc tgagctagcg     4440
   aagttcctat tccgaagttc ctattctcta gaaagtatag gaacttcaga tccactagga     4500
   tccgtcgacg g                                                          4511
<210> 25
   <211> 5437
   <212> DNA
   <213> artificial sequence
<220>
   <223> plasmid PHP19340
<220>
   <221> misc_feature
   <222> (3914)..(3914)
   <223> n is a, c, g, or t
<400> 25
   ggccgcgaca caagtgtgag agtactaaat aaatgctttg gttgtacgaa atcattacac      60
   taaataaaat aatcaaagct tatatatgcc ttccgctaag gccgaatgca aagaaattgg     120
   ttctttctcg ttatcttttg ccacttttac tagtacgtat taattactac ttaatcatct     180
   ttgtttacgg ctcattatat ccgtcgacgg cgcgcccgat catccggata tagttcctcc     240
   tttcagcaaa aaacccctca agacccgttt agaggcccca aggggttatg ctagttattg     300
   ctcagcggtg gcagcagcca actcagcttc ctttcgggct ttgttagcag ccggatcgat     360
   ccaagctgta cctcactatt cctttgccct cggacgagtg ctggggcgtc ggtttccact     420
   atcggcgagt acttctacac agccatcggt ccagacggcc gcgcttctgc gggcgatttg 480
   tgtacgcccg acagtcccgg ctccggatcg gacgattgcg tcgcatcgac cctgcgccca 540
   agctgcatca tcgaaattgc cgtcaaccaa gctctgatag agttggtcaa gaccaatgcg 600
   gagcatatac gcccggagcc gcggcgatcc tgcaagctcc ggatgcctcc gctcgaagta 660
   gcgcgtctgc tgctccatac aagccaacca cggcctccag aagaagatgt tggcgacctc 720
   gtattgggaa tccccgaaca tcgcctcgct ccagtcaatg accgctgtta tgcggccatt 780
   gtccgtcagg acattgttgg agccgaaatc cgcgtgcacg aggtgccgga cttcggggca 840
   gtcctcggcc caaagcatca gctcatcgag agcctgcgcg acggacgcac tgacggtgtc 900
   gtccatcaca gtttgccagt gatacacatg gggatcagca atcgcgcata tgaaatcacg 960
   ccatgtagtg tattgaccga ttccttgcgg tccgaatggg ccgaacccgc tcgtctggct 1020
   aagatcggcc gcagcgatcg catccatagc ctccgcgacc ggctgcagaa cagcgggcag 1080
   ttcggtttca ggcaggtctt gcaacgtgac accctgtgca cggcgggaga tgcaataggt 1140
   caggctctcg ctgaattccc caatgtcaag cacttccgga atcgggagcg cggccgatgc 1200
   aaagtgccga taaacataac gatctttgta gaaaccatcg gcgcagctat ttacccgcag 1260
   gacatatcca cgccctccta catcgaagct gaaagcacga gattcttcgc cctccgagag 1320
   ctgcatcagg tcggagacgc tgtcgaactt ttcgatcaga aacttctcga cagacgtcgc 1380
   ggtgagttca ggcttttcca tgggtatatc tccttcttaa agttaaacaa aattatttct 1440
   agagggaaac cgttgtggtc tccctatagt gagtcgtatt aatttcgcgg gatcgagatc 1500
   tgatcaacct gcattaatga atcggccaac gcgcggggag aggcggtttg cgtattgggc 1560
   gctcttccgc ttcctcgctc actgactcgc tgcgctcggt cgttcggctg cggcgagcgg 1620
   tatcagctca ctcaaaggcg gtaatacggt tatccacaga atcaggggat aacgcaggaa 1680
   agaacatgtg agcaaaaggc cagcaaaagg ccaggaaccg taaaaaggcc gcgttgctgg 1740
   cgtttttcca taggctccgc ccccctgacg agcatcacaa aaatcgacgc tcaagtcaga 1800
   ggtggcgaaa cccgacagga ctataaagat accaggcgtt tccccctgga agctccctcg 1860
   tgcgctctcc tgttccgacc ctgccgctta ccggatacct gtccgccttt ctcccttcgg 1920
   gaagcgtggc gctttctcaa tgctcacgct gtaggtatct cagttcggtg taggtcgttc 1980
   gctccaagct gggctgtgtg cacgaacccc ccgttcagcc cgaccgctgc gccttatccg 2040
   gtaactatcg tcttgagtcc aacccggtaa gacacgactt atcgccactg gcagcagcca 2100
   ctggtaacag gattagcaga gcgaggtatg taggcggtgc tacagagttc ttgaagtggt 2160
   ggcctaacta cggctacact agaaggacag tatttggtat ctgcgctctg ctgaagccag 2220
   ttaccttcgg aaaaagagtt ggtagctctt gatccggcaa acaaaccacc gctggtagcg 2280
   gtggtttttt tgtttgcaag cagcagatta cgcgcagaaa aaaggatctc caagaagatc 2340
   ctttgatctt ttctacgggg tctgacgctc agtggaacga aaactcacgt taagggattt 2400
   tggtcatgac attaacctat aaaaataggc gtatcacgag gccctttcgt ctcgcgcgtt 2460
   tcggtgatga cggtgaaaac ctctgacaca tgcagctccc ggagacggtc acagcttgtc 2520
   tgtaagcgga tgccgggagc agacaagccc gtcagggcgc gtcagcgggt gttggcgggt 2580
   gtcggggctg gcttaactat gcggcatcag agcagattgt actgagagtg caccatatgg 2640
   acatattgtc gttagaacgc ggctacaatt aatacataac cttatgtatc atacacatac 2700
   gatttaggtg acactataga acggcgcgcc aagcttggat cctcgaagag aagggttaat 2760
   aacacatttt ttaacatttt taacacaaat tttagttatt taaaaattta ttaaaaaatt 2820
   taaaataaga agaggaactc tttaaataaa tctaacttac aaaatttatg atttttaata 2880
   agttttcacc aataaaaaat gtcataaaaa tatgttaaaa agtatattat caatattctc 2940
   tttatgataa ataaaaagaa aaaaaaaata aaagttaagt gaaaatgaga ttgaagtgac 3000
   tttaggtgtg tataaatata tcaaccccgc caacaattta tttaatccaa atatattgaa 3060
   gtatattatt ccatagcctt tatttattta tatatttatt atataaaagc tttatttgtt 3120
   ctaggttgtt catgaaatat ttttttggtt ttatctccgt tgtaagaaaa tcatgtgctt 3180
   tgtgtcgcca ctcactattg cagctttttc atgcattggt cagattgacg gttgattgta 3240
   tttttgtttt ttatggtttt gtgttatgac ttaagtcttc atctctttat ctcttcatca 3300
   ggtttgatgg ttacctaata tggtccatgg gtacatgcat ggttaaatta ggtggccaac 3360
   tttgttgtga acgatagaat tttttttata ttaagtaaac tatttttata ttatgaaata 3420
   ataataaaaa aaatatttta tcattattaa caaaatcata ttagttaatt tgttaactct 3480
   ataataaaag aaatactgta acattcacat tacatggtaa catctttcca ccctttcatt 3540
   tgttttttgt ttgatgactt tttttcttgt ttaaatttat ttcccttctt ttaaatttgg 3600
   aatacattat catcatatat aaactaaaat actaaaaaca ggattacaca aatgataaat 3660
   aataacacaa atatttataa atctagctgc aatatattta aactagctat atcgatattg 3720
   taaaataaaa ctagctgcat tgatactgat aaaaaaatat catgtgcttt ctggactgat 3780
   gatgcagtat acttttgaca ttgcctttat tttatttttc agaaaagctt tcttagttct 3840
   gggttcttca ttatttgttt cccatctcca ttgtgaattg aatcatttgc ttcgtgtcac 3900
   aaatacaatt tagntaggta catgcattgg tcagattcac ggtttattat gtcatgactt 3960
   aagttcatgg tagtacatta cctgccacgc atgcattata ttggttagat ttgataggca 4020
   aatttggttg tcaacaatat aaatataaat aatgttttta tattacgaaa taacagtgat 4080
   caaaacaaac agttttatct ttattaacaa gattttgttt ttgtttgatg acgtttttta 4140
   atgtttacgc tttccccctt cttttgaatt tagaacactt tatcatcata aaatcaaata 4200
   ctaaaaaaat tacatatttc ataaataata acacaaatat ttttaaaaaa tctgaaataa 4260
   taatgaacaa tattacatat tatcacgaaa attcattaat aaaaatatta tataaataaa 4320
   atgtaatagt agttatatgt aggaaaaaag tactgcacgc ataatatata caaaaagatt 4380
   aaaatgaact attataaata ataacactaa attaatggtg aatcatatca aaataatgaa 4440
   aaagtaaata aaatttgtaa ttaacttcta tatgtattac acacacaaat aataaataat 4500
   agtaaaaaaa attatgataa atatttacca tctcataaga tatttaaaat aatgataaaa 4560
   atatagatta ttttttatgc aactagctag ccaaaaagag aacacgggta tatataaaaa 4620
   gagtaccttt aaattctact gtacttcctt tattcctgac gtttttatat caagtggaca 4680
   tacgtgaaga ttttaattat cagtctaaat atttcattag cacttaatac ttttctgttt 4740
   tattcctatc ctataagtag tcccgattct cccaacattg cttattcaca caactaacta 4800
   agaaagtctt ccatagcccc ccaagcggcc gctgagtgat tgctcacgag tgtggtcacc 4860
   atgccttcag caagtaccaa tgggttgatg atgttgtggg tttgaccctt cactcaacac 4920
   ttttagtccc ttatttctca tggaaaataa gccatcgccg ccatcactcc aacacaggtt 4980
   cccttgaccg tgatgaagtg tttgtcccaa aaccaaaatc caaagttgca tggttttcca 5040
   agtacttaaa caaccctcta ggaagggctg tttctcttct cgtcacactc acaatagggt 5100
   ggcctatgta tttagccttc aatgtctctg gtagacccta tgatagtttt gcaagccact 5160
   accaccctta tgctcccata tattctaacc gtgagaggct tctgatctat gtctctgatg 5220
   ttgctttgtt ttctgtgact tactctctct accgtgttgc aaccctgaaa gggttggttt 5280
   ggctgctatg tgtttatggg gtgcctttgc tcattgtgaa cggttttctt gtgactatca 5340
   catatttgca gcacacacac tttgccttgc ctcattacga ttcatcagaa tgggactggc 5400
   tgaagggagc tttggcaact atggacagag attaagc 5437
<210> 26
   <211> 7025
   <212> DNA
   <213> artificial sequence
<220>
   <223> plasmid PHP17752
<400> 26
   gatccgtcga cggcgcgccc gatcatccgg atatagttcc tcctttcagc aaaaaacccc       60
   tcaagacccg tttagaggcc ccaaggggtt atgctagtta ttgctcagcg gtggcagcag      120
   ccaactcagc ttcctttcgg gctttgttag cagccggatc gatccaagct gtacctcact      180
   attcctttgc cctcggacga gtgctggggc gtcggtttcc actatcggcg agtacttcta      240
   cacagccatc ggtccagacg gccgcgcttc tgcgggcgat ttgtgtacgc ccgacagtcc      300
   cggctccgga tcggacgatt gcgtcgcatc gaccctgcgc ccaagctgca tcatcgaaat      360
   tgccgtcaac caagctctga tagagttggt caagaccaat gcggagcata tacgcccgga      420
   gccgcggcga tcctgcaagc tccggatgcc tccgctcgaa gtagcgcgtc tgctgctcca      480
   tacaagccaa ccacggcctc cagaagaaga tgttggcgac ctcgtattgg gaatccccga      540
   acatcgcctc gctccagtca atgaccgctg ttatgcggcc attgtccgtc aggacattgt      600
   tggagccgaa atccgcgtgc acgaggtgcc ggacttcggg gcagtcctcg gcccaaagca      660
   tcagctcatc gagagcctgc gcgacggacg cactgacggt gtcgtccatc acagtttgcc      720
   agtgatacac atggggatca gcaatcgcgc atatgaaatc acgccatgta gtgtattgac      780
   cgattccttg cggtccgaat gggccgaacc cgctcgtctg gctaagatcg gccgcagcga      840
   tcgcatccat agcctccgcg accggctgca gaacagcggg cagttcggtt tcaggcaggt      900
   cttgcaacgt gacaccctgt gcacggcggg agatgcaata ggtcaggctc tcgctgaatt      960
   ccccaatgtc aagcacttcc ggaatcggga gcgcggccga tgcaaagtgc cgataaacat     1020
   aacgatcttt gtagaaacca tcggcgcagc tatttacccg caggacatat ccacgccctc     1080
   ctacatcgaa gctgaaagca cgagattctt cgccctccga gagctgcatc aggtcggaga     1140
   cgctgtcgaa cttttcgatc agaaacttct cgacagacgt cgcggtgagt tcaggctttt     1200
   ccatgggtat atctccttct taaagttaaa caaaattatt tctagaggga aaccgttgtg     1260
   gtctccctat agtgagtcgt attaatttcg cgggatcgag atctgatcaa cctgcattaa     1320
   tgaatcggcc aacgcgcggg gagaggcggt ttgcgtattg ggcgctcttc cgcttcctcg     1380
   ctcactgact cgctgcgctc ggtcgttcgg ctgcggcgag cggtatcagc tcactcaaag     1440
   gcggtaatac ggttatccac agaatcaggg gataacgcag gaaagaacat gtgagcaaaa     1500
   ggccagcaaa aggccaggaa ccgtaaaaag gccgcgttgc tggcgttttt ccataggctc     1560
   cgcccccctg acgagcatca caaaaatcga cgctcaagtc agaggtggcg aaacccgaca     1620
   ggactataaa gataccaggc gtttccccct ggaagctccc tcgtgcgctc tcctgttccg     1680
   accctgccgc ttaccggata cctgtccgcc tttctccctt cgggaagcgt ggcgctttct     1740
   caatgctcac gctgtaggta tctcagttcg gtgtaggtcg ttcgctccaa gctgggctgt     1800
   gtgcacgaac cccccgttca gcccgaccgc tgcgccttat ccggtaacta tcgtcttgag     1860
   tccaacccgg taagacacga cttatcgcca ctggcagcag ccactggtaa caggattagc     1920
   agagcgaggt atgtaggcgg tgctacagag ttcttgaagt ggtggcctaa ctacggctac     1980
   actagaagga cagtatttgg tatctgcgct ctgctgaagc cagttacctt cggaaaaaga     2040
   gttggtagct cttgatccgg caaacaaacc accgctggta gcggtggttt ttttgtttgc     2100
   aagcagcaga ttacgcgcag aaaaaaagga tctcaagaag atcctttgat cttttctacg     2160
   gggtctgacg ctcagtggaa cgaaaactca cgttaaggga ttttggtcat gacattaacc     2220
   tataaaaata ggcgtatcac gaggcccttt cgtctcgcgc gtttcggtga tgacggtgaa     2280
   aacctctgac acatgcagct cccggagacg gtcacagctt gtctgtaagc ggatgccggg     2340
   agcagacaag cccgtcaggg cgcgtcagcg ggtgttggcg ggtgtcgggg ctggcttaac     2400
   tatgcggcat cagagcagat tgtactgaga gtgcaccata tggacatatt gtcgttagaa     2460
   cgcggctaca attaatacat aaccttatgt atcatacaca tacgatttag gtgacactat 2520
   agaacggcgc gccaagcttg gatcccccct cgaggtcgac ggtatcgata agcttctgca 2580
   ggaattctga gctagcgaag ttcctattcc gaagttccta ttcttcaaaa agtataggaa 2640
   cttcagacgt cctcgagtcc gtcctgtaga aaccccaacc cgtgaaatca aaaaactcga 2700
   cggcctgtgg gcattcagtc tggatcgcga aaactgtgga attgatccag aattcgctag 2760
   cgaagttcct attccgaagt tcctattctc tagaaagtat aggaacttca gatccagaat 2820
   tcggtccggg ccatcgtggc ctcttgctct tcaggatgaa gagctatgtt tcgcgccaag 2880
   cttggatcct agaactagaa acgtgatgcc acttgttatt gaagtcgatt acagcatcta 2940
   ttctgtttta ctatttataa ctttgccatt tctgactttt gaaaactatc tctggatttc 3000
   ggtatcgctt tgtgaagatc gagcaaaaga gacgttttgt ggacgcaatg gtccaaatcc 3060
   gttctacatg aacaaattgg tcacaatttc cactaaaagt aaataaatgg caagttaaaa 3120
   aaggaatatg cattttactg attgcctagg tgagctccaa gagaagttga atctacacgt 3180
   ctaccaaccg ctaaaaaaag aaaaacattg atatgtaacc tgattccatt agcttttgac 3240
   ttcttcaaca gattctctac ttagatttct aacagaaata ttattactag cacatcattt 3300
   tcagtctcac tacagcaaaa aatccaacgg cacaatacag acaacaggag atatcagact 3360
   acagagatag atagatgcta ctgcatgtag taagttaaat aaaaggaaaa taaaatgtct 3420
   tgctaccaaa actactacag actatgatgc tcaccacagg ccaaatcctg caactaggac 3480
   agcattatct tatatatatt gtacaaaaca agcatcaagg aacatttggt ctaggcaatc 3540
   agtacctcgt tctaccatca ccctcagtta tcacatcctt gaaggatcca ttactgggaa 3600
   tcatcggcaa cacatgctcc tgatggggca caatgacatc aagaaggtag gggccagggg 3660
   tgtccaacat tctctgaatt gccgctctaa gctcttcctt cttcgtcact cgcgctgccg 3720
   gtatcccaca agcatcagca aacttgagca tgtttgggaa tatctcgctc tcgctagacg 3780
   gatctccaag ataggtgtga gctctattgg acttgtagaa cctatcctcc aactgaacca 3840
   ccatacccaa atgctgattg ttcaacaaca atatcttaac tgggagattc tccactctta 3900
   tagtggccaa ctcctgaaca ttcatgatga aactaccatc cccatcaatg tcaaccacaa 3960
   cagccccagg gttagcaaca gcagcaccaa tagccgcagg caatccaaaa cccatggctc 4020
   caagaccccc tgaggtcaac cactgcctcg gtctcttgta cttgtaaaac tgcgcagccc 4080
   acatttgatg ctgcccaacc ccagtactaa caatagcatc tccattagtc aactcatcaa 4140
   gaacctcgat agcatgctgc ggagaaatcg cgtcctggaa tgtcttgtaa cccaatggaa 4200
   acttgtgttt ctgcacatta atctcttctc tccaacctcc aagatcaaac ttaccctcca 4260
   ctcctttctc ctccaaaatc atattaattc ccttcaaggc caacttcaaa tccgcgcaaa 4320
   ccgacacgtg cgcctgcttg ttcttcccaa tctcggcaga atcaatatca atgtgaacaa 4380
   tcttagccct actagcaaaa gcctcaagct tcccagtaac acggtcatca aaccttaccc 4440
   caaaggcaag caacaaatca ctattgtcaa cagcatagtt agcataaaca gtaccatgca 4500
   tacccagcat ctgaagggaa tattcatcac caataggaaa agttccaaga cccattaaag 4560
   tgctagcaac gggaatacca gtgagttcaa caaagcgcct caattcagca ctggaattca 4620
   aactgccacc gccgacgtag agaacgggct tttgggcctc catgatgagt ctgacaatgt 4680
   gttccaattg ggcctcggcg gggggcctgg gcagcctggc gaggtaaccg gggaggttaa 4740
   cgggctcgtc ccaattaggc acggcgagtt gctgctgaac gtctttggga atgtcgatga 4800
   ggaccggacc ggggcggccg gaggtggcga cgaagaaagc ctcggcgacg acgcggggga 4860
   tgtcgtcgac gtcgaggatg aggtagttgt gcttcgtgat ggatctgctc acctccacga 4920
   tcggggtttc ttggaaggcg tcggtgccga tcatccggcg ggcgacctgg ccggtgatgg 4980
   cgacgactgg gacgctgtcc attaaagcgt cggcgaggcc gctcacgagg ttggtggcgc 5040
   cggggccgga ggtggcaatg cagacgccgg ggaggccgga ggaacgcgcg tagccttcgg 5100
   cggcgaagac gccgccctgc tcgtggcgcg ggagcacgtt gcggatggcg gcggagcgcg 5160
   tgagcgcctg gtggatctcc atcgacgcac cgccggggta cgcgaacacc gtcgtcacgc 5220
   cctgcctctc cagcgcctcc acaaggatgt ccgcgccctt gcgaggttcg ccggaggcga 5280
   accgtgacac gaagggctcc gtggtcggcg cttccttggt gaagggcgcc gccgtggggg 5340
   gtttggagat ggaacatttg attttgagag cgtggttggg tttggtgagg gtttgatgag 5400
   agagagggag ggtggatcta gtaatgcgtt tggggaaggt ggggtgtgaa gaggaagaag 5460
   agaatcgggt ggttctggaa gcggtggccg ccattgtgtt gtgtggcatg gttatacttc 5520
   aaaaactgca caacaagcct agagttagta cctaaacagt aaatttacaa cagagagcaa 5580
   agacacatgc aaaaatttca gccataaaaa aagttataat agaatttaaa gcaaaagttt 5640
   cattttttaa acatatatac aaacaaactg gatttgaagg aagggattaa ttcccctgct 5700
   caaagtttga attcctattg tgacctatac tcgaataaaa ttgaagccta aggaatgtat 5760
   gagaaacaag aaaacaaaac aaaactacag acaaacaagt acaattacaa aattcgctaa 5820
   aattctgtaa tcaccaaacc ccatctcagt cagcacaagg cccaaggttt attttgaaat 5880
   aaaaaaaaag tgattttatt tctcataagc taaaagaaag aaaggcaatt atgaaatgat 5940
   ttcgactaga tctgaaagtc caacgcgtat tccgcagata ttaaagaaag agtagagttt 6000
   cacatggatc ctagatggac ccagttgagg aaaaagcaag gcaaagcaaa ccagaagtgc 6060
   aagatccgaa attgaaccac ggaatctagg atttggtaga gggagaagaa aagtaccttg 6120
   agaggtagaa gagaagagaa gagcagagag atatatgaac gagtgtgtct tggtctcaac 6180
   tctgaagcga tacgagttta gaggggagca ttgagttcca atttataggg aaaccgggtg 6240
   gcaggggtga gttaatgacg gaaaagcccc taagtaacga gattggattg tgggttagat 6300
   tcaaccgttt gcatccgcgg cttagattgg ggaagtcaga gtgaatctca accgttgact 6360
   gagttgaaaa ttgaatgtag caaccaattg agccaacccc agcctttgcc ctttgatttt     6420
   gatttgtttg ttgcatactt tttatttgtc ttctggttct gactctcttt ctctcgtttc     6480
   aatgccaggt tgcctactcc cacaccactc acaagaagat tctactgtta gtattaaata     6540
   ttttttaatg tattaaatga tgaatgcttt tgtaaacaga acaagactat gtctaataag     6600
   tgtcttgcaa cattttttaa gaaattaaaa aaaatatatt tattatcaaa atcaaatgta     6660
   tgaaaaatca tgaataatat aattttatac atttttttaa aaaatctttt aatttcttaa     6720
   ttaatatctt aaaaataatg attaatattt aacccaaaat aattagtatg attggtaagg     6780
   aagatatcca tgttatgttt ggatgtgagt ttgatctaga gcaaagctta ctagagtcga     6840
   ccgatccgtc gacggcgcgc gcgcctctag ttgaagacac gttcatgtct tcatcgtaag     6900
   aagacactca gtagtcttcg gccagaatgg cccggaccga agcttctgca ggaattctga     6960
   gctagcgaag ttcctattcc gaagttccta ttctctagaa agtataggaa cttcagatcc     7020
   actag                                                                 7025

## Description

### FIELD OF THE INVENTION

This invention relates to blended soy protein products with improved whiteness, altered viscosity and altered gel-strength.

### BACKGROUND OF THE INVENTION

Soybeans have the highest protein content of all cereals and legumes. In particular, soybeans have about 40% protein, while other legumes have 20-30%, and cereals have about 8-15% protein. Soybeans also contain about 20% oil with the remaining dry matter mostly carbohydrate (35%). On a wet basis (as is), soybeans contain about 35% protein, 17% oil, 31% carbohydrates and 4.4% ash. Soybean storage protein and lipid bodies are contained in the usable meat of the soybean called the cotyledon. The complex carbohydrate (or dietary fiber) is also contained in the cell walls of the cotyledon. The outer layer of cells (called the seed coat) makes up about 8% of the soybean's total weight. The raw, dehulled soybean is, depending on the variety, approximately 18% oil, 15% insoluble carbohydrates, 14% moisture and ash and 38% protein.

Plant protein materials are used as functional food ingredients, and have numerous applications in enhancing desirable characteristics in food products. Soy protein materials, in particular, have seen extensive use as functional food ingredients. Soy protein materials are used as an emulsifier in meats to bind the meat and give the meat a good texture and a firm bite. Another common application for soy protein materials as functional food ingredients is as a thickening agent to provide a creamy viscosity to the food product.

In general, soy protein materials include soy flakes, soy grits, soy meal, soy flour, soy protein concentrates, and soy protein isolates with a primary difference between these materials being the degree of refinement relative to whole soybeans.

Apart from the soy protein content, flavor, gel-strength, whiteness-index, and viscosity of a soy protein material are also a relevant criteria for the selection of a soy protein material as a functional food ingredient. Conventional soy protein material may have a strong beany, bitter flavor and odor as a result of the presence. of certain volatile compounds and/or an undesired appearance due to the presence of other relatively low molecular weight compounds in the soy protein material.

The present disclosure generally relates to a blended soy protein product-containing composition having altered (e.g. increased or reduced) gel-strength, viscosity, or whiteness.
US Patent No. 6,599,556 B2, issued to Stark et al. on July 29, 2003, describes confectionary products, which include high protein content modified oilseed material.

US Patent No. 6,716,469 B2, issued to Stark et al. on April 06, 2004, describes frozen dessert products, which include high protein content modified oilseed material.

US Patent No. 6,720,020 B2, issued to Karleskind et al. on April 13, 2004, describes beverage compositions, which include high protein content modified oilseed material.

JP Patent No. 5,168,416 A1, issued to Takeshi et al. on July 02, 1993, describes obtaining a concentrated soybean having improved taste, flavor and color tone and useful as a food material, etc., with simple operation at a low cost without changing the nature of the protein by washing soybeans, etc., with a water-containing alcohol under weakly acidic condition in the presence of an acid.

JP Patent No. 4,207,159 A1, issued to Hiroko et al on July 29, 1992, describes the title raw material having bright and white color tone and useful for marine and knead eater-dispersed liquid of acid-precipitated soybean protein with an alkali metal hydroxide to control pH.

WO2007013146A1 published February 1st, 2007, describes compositions for processed soy protein foods.

WO2009086047A2, published July 9th, 2009, describes soybean protein products having altered characteristics.

WO2009086047, published August 27th 2009 describes soy protein products obtained from high oleic soybeans with improved properties.

US4309344 describes a process for the production of a protein isolate from vegetable protein material with improved whiteness.

### SUMMARY OF THE INVENTION

In a first embodiment, the invention concerns a method of preparing a blended soy protein product, said method comprising blending commodity soybean defatted flakes and high oleic soybean defatted flakes and preparing a first soy protein product therefrom having a combined PUFA content of about 0.17% to about 1.65% by weight, wherein said high oleic soybean flakes are obtained from soybean seeds having an oleic acid content of at least 60% of the fatty acid moieties and wherein a slurry or solution of said first product has reduced viscosity when compared to a slurry or solution of a second soy protein product obtained from the commodity soybean defatted flakes, and prepared using the same process.

In a second embodiment, the invention concerns a method wherein said high oleic soybean defatted flakes comprise at least 40% of the total defatted flakes.

In a third embodiment said first product has improved drying efficiency when compared to a second soy protein product obtained from commodity soybean defatted flakes.

In a fourth embodiment said first product has an improved whiteness index when compared to a second soy protein product obtained from the commodity soybean defatted flakes.

In a fifth embodiment the viscosity of the first soy protein product is reduced by at least 25%.

In a sixth embodiment the drying efficiency of the first soy protein product is increased by at least 1%.

In a seventh embodiment the whiteness index of the first soy protein product is increased by at least 2.5%.

In another embodiment said protein product has at least 40%, at least 65% or at least 90% protein (N x 6.25) on a moisture-free basis.

In another embodiment said method comprises preparing a product selected from the group consisting of: a soy protein isolate, a soy protein concentrate, soy meal, full fat flour, soymilk powder, defatted flour, soymilk, textured protein, textured flours, textured concentrates and textured isolates.

In another embodiment said method further comprises incorporating said first soy protein product in a food, beverage or animal feed.

### BRIEF DESCRIPTION OF THE

### DRAWINGS AND SEQUENCE LISTINGS

The invention can be more fully understood from the following detailed description and the accompanying drawings and Sequence Listing, which form a part of this application.
Fig.1 depicts plasmid pKS210.
Fig. 2 depicts plasmid PHP17731.
Fig. 3 depicts plasmid PHP17064.
Fig. 4 depicts fragment PHP19340A.
Fig. 5 depicts fragment PHP17752A.
Fig. 6 depicts plasmid PHP19340.
Fig. 7 depicts plasmid PHP17752.
Fig. 8 shows the effect of mixing high oleic and commodity flakes on the resultant isolate slurrys' in-process apparent viscosity as a function of shear.
Fig. 9 shows the effect of PUFA content of the high oleic and commodity flake mix on the in-process apparent viscosity
Fig. 10 shows the effect of PUFA addition on the whiteness index of the freeze dried soy protein isolate powder.
Fig.11 shows the effect of individual fatty acids on the viscosity of the soy protein products prepared using the method of the invention.
   SEQ ID NO:1 sets forth the sequence of the recombinant DNA fragment PHP21676A
   SEQ ID NO:2 sets forth the sequence of the 1533 polynucleotide fragment comprising 470 nucleotides from the soybean FAD2-2 gene, 420 nucleotides from the soybean FAD2-1 gene, 643 nucleotides from the soybean FAD3 gene.
   SEQ ID NO:3 sets forth the nucleotide sequence of oligonucleotide primer BM35 used to amplify an approximately 0.9 Kb fragment from recombinant DNA fragment KSFAD2-hybrid.
   SEQ ID NO:4 sets forth the nucleotide sequence of oligonucleotide primer BM39 used to amplify an approximately 0.9 kb fragment from recombinant DNA fragment KSFAD2-hybrid.
   SEQ ID NO:5 sets forth the nucleotide sequence of oligonucleotide primer BM40 used to amplify an approximately 0.65 kb DNA fragment from plasmid XF1.
   SEQ ID NO:6 sets forth the nucleotide sequence of oligonucleotide plasmid BM41 used to amplify an approximately 0.65 kb DNA fragment from plasmid pXF1.
   SEQ ID NO:7 sets forth the nucleotide sequence of recombinant DNA fragment KSFAD2-hybrid which contains about 470 nucleotides from the soybean FAD2-2 gene and 420 nucleotides from the soybean FAD2-1 gene.
   SEQ ID NO:8 sets forth the nucleotide sequence of oligonucleotide primer KS1 used to amplify about 470 nucleotides from the soybean FAD2-2 gene.
   SEQ ID NO:9 sets forth the nucleotide sequence of oligonucleotide primer KS2 used to amplify about 470 nucleotides of the soybean FAD2-2 gene.
   SEQ ID NO:10 sets forth the nucleotide sequence of oligonucleotide primer KS3 used to amplify about 420 nucleotides of the soybean FAD2-1 gene.
   SEQ ID NO:11 sets forth the nucleotide sequence of oligonucleotide primer KS4 used to amplify about 420 nucleotides of the soybean FAD2-1 gene.
   SEQ ID NO:12 sets forth the nucleotide sequence of the seed-specific gene expression-silencing cassette from pKS133 which comprises nucleotides for a Kti3 promoter and terminator bordering a string of nucleotides that promote formation of a stem structure which are surrounding a unique Not I restriction endonuclease site.
   SEQ ID NO:13 sets forth the nucleotide sequence of plasmid pKS210.
   SEQ ID NO:14 sets forth the nucleotide sequence of plasmid PHP17731.
   SEQ ID NO:15 sets forth the nucleotide sequence of recombinant DNA fragment PHP17731A.
   SEQ ID NO:16 sets forth the nucleotide sequence of the ALS selectable marker recombinant DNA fragment. This recombinant DNA fragment comprises a promoter operably linked to a nucleotide fragment encoding a soybean acetolactate synthase to which mutations have been introduced to make it resistant to treatment with sulfonylurea herbicides.
   SEQ ID NO:17 sets forth the amino acid sequence of the soybean herbicide-resistant ALS including mutations in subsequences B and F.
   SEQ ID NO:18 is the wild type amino acid sequence of conserved ALS "subsequence B" disclosed in U. S. Patent No. 5,013,659.
   SEQ ID NO:19 sets forth the wild type amino acid sequence of conserved ALS "subsequence F" disclosed in U. S. Patent No. 5,013,659.
   SEQ ID NO:20 sets forth the amino acid sequence of the additional five amino acids introduced during cloning at the amino-terminus of the soybean ALS.
   SEQ ID NO:21 sets forth the nucleotide sequence of plasmid PHP17064
   SEQ ID NO:22 sets forth the nucleotide sequence of recombinant DNA fragment PHP17064A.
   SEQ ID NO:23 sets forth the nucleotide sequence of fragment PHP19340A.
   SEQ ID NO:24 sets forth the nucleotide sequence of fragment PHP17752A.
   SEQ ID NO:25 sets forth the nucleotide sequence of plasmid PHP19340.
   SEQ ID NO:26 sets forth the nucleotide sequence of plasmid PHP17752.

The Sequence Listing contains the one letter code for nucleotide sequence characters and the three letter codes for amino acids as defined in conformity with the IUPAC-IUBMB standards described in Nucleic Acids Res. 13:3021-3030 (1985) and in the Biochemical J. 219 (No. 2):345-373 (1984). The symbols and format used for nucleotide and amino acid sequence data comply with the rules set forth in 37C.F.R. §1.822.

### DETAILED DESCRIPTION OF THE INVENTION

The present disclosure generally relates to a blended soy protein product-containing composition having altered (e.g. increased or reduced) gel-strength, viscosity, or whiteness.
In the context of this disclosure, a number of terms shall be utilized.

As used herein, "soybean" refers to the species *Glycine max, Glycine soja,* or any species that is sexually cross compatible with *Glycine max*. A "line" is a group of plants of similar parentage that display little or no genetic variation between individuals for a least one trait. Such lines may be created by one or more generations of self-pollination and selection, or vegetative propagation from a single parent including by tissue or cell culture techniques. An "agronomically elite line" or "elite line" refers to a line with desirable agronomic performance that may or may not be used commercially. A "variety", "cultivar", "elite variety", or "elite cultivar" refers to an agronomically superior elite line that has been extensively tested and is or was being used for commercial soybean production. "Mutation" refers to a detectable and heritable genetic change (either spontaneous or induced) not caused by segregation or genetic recombination. "Mutant" refers to an individual, or lineage of individuals, possessing a mutation.

The "whiteness index" of a soy protein product refers to the color of the soy-protein-containing composition. Many soy protein-containing feed compositions will have, to varying degrees, a yellowish or brownish color. In general, the color of these compositions can be "improved," *i*.*e*., the "whiteness index" of the product can be increased by the process of the present invention. In general, the whiteness index is determined using a colorimeter which provides the L, a, and b color values for the composition from which the whiteness index may be calculated using a standard expression of the Whiteness Index (WI), WI = L-3b. The L component generally indicates the whiteness or, "lightness", of the sample; L values near 0 indicate a black sample while L values near 100 indicate a white sample. The b value indicates yellow and blue colors present in the sample; positive b values indicate the presence of yellow colors while negative b values indicate the presence of blue colors. The a value, which may be used in other color measurements, indicates red and green colors; positive values indicate the presence of red colors while negative values indicate the presence of green colors. For the b and a values, the absolute value of the measurement increases directly as the intensity of the corresponding color increases. Generally, the colorimeter is standardized using a white standard tile provided with the colorimeter. A sample is then placed into a glass cell which is introduced to the colorimeter. The sample cell is covered with an opaque cover to minimize the possibility of ambient light reaching the detector through the sample and serves as a constant during measurement of the sample. After the reading is taken, the sample cell is emptied and typically refilled as multiple samples of the same material are generally measured and the whiteness index of the material expressed as the average of the measurements. Suitable colorimeters generally include those manufactured by HunterLab (Reston, VA) including, for example, Model # DP-9000 with Optical Sensor D 25.

Whiteness index measurements of a 5% by weight solids sample of the suspension before and after treatment are determined using a HunterLab DP-9000 colorimeter including an optical sensor D-25, both manufactured by Hunter Associates Laboratory (HunterLab) (Reston, VA). For the whiteness index measurement in the large scale production platform, protein samples are dispersed on a 5% w/w basis: (5.25 g) is added to deionized water (100 mL). For the whiteness index measurement in the small scale production platform, 1 g protein sample is dispersed in 19mL of deionized water on a w/v basis. The results obtained using the Hunter Colorimeter are reported in units of L, a, and b. Whiteness Index is calculated from the L and b scale values using the following: Whiteness Index = L - 3b.

In addition to the improved color, the soy protein product produced by the processes in the present disclosure can have a reduced viscosity.

Viscosity, gelation and other indicators of structure formation are important properties of soybean proteins since they contribute to the overall utility of the product in use. Proteins contribute to the solidity and elasticity of products by formation of a three dimensional network of aggregated protein molecules which entrap water. It is sometimes desirable to have these properties, for example in the case of meat-like products, or it may be desired to have less functionality, for example in beverage applications. For beverage applications, a lower viscosity may be desirable for sensory, mouthfeel and textural properties of the beverage. Lower viscosity soy protein-containing compositions may be intended for use in liquid products (i.e., beverages); and additionally, in some embodiments, higher viscosity soy protein-containing compositions may be desired for use in meat products.

As used herein, the term "viscosity" means the apparent viscosity of aqueous slurry or a solution as measured with a rotating spindle viscometer utilizing a large annulus, where a particularly preferred rotating spindly viscometer is an Anton Paar MCR-300. The apparent viscosity can also be measured using a Rapid Visco Analyzer (RVA) viscometer, Brookfield viscometer, or other viscometers and rheometers known in the art.

The apparent viscosity of a soy protein material may be measured, for example, by weighing a sample of the soy material and water to obtain a known ratio of the soy material to water (preferably 1 part soy material to 9 parts water, by weight), combining and mixing the soy material and water in a blender or mixer to form a homogenous slurry of the soy material and water at ambient temperature and neutral pH, and measuring the apparent viscosity of the slurry with the rotating spindle viscometer utilizing a large annulus, operated at approximately 60 revolutions per minute and at a torque of from 30 to 70%.

Another important functional characteristic is the gel forming property of a protein. Protein gelation is important to obtain desirable sensory and textural structures in foods.

The formation of a protein gel is a two step process which initiates through partial denaturation of the protein molecules. As the proteins denature, the viscosity of the slurry increases as a result of an increase in the molecular changes associated with the unfolding proteins. During the second part of the process there is a large increase in viscosity resulting from protein association and development of the molecular network.

Gelation phenomenon requires a driving force to unfold the native protein structure, followed by an aggregation retaining a certain degree of order in the matrix formed by association between protein strands. Protein gelation has been traditionally achieved by heating, but some physical and chemical processes form protein gels in an analogous way to heat-induction. A physical means, besides heat, is high pressure. Chemical means are acidification, enzymatic cross-linking, and use of salts and urea, causing modifications in protein-protein and protein-medium interactions. The characteristics of each gel are different and dependent upon factors like protein concentration, degree of denaturation caused by pH, temperature, ionic strength and/or pressure.

The term "gel-strength" refers to the ability or a measure of a protein to form gels.

"Polyunsaturated fatty acid(s) and their triglycerides" are abbreviated PUFA(s).

The term "fatty acids" refers to long-chain aliphatic acids (alkanoic acids) of varying chain length, from about C₁₂ to C₂₂ (although both longer and shorter chain-length acids are known). The predominant chain lengths are between C₁₆ and C₂₂. The structure of a fatty acid is represented by a simple notation system of "X:Y", where X is the total number of C atoms in the particular fatty acid and Y is the number of double bonds.

Generally, fatty acids are classified as saturated or unsaturated. The term "saturated fatty acids" refers to those fatty acids that have no "double bonds" between their carbon backbone. In contrast, "unsaturated fatty acids" have "double bonds" along their carbon backbones (which are most commonly in the *cis-*configuration). "Monounsaturated fatty acids" have only one "double bond" along the carbon backbone (e.g., usually between the 9^{th} and 10^{th} carbon atom as for palmitoleic acid (16:1) and oleic acid (18:1)), while "polyunsaturated fatty acids" (or "PUFAs") have at least two double bonds along the carbon backbone (e.g., between the 9^{th} and 10^{th}, and 12^{th} and 13^{th} carbon atoms for linoleic acid (18:2); and between the 9^{th} and 10^{th}, 12^{th} and 13^{th}, and 15^{th} and 16^{th} for α-linolenic acid (18:3)).

The term "total fatty acid content" refers to the sum of the five major fatty acid components found in soybeans, namely C16:0, C18:0, C18:1, C18:2, and C18:3. The term "total polyunsaturated fatty acid content" refers to the total C18:2 plus C18:3 content.

For the purposes of the present disclosure, the omega-reference system will be used to indicate the number of carbons, the number of double bonds and the position of the double bond closest to the omega carbon, counting from the omega carbon (which is the terminal carbon of the aliphatic chain and is numbered 1 for this purpose). This nomenclature is shown below in Table 1, in the column titled "Shorthand Notation".

**Table 1**

| Nomenclature of Polyunsaturated Fatty Acids | | | |
|---|---|---|---|
| Common Name | Abbreviation | Chemical Name | Shorthand Notation |
| Linoleic | LA | *cis*-9,12-octadecadienoic | 18:2 ω-6 |
| α-Linolenic | αLIN | *cis*-9*,* 12, 15-octadecatrienoic | 18:3 ω-3 |

The term "desaturase" refers to a polypeptide that can desaturate, i.e., introduce a double bond, in one or more fatty acids to produce a mono- or polyunsaturated fatty acid or precursor which is of interest. Despite use of the omega-reference system throughout the specification in reference to specific fatty acids, it is more convenient to indicate the activity of a desaturase by counting from the carboxyl end of the substrate using the Δ-system.

The terms "FAD" and fatty acid desaturase are used interchangeably and refer to membrane bound microsomal oleoyl- and linoleoyl-phosphatidylcholine desaturases that convert oleic acid to linoleic acid and linoleic acid to linolenic acid, respectively, in reactions that reduce molecular oxygen to water and require the presence of NADH.

The term "high oleic soybean" refers to soybean seeds that have an oleic acid content of at least 60%, 61%, 62%, 63%, 64%, 65%, 66%, 67%, 68%, 69%, 70%, 71 %, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, and 95% of the fatty acid moieties. Preferred high oleic soybean oil starting materials are disclosed in World Patent Publication WO94/11516.

Some embodiments of the instant invention comprise methods utilizing soybean seeds with an oleic acid content of at least 50%, 51%, 52%, 53%, 54%, 55%, 56%, 57%, 58%, 59%, 60%, 61%, 62%, 63%, 64%, 65%, 66%, 67%, 68%, 69%, 70%, 71 %, 72%, 73%, 74%, 75%, 76%, 77%, 78%, 79%, 80%, 81 %, 82%, 83%, 84%, 85%, 86%, 87%, 88%, 89%, 90%, 91 %, 92%, 93%, 94%, and 95% of the fatty acid moieties.

The term enzyme "activity" refers to the ability of an enzyme to convert a substrate to a product.

The terms "polynucleotide", "polynucleotide sequence", "nucleic acid sequence", "nucleic acid fragment", and "isolated nucleic acid fragment" are used interchangeably herein. These terms encompass nucleotide sequences and the like. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof. Nucleotides (usually found in their 5'-monophosphate form) are referred to by a single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of chimeric genes to produce the desired phenotype in a transformed plant.

Chimeric genes can be designed for use in suppression by linking a nucleic acid fragment or subfragment thereof, whether or not it encodes an active enzyme, in the sense or antisense orientation relative to a plant promoter sequence.

The terms "homology", "homologous", "substantially similar" and "corresponding substantially" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments such as deletion or insertion of one or more nucleotides that do not substantially alter the functional properties of the resulting nucleic acid fragment relative to the initial, unmodified fragment.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure. An "allele" is one of several alternative forms of a gene occupying a given locus on a chromosome. When all the alleles present at a given locus on a chromosome are the same that plant is homozygous at that locus. If the alleles present at a given locus on a chromosome differ that plant is heterozygous at that locus. A "codon-optimized gene" is a gene having its frequency of codon usage designed to mimic the frequency of preferred codon usage of the host cell.

"Coding sequence" refers to a DNA sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a region of DNA capable of controlling the expression of a coding sequence or functional RNA. The promoter sequence consists of proximal and more distal upstream elements. These upstream elements are often referred to as enhancers. Accordingly, an "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters". New promoters of various types useful in plant cells are constantly being discovered; numerous examples may be found in the compilation by Okamuro and Goldberg (1989) Biochemistry of Plants 15:1-82.

Any seed-specific promoter can be used in accordance with the method of the invention. Thus, the origin of the promoter chosen to drive expression of the recombinant DNA fragment is not critical as long as it is capable of accomplishing the invention by transcribing enough RNA from the desired nucleic acid fragment(s) in the seed.

A plethora of promoters is described in WO 00/18963, published on April 6, 2000. Examples of seed-specific promoters include, and are not limited to, the promoter for soybean
Kunitz trypsin inhibitor (Kti3, Jofuku and Goldberg (1989) Plant Cell 1:1079-1093) β-conglycinin (Chen et al. (1989) Dev. Genet. 10: 112-122), the napin promoter, and the phaseolin promoter.

Specific examples of promoters that may be useful in expressing the nucleic acid fragments of the invention include, but are not limited to, the SAM synthetase promoter (PCT Publication WO00/37662, published June 29, 2000), the CaMV 35S (Odell et al (1985) Nature 313:810-812), and the promoter described in PCT Publication WO02/099063 published December 12, 2002.

The "translation leader sequence" refers to a polynucleotide sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner and Foster (1995) Mol. Biotechnol. 3:225-236).

The "3' non-coding sequences" or "transcription terminator/termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3'non-coding sequences is exemplified by Ingelbrecht et al. (1989) Plant Cell 1:671-680.

An "intron" is an intervening sequence in a gene that does not encode a portion of the protein sequence. Thus, such sequences are transcribed into RNA but are then excised and are not translated. The term is also used for the excised RNA sequences. An "exon" is a portion of the sequence of a gene that is transcribed and is found in the mature messenger RNA derived from the gene, but is not necessarily a part of the sequence that encodes the final gene product.

"RNA transcript" refers to the product resulting from RNA polymerase-catalyzed transcription of a DNA sequence. When the RNA transcript is a perfect complementary copy of the DNA sequence, it is referred to as the primary transcript. An RNA transcript is referred to as the mature RNA when it is an RNA sequence derived from post-transcriptional processing of the primary transcript. "Messenger RNA (mRNA)" refers to the RNA that is without introns and that can be translated into protein by the cell. "cDNA" refers to a DNA that is complementary to and synthesized from a mRNA template using the enzyme reverse transcriptase. The cDNA can be single-stranded or converted into the double-stranded form using the Klenow fragment of DNA polymerase I. "Sense" RNA refers to RNA transcript that includes the mRNA and can be translated into protein within a cell or in vitro. "Antisense RNA" refers to an RNA transcript that is complementary to all or part of a target primary transcript or mRNA, and that blocks the expression of a target gene (U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

The term "endogenous RNA" refers to any RNA which is encoded by any nucleic acid sequence present in the genome of the host prior to transformation with the recombinant construct of the present invention, whether naturally-occurring or non-naturally occurring, i.e., introduced by recombinant means, mutagenesis, etc.

The term "non-naturally occurring" means artificial, not consistent with what is normally found in nature.

Standard recombinant DNA and molecular cloning techniques used herein are well known in the art and are described more fully in Sambrook et al.,Molecular Cloning: A Laboratory Manual; Cold Spring Harbor Laboratory Press: Cold Spring Harbor, 1989. Transformation methods are well known to those skilled in the art and are described below.

"PCR" or "Polymerase Chain Reaction" is a technique for the synthesis of large quantities of specific DNA segments, consists of a series of repetitive cycles (Perkin Elmer Cetus Instruments, Norwalk, CT). Typically, the double stranded DNA is heat denatured, the two primers complementary to the 3' boundaries of the target segment are annealed at low temperature and then extended at an intermediate temperature. One set of these three consecutive steps is referred to as a cycle.

The term "recombinant" refers to an artificial combination of two otherwise separated segments of sequence, e.g., by chemical synthesis or by the manipulation of isolated segments of nucleic acids by genetic engineering techniques.

The terms "plasmid", "vector" and "cassette" refer to an extra chromosomal element often carrying genes that are not part of the central metabolism of the cell, and usually in the form of circular double-stranded DNA fragments. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3'untranslated sequence into a cell. "Transformation cassette" refers to a specific vector containing a foreign gene and having elements in addition to the foreign gene that facilitates transformation of a particular host cell. "Expression cassette" refers to a specific vector containing a foreign gene and having elements in addition to the foreign gene that allow for enhanced expression of that gene in a foreign host.

The terms "recombinant construct", "expression construct", "chimeric construct", "construct", and "recombinant DNA construct" are used interchangeably herein. A recombinant construct comprises an artificial combination of nucleic acid fragments, e.g., regulatory and coding sequences that are not found together in nature. For example, a chimeric construct may comprise regulatory sequences and coding sequences that are derived from different sources, or regulatory sequences and coding sequences derived from the same source, but arranged in a manner different than that found in nature. Such construct may be used by itself or may be used in conjunction with a vector. If a vector is used then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells comprising isolated nucleic acid fragments of the. The skilled artisan will also recognize that different independent transformation events will result in different levels and patterns of expression (Jones et al., (1985) EMBO J. 4:2411-2418; De Almeida et al., (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, immunoblotting analysis of protein expression, or phenotypic analysis, among others.

The term "expression", as used herein, refers to the production of a functional end-product e.g., a mRNA or a protein (precursor or mature).

The term "expression cassette" as used herein, refers to a discrete nucleic acid fragment into which a nucleic acid sequence or fragment can be moved.

"Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre- and propeptides may be but are not limited to intracellular localization signals.

"Cosuppression" refers to the production of sense RNA transcripts capable of suppressing the expression of identical or substantially similar native genes (U.S. Patent No. 5,231,020, which issued to Jorgensen et al. on July 27, 1999). Co-suppression constructs in plants have been previously designed by focusing on overexpression of a nucleic acid sequence having homology to a native mRNA, in the sense orientation, which results in the reduction of all RNA having homology to the overexpressed sequence (see Vaucheret et al. (1998) Plant J. 16:651-659; and Gura (2000) Nature 404:804-808). "Antisense inhibition" refers to the production of antisense RNA transcripts capable of suppressing the expression of the target protein. Plant viral sequences may be used to direct the suppression of proximal mRNA encoding sequences (PCT Publication WO 98/36083 published on August 20, 1998). "Hairpin" structures that incorporate all, or part, of an mRNA encoding sequence in a complementary orientation resulting in a potential "stem-loop" structure for the expressed RNA have been described (PCT Publication WO 99/53050 published on October 21, 1999). In this case the stem is formed by polynucleotides corresponding to the gene of interest inserted in either sense or anti-sense orientation with respect to the promoter and the loop is formed by some polynucleotides of the gene of interest, which do not have a complement in the construct. This increases the frequency of cosuppression or silencing in the recovered transgenic plants. For review of hairpin suppression see Wesley et al. (2003) Methods in Molecular Biology, Plant Functional Genomics: Methods and Protocols 236:273-286. A construct where the stem is formed by at least 30 nucleotides from a gene to be suppressed and the loop is formed by a random nucleotide sequence has also effectively been used for suppression (WO 99/61632 published on December 2, 1999). The use of poly-T and poly-A sequences to generate the stem in the stem-loop structure has also been described (WO 02/00894 published January 3, 2002). Yet another variation includes using synthetic repeats to promote formation of a stem in the stem-loop structure. Transgenic organisms prepared with such recombinant DNA fragment show reduced levels of the protein encoded by the polynucleotide from which the nucleotide fragment forming the loop is derived as described in PCT Publication WO 02/00904, published January 3, 2002. The use of constructs that result in dsRNA has also been described. In these constructs convergent promoters direct transcription of gene-specific sense and antisense RNAs inducing gene suppression (see for example Shiet al. (2000) RNA 6:1069-1076; Bastinet al. (2000) J. Cell Sci. 113:3321-3328; Giordanoet al. (2002) Genetics 160:637-648; LaCount, and Donelson. US patent Application No. 20020182223, published December 5, 2002;Tranet al. (2003) BMC Biotechnol. 3:21; and Applicant's U.S. Provisional Application No. 60/578,404, filed June 9, 2004).

Other methods for suppressing an enzyme include, but are not limited to, use of polynucleotides that may form a catalytic RNA or may have ribozyme activity (U.S. Patent No. 4,987,071 issued January 22, 1991), and micro RNA (also called miRNA) interference (Javier et al. (2003) Nature 425:257-263).

MicroRNAs (miRNA) are small regulatory RNAs that control gene expression. miRNAs bind to regions of target RNAs and inhibit their translation and, thus, interfere with production of the polypeptide encoded by the target RNA. miRNAs can be designed to be complementary to any region of the target sequence RNA including the 3' untranslated region, coding region, etc. miRNAs are processed from highly structured RNA precursors that are processed by the action of a ribonuclease III termed DICER. While the exact mechanism of action of miRNAs is unknown, it appears that they function to regulate expression of the target gene. See, e.g., U.S. Patent Publication No. 2004/0268441 A1 which was published on December 30, 2004.

The term "expression", as used herein, refers to the production of a functional end-product, be it mRNA or translation of mRNA into a polypeptide.

"Antisense inhibition" refers to the production of antisense RNA transcripts capable of suppressing the expression of the target protein. "Co-suppression" refers to the production of sense RNA transcripts capable of suppressing the expression of identical or substantially similar foreign or endogenous genes (U.S. Patent No. 5,231,020).

"Overexpression" refers to the production of a functional end-product in transgenic organisms that exceeds levels of production when compared to expression of that functional end-product in a normal, wild type or non-transformed organism.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms.

Standard recombinant DNA and molecular cloning techniques used herein are well known in the art and are described by Sambrook et al.,Molecular Cloning: A Laboratory Manual, 2nd ed., Cold Spring Harbor Laboratory: Cold Spring Harbor, NY (1989); by Silhavy et al.,Experiments with Gene Fusions, Cold Spring Harbor Laboratory: Cold Spring Harbor, NY (1984); and by Ausubel et al., Current Protocols in Molecular Biology, published by Greene Publishing Assoc. and Wiley-Interscience (1987). Once the recombinant construct has been made, it may then be introduced into a plant cell or yeast cell of choice by methods well known to those of ordinary skill in the art including, for example, transfection, transformation and electroporation (see below). Oilseed plant cells are the preferred plant cells. The transformed plant cell is then cultured and regenerated under suitable conditions permitting expression of the recombinant construct which is then recovered and purified.

Recombinant constructs may be introduced into one plant cell or, alternatively, a construct may be introduced into separate plant cells.

Expression in a plant cell may be accomplished in a transient or stable fashion as is described above.

Plant parts include differentiated and undifferentiated tissues, including but not limited to: roots, stems, shoots, leaves, pollen, seeds, tumor tissue, and various forms of cells and culture such as single cells, protoplasts, embryos, and callus tissue. The plant tissue may be in plant or in organ, tissue or cell culture.

The term "plant organ" refers to plant tissue or group of tissues that constitute a morphologically and functionally distinct part of a plant. The term "genome" refers to the following: 1. The entire complement of genetic material (genes and non-coding sequences) is present in each cell of an organism, or virus or organelle. 2. A complete set of chromosomes inherited as a (haploid) unit from one parent. The term "stably integrated" refers to the transfer of a nucleic acid fragment into the genome of a host organism or cell resulting in genetically stable inheritance.

Methods for transforming dicots, primarily by use of *Agrobacterium tumefaciens*, and obtaining transgenic plants have been published, among others, for cotton (U.S. Patent No. 5,004,863, U.S. Patent No. 5,159,135); soybean (U.S. Patent No. 5,569,834, U.S. Patent No. 5,416,011); *Brassica* (U.S. Patent No. 5,463,174); peanut (Cheng et al. (1996) Plant Cell Rep. 15:653-657, McKently et al. (1995) Plant Cell Rep. 14:699-703); papaya (Ling et al. (1991) Bio/technology 9:752-758); and pea (Grant et al. (1995) Plant Cell Rep. 15:254-258). For a review of other commonly used methods of plant transformation see Newell (2000) Mol. Biotechnol. 16:53-65. One of these methods of transformation uses *Agrobacterium rhizogenes* (Tepfler, and Casse-Delbart (1987) Microbiol. Sci. 4:24-28). Transformation of soybeans using direct delivery of DNA has been published using PEG fusion (PCT publication WO 92/17598), electroporation (Chowrira et al. (1995) Mol. Biotechnol. 3:17-23; Christou et al. (1987) Proc. Natl. Acad. Sci. U.S.A. 84:3962-3966), microinjection, or particle bombardment (McCabe et. al. (1988) Bio/Technology 6:923; Christou et al. (1988) Plant Physiol. 87:671-674).

There are a variety of methods for the regeneration of plants from plant tissue. The particular method of regeneration will depend on the starting plant tissue and the particular plant species to be regenerated. The regeneration, development and cultivation of plants from single plant protoplast transformants or from various transformed explants is well known in the art (Weissbach and Weissbach, (1988) In.: Methods for Plant Molecular Biology, (Eds.), Academic: San Diego, CA). This regeneration and growth process typically includes the steps of selection of transformed cells, culturing those individualized cells through the usual stages of embryonic development through the rooted plantlet stage. Transgenic embryos and seeds are similarly regenerated. The resulting transgenic rooted shoots are thereafter planted in an appropriate plant growth medium such as soil. Preferably, the regenerated plants are self-pollinated to provide homozygous transgenic plants. Otherwise, pollen obtained from the regenerated plants is crossed to seed-grown plants of agronomically important lines. Conversely, pollen from plants of these important lines is used to pollinate regenerated plants. A transgenic plant useful in the present invention containing a desired polypeptide is cultivated using methods well known to one skilled in the art.

In addition to the above discussed procedures, practitioners are familiar with the standard resource materials which describe specific conditions and procedures for the construction, manipulation and isolation of macromolecules (e.g., DNA molecules, plasmids, etc.), generation of recombinant DNA fragments and recombinant expression constructs and the screening and isolating of clones, (see for example, Sambrook et al. (1989) Molecular Cloning: A Laboratory Manual, Cold Spring Harbor: NY; Maliga et al. (1995) Methods in Plant Molecular Biology, Cold Spring Harbor: NY; Birren et al. (1998) Genome Analysis: Detecting Genes, 1, Cold Spring Harbor: NY; Birren et al. (1998) Genome Analysis: Analyzing DNA, 2, Cold Spring Harbor:NY; Plant Molecular Biology: A Laboratory Manual, eds. Clark, Springer: NY (1997)).

In one aspect, the present invention provides methods for producing protein products derived from high oleic soybeans.

The present invention includes a method of preparing a blended soy protein product.

The various soy protein products produced by the method can be blended with other protein products, vegetable oils, PUFAs or combinations thereof to alter or improve the protein products to suit desirable characteristics, such as, but not limited to, fatty acid composition, flavor, color, viscosity, gel strength or drying efficiency. The amount of other protein products, vegetable oils, PUFAs or combinations thereof which can be used depends on the desired functionality of the final product.

A first soy protein product may be obtained from high oleic soybean defatted flakes, by the method described above by preparing defatted flakes from a high oleic soybean; adding PUFAs; and measuring the viscosity of the soy protein product; wherein said first soy protein product has increased viscosity when compared to a second soy protein product obtained from high oleic soybean defatted flakes without addition of PUFAs, and further wherein the same process is used to produce the first and second protein product. In some products the added PUFA content may be about 0.066% to about 3.85% by weight of the flake.

Useful examples of the PUFA content (percent weight) of the various protein products, either added or present in native form or a combination thereof are 0.05%, 0.051%, 0.052%, 0.053%, 0.054%, 0.055, 0.056%, 0.057%, 0.058%, 0.059%, 0.06%, 0.061%, 0.062%, 0.063%, 0.064%, 0.065, 0.066%, 0.067%, 0.068%, 0.069%, 0.07%, 0.071%, 0.072%, 0.073%, 0.074%, 0.075, 0.076%, 0.077%, 0.078%, 0.079%, 0.08%, 0.081%, 0.082%, 0.083%, 0.084%, 0.085, 0.086%, 0.087%, 0.088%, 0.089%, 0.09%, 0.091 %, 0.092%, 0.093%, 0.094%, 0.095, 0.096%, 0.097%, 0.098%, 0.099%, 0.1%, 0.11%, 0.12%, 0.13%, 0.14%, 0.15, 0.16%, 0.17%, 0.18%, 019%, 0.2%, 0.21%, 0.22%, 0.23%, 0.24%, 0.25, 0.26%, 0.27%, 0.28%, 0.29%, 0.3%, 0.31%, 0.32%, 0.33%, 0.34%, 0.35, 0.36%, 0.37%, 0.38%, 0.39%, 0.4%, 0.41%, 0.42%, 0.43%, 0.44%, 0.45, 0.46%, 0.47%, 0.48%, 0.49%, 0.5%, 0.51%, 0.52%, 0.53%, 0.54%, 0.55, 0.56%, 0.57%, 0.58%, 0.59%, 0.6%, 0.61 %, 0.62%, 0.63%, 0.64%, 0.65, 0.66%, 0.67%, 0.68%, 0.69%, 0.7%, 0.71%, 0.72%, 0.73%, 0.74%, 0.75, 0.76%, 0.77%, 0.78%, 0.79%, 0.8%, 0.81%, 0.82%, 0.83%, 0.84%, 0.85, 0.86%, 0.87%, 0.88%, 0.89%, 0.9%, 0.91%, 0.92%, 0.93%, 0.94%, 0.95, 0.96%, 0.97%, 0.98%, 0.99%, 1%, 1.01%, 1.02%, 1.03%, 1.04%, 1.05%, 1.06%, 1.07%, 1.08%, 1.09%, 1.1%, 1.11%, 1.12%, 1.13%, 1.14%, 1.15%, 1.16%, 1.17%, 1.18%, 1.19%, 1.2%, 1.21%, 1.22%, 1.23%, 1.24%, 1.25%, 1.26%, 1.27%, 1.28%, 1.29%, 1.3%, 1.31%, 1.32%, 1.33%, 1.34%, 1.35%, 1.36%, 1.37%, 1.38%, 1.39%, 1.4%, 1.41%, 1.42%, 1.43%, 1.44%, 1.45%, 1.46%, 1.47%, 1.48%, 1.49%, 1.5%, 1.51%, 1.52%, 1.53%, 1.54%, 1.55%, 1.56%, 1.57%, 1.58%, 1.59%, 1.6%, 1.61%, 1.62%, 1.63%, 1.64%, 1.65%, 1.66%, 1.67%, 1.68%, 1.69%, 1.7%, 1.71%, 1.72%, 1.73%, 1.74%, 1.75%, 1.76%, 1.77%, 1.78%, 1.79%, 1.8%, 1.81%, 1.82%, 1.83%, 1.84%, 1.85%, 1.86%, 1.87%, 1.88%, 1.89%, 1.9%, 1.91%, 1.92%, 1.93%, 1.94%, 1.95%, 1.96%, 1.97%, 1.98%, 1.99%, 2%, 2.01%, 2.02%, 2.03%, 2.04%, 2.05%, 2.06%, 2.07%, 2.08%, 2.09%, 2.1%, 2.11%, 2.12%, 2.13%, 2.14%, 2.15%, 2.16%, 2.17%, 2.18%, 2.19%, 2.2%, 2.21%, 2.22%, 2.23%, 2.24%, 2.25%, 2.26%, 2.27%, 2.28%, 2.29%, 2.3%, 2.31%, 2.32%, 2.33%, 2.34%, 2.35%, 2.36%, 2.37%, 2.38%, 2.39%, 2.4%, 2.41%, 2.42%, 2.43%, 2.44%, 2.45%, 2.46%, 2.47%, 2.48%, 2.49%, 2.5%, 2.51%, 2.52%, 2.53%, 2.54%, 2.55%, 2.56%, 2.57%, 2.58%, 2.59%, 2.6%, 2.61%, 2.62%, 2.63%, 2.64%, 2.65%, 2.66%, 2.67%, 2.68%, 2.69%, 2.7%,2.71%, 2.72%, 2.73%, 2.74%, 2.75%, 2.76%, 2.77%, 2.78%, 2.79%, 2.8%, 2.81%, 2.82%, 2.83%, 2.84%, 2.85%, 2.86%, 2.87%, 2.88%, 2.89%, 2.9%, 2.91%, 2.92%, 2.93%, 2.94%, 2.95%, 2.96%, 2.97%, 2.98%, 2.99%, 3%, 3.01%,3.02%, 3.03%, 3.04%, 3.05%,3.06%, 3.07%, 3.08%, 3.09%, 3.1%, 3.11%,3.12%, 3.13%, 3.14%, 3.15%,3.16%, 3.17%, 3.18%, 3.19%, 3.2%, 3.21%,3.22%, 3.23%, 3.24%, 3.25%, 3.26%, 3.27%, 3.28%, 3.29%, 3.3%, 3.31%, 3.32%, 3.33%, 3.34%, 3.35%,3.36%, 3.37%, 3.38%, 3.39%, 3.4%, 3.41%, 3.42%, 3.43%, 3.44%, 3.45%, 3.46%, 3.47%, 3.48%, 3.49%, 3.5%, 3.51%, 3.52%, 3.53%, 3.54%, 3.55%, 3.56%, 3.57%, 3.58%, 3.59%, 3.6%, 3.61%, 3.62%, 3.63%, 3.64%, 3.65%, 3.66%, 3.67%, 3.68%, 3.69%, 3.7%, 3.71%,3.72%, 3.73%, 3.74%, 3.75%, 3.76%, 3.77%, 3.78%, 3.79%, 3.8%, 3.81%, 3.82%, 3.83%, 3.84%, 3.85%,3.86%, 3.87%, 3.88%, 3.89%, 3.9%, 3.91%, 3.92%, 3.93%, 3.94%, 3.95%, 3.96%, 3.97%, 3.98%, 3.99%. The amount and range of PUFAS which can be used will depend upon the desired properties sought to be achieved in the resulting final blended protein product. One of a preferred PUFA content range is of about 0.17% to about 1.65% by weight.

When an amount, concentration, or other value or parameter is given as either a range, preferred range or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

When the term "about" is used in describing a value or an end-point of a range, the disclosure should be understood to include the specific value or end-point referred to.

Another method may concern a first soy protein product obtained from commodity soybean defatted flakes and at least one other different soybean defatted flake with a combined PUFA content of about 0.17% to about 1.65% by weight, wherein said first product, has reduced viscosity of at least 25%, 26%, 27%, 28%, 29%, 30%, 31%, 32%, 33%, 34%, 35%, 36%, 37%, 38%, 39%, 40%, 41%,42%, 43%, 44%, 45%, 46%, 47%, 48%, 49%, 50%, 51%, 52%, 53%, 54%, 55%, 56%, 57%, 58%, 58%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, or 87% when compared to a second soy protein product obtained from the commodity soybean defatted flakes, and further wherein the same process is used to produce the first and second protein product.

A blended protein product may be produced using the methods described above, wherein a first soy protein product obtained from commodity soybean defatted flakes and at least one other different soybean defatted flake may have a combined PUFA content of about 0.17% to about 1.65% by weight, wherein the whiteness index is increased by at least 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, 25%, 25.5%, 26%, 26.5%, 27%, 27.5%, 28%, 28.5%, 29%, 29.5%, 30%, 30.5%, 31%, 31.5%, 32%, 32.5%, 33%, 33.5%, 34%, 34.5%, 35%, 35.5%, 36%, 36.5%, 37%, 37.5%, 38%, 38.5%, 39%, 39.5%, 40%, 40.5%, 41%, 41.5%, 42%, 42.5%, 43%, 44%, 44.5%, 45%, 45.5%, 46%, 46.5%, 47%, 47.5%, 48%, 48.5%, 49%, 49.5% or 50% when compared to a second soy protein product obtained from the commodity soybean defatted flakes, and further wherein the same process is used to produce the first and second protein product.

Another blended protein product may be produced using the methods described above, wherein a first soy protein product obtained from commodity soybean defatted flakes and at least one other different soybean defatted flake may have a combined PUFA content of about 0.17% to about 1.65% by weight, wherein said first product, has an increased
drying efficiency of at least 1%, 1.5%, 2%, 2.5%%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, 25%, 25.5%, 26%, 26.5%, 27%, 27.5%, 28%, 28.5%, 29%, 29.5%, and 30% when compared to a second soy protein product obtained from the commodity soybean defatted flakes, and further wherein the same process is used to produce the first and second protein product.

A first soy protein product obtained from commodity soybean defatted flakes and at least one other different soybean defatted flake with a combined PUFA content of about 0.17% to about 1.65% by weight, wherein said first product, has the other different soybean defatted flakes, and further wherein the same process is used to produce the first and second protein product.

A first soy protein product obtained from a commodity soybean defatted flake and at least one other different soybean defatted flake with a combined PUFA content of more than 1.65% by weight, wherein said first product, has increased gel strength when compared to a second soy protein product obtained from the other different soybean defatted flakes, and further wherein the same process is used to produce the first and second protein product.

Useful examples of altered (either reduced or increased) gel strength of the protein product are alterations in gel strength by at least 25%, 26%, 27%, 28%, 29%, 30%, 31%, 32%, 33%, 34%, 35%, 36%, 37%, 38%, 39%. 40%, 41%, 42%, 43%, 44%, 45%, 46%, 47%, 48%, 49%, 50%, 51%, 52%, 53%, 54%, 55%, 56%, 57%, 58%, 59%, or 60%.

One advantage to having reduced viscosity is that it improves drying efficiency. Currently with commodity soy there is a limitation in the feed solids concentration that can be fed to the dryer as a result of the viscosity and propensity to aggregate and form a gel as a result of exposure to heat. If commodity soy must be dried at high solids, it is necessary to increase the temperature of the feed solids to prevent protein aggregation with resultant gelling; this increased heat is costly and results in severe damage to the solubility of the protein.

Reduced viscosity and gel properties allow the operator to significantly increase the feed solid concentration, because the slurry can be easily pumped through the equipment at normal temperatures without gelling. That means that during the drying process, less water has to be removed for every pound fed to the dryer. This translates into decreased energy usage and more solids that can be dried per hour resulting in more protein product for sale.

Soy protein products selected from the group consisting of a soy protein isolate, a soy protein concentrate, soy meal, full fat flour, defatted flour, soymilk, textured proteins, textured flours, textured concentrates and textured isolates may be produced using the claimed method.

As used herein, "soymilk" refers to an aqueous mixture of any one or more of the following, finely ground soybeans, soy flour, soy flakes, soy concentrate, isolated soy protein, soy whey protein, and aqueous extracts of any one or more of isolated soy protein, soy whey protein, and aqueous extracts of any one or more of the following, soybeans, soy flakes and soy flour where insoluble material has been removed. Soymilk may comprise additional components including but not limited to fats, carbohydrates, sweeteners, colorants, stabilizers, thickeners, flavorings, acids, bases.

As used herein, "soymilk powder" refers to a dewatered soymilk. Soymilk may be dewatered by many processes that include but are not limited to spray drying, tray drying, tunnel drying, and freeze drying.

A method for improving drying efficiency of a soy protein product may comprise feeding at least one soy protein product obtained from a high oleic soybean seed at higher feed solids to a pasteurizer or a dryer compared to feeding at least one soy protein product obtained from a commodity soybean to a pasteurizer or dryer.

A method for improving drying efficiency may comprise feeding a first blended soy protein product to a pasteurizer or a dryer at no less than 10%, 11%, 12, 13%, 14%, 15%, 15%, 17%, 18%, 19%, 20%, 21%, 22%, 23%, 24%, 25%, 26%, 27%, 28%, 29%, or 30% feed solids compared to feeding commodity soy protein products using the same process as that to obtain the soy protein product from a high oleic soybean.

Soy protein products fall into three major groups. These groups are based on protein content, and range from 40% to over 90%. All three basic soy protein product groups (except full-fat flours) are derived from defatted flakes. They are the following: soy flours and grits, soy protein concentrates and soy protein isolates. These are discussed more fully below.

As used herein the term "unhydrolyzed protein product", "unhydrolyzed soy protein product" refers to a protein product that has not undergone an enzymatic protein hydrolysis step.

As used herein the term "enzymatic hydrolysis" refers to the breakdown of proteins or chemical compounds by the addition of specific enzymes.

Blended soy protein products may contain at least 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%,%, 89% ,90%, 91%, 92%, 93%, 94%, 95%, 96% or 97% protein (N x 6.25) on a moisture-free basis.

The soy protein products produced by the method of the invention can be incorporated into food, beverages, and animal feed.

The term "animal feed" refers to food that is given to animals, such as livestock and pets. Some feeds provide a healthy and nutritious diet, while others may be lacking in nutrients. Animals are given a wide range of different feeds, but the two major types of animal feed are processed animal feeds (compound feed) and fodder.

Compound feeds are feedstuffs that are blended from various raw materials and additives. The main ingredients used in commercially prepared feed are the feed grains, which include corn, soybeans, sorghum, oats, and barley. These blends are formulated according to the specific requirements of the target animal (including different types of livestock and pets).
They are manufactured by feed compounders as meal type, pellets or crumbles.

Compound feeds can be complete feeds that provide all the daily required nutrients, concentrates that provide a part of the ration (protein, energy) or supplements that only provide additional micro-nutrients such as minerals and vitamins.

Oxidation and therefore the shelf life of animal feed ingredients is a common problem in the industry. Oxidation is an irreversible chemical reaction in which oxygen reacts with feed and feed components and can result in decreased animal health and performance. The negative effects of oxidation can be seen in loss of palatability, degradation of the oil component, development of unwanted breakdown products, changes in color, and loss of energy. Meat obtained from animals grown on oxidized feed has significantly lower oxidative status compared to animals fed a feed that has not undergone significant oxidation. Meat from animals fed diets containing high oleic corn products show extended shelf life and greater oxidative stability (PCT Publication WO/2006/002052, published January 5th, 2006), particularly when combined with antioxidants such as tocols. Therefore it is highly desirable to prevent oxidation of feed and feed ingredients to protect both nutritional value and organoleptic quality.

Synthetic antioxidants are used to preserve feed quality by preventing the oxidation of lipids, which can lead to improved animal performance. Generally, synthetic antioxidants can act as free radical scavengers and thereby reduce lipid oxidation. Synthetic antioxidants can prolong animal feed shelf-life and protect nutritional and organoleptic quality.

Methods for obtaining soy protein products are well known to those skilled in the art. For example soybean protein products can be obtained in a variety of ways. Conditions typically used to prepare soy protein isolates have been described by (Cho, et al, (1981) U.S. Patent No. 4,278,597; Goodnight, et al. (1978) U.S. Patent No. 4,072,670). Soy protein concentrates are produced by three basic processes: acid leaching (at about pH 4.5), extraction with alcohol (about 55-80%), and denaturing the protein with moist heat prior to extraction with water. Conditions typically used to prepare soy protein concentrates have been described by Pass ((1975) U.S. Patent No. ,897,574) and Campbell et al. ((1985) in New Protein Foods, ed. by Altschul and Wilcke, Academic Press, Vol., Chapter 10, Seed Storage Proteins, pp 302-338).

"Soybean-containing products" or "Soy products" can be defined as those products containing/incorporating a soy protein product.

For example, "soy protein products" can include, and are not limited to, those items listed in Table 2.

**TABLE 2**

| Soy Protein Products Derived from Soybean Seeds^{a} | |
|---|---|
| Whole Soybean Products | Processed Soy Protein Products |
| Roasted Soybeans | Full Fat and Defatted Flours |
| Baked Soybeans | Soy Grits |
| Soy Sprouts | Soy Hypocotyls |
| Soy Milk | Soybean Meal |
| | Soy Milk |
| | Soy Milk Powder |
| | Soy Protein Isolates |

| Specialty Soy Foods/lngredients | |
|---|---|
| Soy Milk | Soy Protein Concentrates |
| Tofu | Textured Soy Proteins |
| Tempeh | Textured Flours and Concentrates |
| Miso | Textured Concentrates |
| Soy Sauce | Textured Isolates |
| Hydrolyzed Vegetable Protein | Soy Crisps |
| Whipping Protein | |

| | |
|---|---|
| ^{a}See Soy Protein Products: Characteristics, Nutritional Aspects and Utilization (1987). Soy Protein Council. | |

"Processing" refers to any physical and chemical methods used to obtain the products listed in Table 2 and includes, and is not limited to, heat conditioning, flaking and grinding, extrusion, solvent extraction, or aqueous soaking and extraction of whole or partial seeds. Furthermore, "processing" includes the methods used to concentrate and isolate soy protein from whole or partial seeds, as well as the various traditional Oriental methods in preparing fermented soy food products. Trading Standards and Specifications have been established for many of these products (see National Oilseed Processors Association Yearbook and Trading Rules 1991-1992).

Defatted flakes refer to flaked, dehulled cotyledons that have been defatted and treated with controlled heat to remove the remaining hexane. This term can also refer to a flour or grit that has been ground.

"White" flakes refer to flaked, dehulled cotyledons that have been defatted and treated with controlled heat to remove the remaining hexane. This term can also refer to a flour that has been ground.

"Grits" refer to defatted, dehulled cotyledons having a U.S. Standard screen size of between No. 10 and 80.

"Soy Protein Concentrates" refer to those products produced from dehulled, defatted soybeans and typically contain 65 wt % to 90 wt % soy protein on a moisture free basis. Soy protein concentrates are typically manufactured by three basic processes: acid leaching (at about pH 4.5), extraction with alcohol (about 55-80%), and denaturing the protein with moist heat prior to extraction with water. Conditions typically used to prepare soy protein concentrates have been described by Pass (1975) U.S. Patent No. 3,897,574; Campbell et al., (1985) in New Protein Foods, ed. by Altschul and Wilcke, Academic Press, Vol. 5, Chapter 10, Seed Storage Proteins, pp 302-338).

As used herein, the term "soy protein isolate" or "isolated soy protein" refers to a soy protein containing material that contains at least 90% soy protein by weight on a moisture free basis.

"Extrusion" refers to processes whereby material (grits, flour or concentrate) is passed through a jacketed auger using high pressures and temperatures as a means of altering the texture of the material. "Texturing" and "structuring" refer to extrusion processes used to modify the physical characteristics of the material. The characteristics of these processes, including thermoplastic extrusion, have been described previously (Atkinson (1970) U.S. Patent No. 3,488,770, Horan (1985) In New Protein Foods, ed. by Altschul and Wilcke, Academic Press, Vol. 1A, Chapter 8, pp 367-414). Moreover, conditions used during extrusion processing of complex foodstuff mixtures that include soy protein products have been described previously (Rokey (1983) Feed Manufacturing Technology III, 222-237; McCulloch, U.S. Patent No. 4,454,804).

Residual fatty acid analysis. The commercial process used to de-fat soy flakes with hexane leaves a residue of fatty acids that can act as substrate for generation of off-flavor compounds. Depending on the method of analysis, the residual fat content of hexane-defatted soy flakes can range from, 0.6-1.0% (W:W) (ether extractable; AOCS Method 920.39 (Official Methods of Analysis of the AOAC International (1995), 16^{th} Edition, Method 920.39C, Locator #4.2.01 (modified)) to 2.5 - 3% (W:W) (acid hydrolysable; AOAC Method 922.06 (Official Methods of Analysis of the AOAC International (1995), 16^{th} Edition, Method 922.06, Locator 32.1.13 (modified)). The principle reason for the discrepancy between these two methods of estimating residual fatty acids is the chemical nature of the fat classes associated with the protein matrix after hexane extraction. A small proportion of the residual fatty acid is in the form of neutral lipid (i.e., triglyceride) and the remainder is present as polar lipid (e.g., phospholipids, a.k.a., lecithin). Because of its polar nature the phospholipid is inaccessible to ether extraction and is only removed from the protein matrix if acid hydrolysis or some other stringent extraction protocol is performed. Therefore, the ether extraction technique gives an estimation of the neutral lipid fraction whereas the acid hydrolysable method gives a better estimate of the total residual fatty acid content (i.e., neutral and polar fractions).

Both of the AOAC methods described above rely on gravimetric determinations of the residual fatty acids and, although in combination they give an indication of the fat classes (neutral vs. polar), such estimates are crude and are subject to interference from other hydrophobic materials (e.g. saponins). Further, no information is obtained on the fatty acid composition and how it may have been affected by various experimental treatments or by the genetics of the starting material. AOAC methods for the determination of the fatty acid composition of residual fatty acids are available (Official Methods of Analysis of the AOAC International (2000), 17^{th} Edition, Method 983.23 Locator 45.4.02, Method 969.33 Locator 41.1.28, Method 996.06 Locator 41.1.28A). These are based on the conversion of residual fatty acids, extracted by acid hydrolysis, to fatty acid methyl esters prior to analysis by gas chromatography. Such techniques are rarely used to assess the residual fatty acid content of food materials in commercial settings although they are used for fatty acid evaluations in support of nutritional labeling. A report in which these methods have been used to determine the residual fatty acid composition of commercial soy protein isolates has recently been published (Solina et al. (2005) Volatile aroma components of soy protein isolate and acid-hydrolysed vegetable protein Food Chemistry 90: 861-873)

A facile method for determining the fatty acid composition of the residual fats in soy protein products is described in Example 24. The advantage of this method over others is that it requires no extraction of the residual fats from the matrix prior to derivatization for GC analysis. Further, the technique is suitable for all forms of fatty acids i.e., whether they are initially present as free fatty acids or as fatty acid esters e.g., tri-glycerides or phospholipids (Chistie (1989) Gas Chromatography and Lipids; The Oily Press. Ayr, Scotland). The technique will also remove fatty acids from the protein matrix even if the polar head group of the phospholipid is covalently bound to the protein.

Food, food supplements, food bars, and beverages as well as animal feed (such as pet foods) may incorporate therein a soybean protein product. The beverage can be in a liquid or in a dry powdered form.

The foods to which soybean protein products can be incorporated/added include almost all foods, beverages and feed (such as pet
foods). For example, there can be mentioned food supplements, food bars, meats such as meat alternatives, ground meats, emulsified meats, marinated meats, and meats injected with a soybean protein product of the invention. Included may be beverages such as nutritional beverages, sports beverages, protein-fortified beverages, juices, milk, milk alternatives, and weight loss beverages. Mentioned may also be cheeses such as hard and soft cheeses, cream cheese, and cottage cheese. Included may also be frozen desserts such as ice cream, ice milk, low fat frozen desserts, and non-dairy frozen desserts. Finally, yogurts, soups, puddings, bakery products, salad dressings, spreads, and dips (such as mayonnaise and chip dips) may be included.

A soy protein product can be added in an amount selected to deliver a desired amount to a food and/or beverage. The terms "soybean protein product" and "soy protein product" are used interchangeably herein.

Any soybean seed with a combined linoleic and linolenic acid content less than that of commodity soybeans (about 50-60% on a total oil basis), whether it was obtained by transgenic or non-transgenic means, can be used as a blending source of the soy protein product.

Soybeans with decreased levels of saturated fatty acids have been described resulting from mutation breeding (Erickson et al. (1994) J. Hered. 79:465-468; Schnebly et al. (1994) Crop Sci. 34:829-833; and Fehr et al. (1991) Crop Sci. 31:88-89) and transgenic modification (U.S. Patent No. 5,530,186). Soybeans with decreased levels of polyunsaturated fatty acids have been described resulting from mutation breeding and selection. Reduced levels of linolenic acid have been achieved at relatively constant linoleic acid (U.S. Patent No. 5,710,369 and U.S. Patent No. 5,986,118). Decreased linoleic and linolenic acids combined have also been achieved using mutation breeding, genetic crosses and selection (Rahman, S. M. et al. (2001) Crop Sci. 41:26-29). These methods produced soybean seeds with oil profiles having linolenic acid contents of from 1% to 3% of the total fatty acids and total levels of polyunsaturated fatty acids of about 30 to 35% as compared to greater than 6% linolenic acid and greater than 50% total polyunsaturated fatty acids in commodity soybeans.

The discovery of a method for altering the expression of the enzymes responsible for introduction of the second (international patent publication WO 94/11516) and third (international patent publication WO 93/11245) double bonds into soybean seed storage lipid in a directed manner has allowed the production of soybeans with a high mono-unsaturated, very low polyunsaturated fatty acid content and especially a very low linolenic acid content. The genetic combination of these two transgene profiles described in U.S. Patent No. 6,426,448 leads to a soybean line with minimal poly-unsaturates and high mono-unsaturates and extreme environmental stability of the seed fatty acid profile.

The gene for microsomal delta-12 fatty acid desaturases described in WO 94/11516, can be used to make a high oleic acid soybean variety. The resulting high oleic acid soybean variety was one in which the polyunsaturated fatty acids were reduced from 70% of the total fatty acids to less than 5%.

Two soybean fatty acid desaturases, designated FAD2-1 and FAD2-2, are Δ-12 desaturases that introduce a second double bond into oleic acid to form linoleic acid, a polyunsaturated fatty acid. FAD2-1 is expressed only in the developing seed (Heppard et al. (1996) Plant Physiol. 110:311-319). The expression of this gene increases during the period of oil deposition, starting around 19 days after flowering, and its gene product is responsible for the synthesis of the polyunsaturated fatty acids found in soybean oil. GmFad 2-1 is described in detail by Okuley, J. et al. (1994) Plant Cell 6:147-158 and in WO94/11516. It is available from the ATCC in the form of plasmid pSF2-169K (ATCC accession number 69092). FAD 2-2 is expressed in the seed, leaf, root and stem of the soy plant at a constant level and is the "housekeeping" 12-desaturase gene. The Fad 2-2 gene product is responsible for the synthesis of polyunsaturated fatty acids for cell membranes.

Since FAD2-1 is the major enzyme of this type in soybean seeds, reduction in the expression of FAD2-1 results in increased accumulation of oleic acid (18:1) and a corresponding decrease in polyunsaturated fatty acid content.

Reduction of expression of FAD2-2 in combination with FAD2-1 leads to a greater accumulation of oleic acid and corresponding decrease in polyunsaturated fatty acid content.

FAD3 is a Δ-15 desaturase that introduces a third double bond into linoleic acid (18:2) to form linolenic acid (18:3). Reduction of expression of FAD3 in combination with reduction of FAD2-1 and FAD2-2 leads to a greater accumulation of oleic acid and corresponding decrease in polyunsaturated fatty acid content, especially linolenic acid.

Nucleic acid fragments encoding FAD2-1, FAD2-2, and FAD3 have been described in WO 94/11516 and WO 93/11245. Chimeric recombinant constructs comprising all or a part of these nucleic acid fragments or the reverse complements thereof operably linked to at least one suitable regulatory sequence can be constructed wherein expression of the chimeric gene results in an altered fatty acid phenotype. A chimeric recombinant construct can be introduced into soybean plants via transformation techniques well known to those skilled in the art.

Transgenic soybean plants resulting from a transformation with a recombinant DNA are assayed to select plants with altered fatty acid profiles. The recombinant construct may contain all or part of 1) the FAD2-1 gene or 2) the FAD2-2 gene or 3) the FAD3 gene or 4) combinations of all or portions of the FAD2-1, Fad2-2, or FAD3 genes.

Recombinant constructs comprising all or part of 1) the FAD2-1 gene with or without 2) all or part of the Fad2-2 gene with or without all or part of the FAD3 gene can be used in making a transgenic soybean plant having a high oleic phenotype. An altered fatty acid profile, specifically an increase in the proportion of oleic acid and a decrease in the proportion of the polyunsaturated fatty acids, indicates that one or more of the soybean seed FAD genes (FAD2-1, Fad2-2, FAD3) have been suppressed. Assays may be conducted on soybean somatic embryo cultures and seeds to determine suppression of FAD2-1, Fad2-2, or FAD3.

It is well understood by those skilled in the art that recombinant constructs comprising sequences other than those specifically exemplified which have similar functions, may be used. These constructs may include any seed-specific promoter. These constructs may or may not also include any nucleotides that promote stem-loop formation. These constructs may contain a polynucleotide having a nucleotide sequence identical to any portion of the gene or genes mentioned above inserted in sense or anti-sense orientation with respect to the promoter. Finally, these constructs may or may not contain any transcription termination signal.

It was observed that when the commodity and high oleic flake slurries were mixed together and processed further to the neutralized curd slurry, the slurry viscosity was much lower. With as little as 40% high oleic flake, there was a large drop in viscosity of the resultant in-process slurry. This allows the functionality of high oleic isolate to be obtained by blending only a small amount of it with the commodity isolate. It also shows processing benefits like improvement in the solids to dryer due to drastically reduced viscosity of the mixture. One could also gain similar viscosity manipulation benefits by modulating the fatty acid profiles by genetic manipulation or by selective breeding, e.g. mid oleic beans. The resultant isolates may be used for various applications ranging from milk like products e.g. soy milk with viscosities in range of 1-10 cP to puddings or applications like gels and meat analogues.

Surprisingly, it has been found that addition of a small amount of polyunsaturated free fatty acids, such as a mixture of linoleic and linolenic acids, to defatted high oleic flake at the time of extraction of the protein leads to a dramatic increase in the viscosity of the extracted soy protein. The combination of appropriate levels of PUFAs and high oleic flake can therefore be used to generate soy proteins having any desired viscosity between the low value of isolate from high oleic flake and the high value of isolate obtained from commodity flake. These isolated soy proteins with different viscosities are desirable for different food applications, for example most beverages require lower viscosity while some beverages and solid foods require higher viscosity.

The viscosity of the in-process protein was found to increase incrementally in a non-linear fashion with increasing amounts of added PUFA mixture. The slope of a plot of the viscosity vs amount of added PUFA decreases with increasing PUFA. At higher levels of PUFA addition the viscosity of the in-process protein isolated from high oleic flake exceeded that isolated from the commodity flake.

The extent of the viscosity increase in the isolated protein is dependent on the degree of unsaturation of the added fatty acid. The viscosity of the in-process protein generally increased as the number of double bonds in the fatty acid increased.

The increase in viscosity with the addition of PUFAs was accompanied by a decrease in Whiteness index.

### EXAMPLES

The present invention is further defined in the following Examples, in which parts and percentages are by weight and degrees are Celsius, unless otherwise stated. It should be understood that these Examples, while indicating preferred embodiments of the invention, are given by way of illustration only.

The production of high oleic soybean lines is described in detail in Examples 3, 5 and 8, but is not limited to the methods described therein.

### EXAMPLE 1

### Transformation of Soybean (Glycine max) Embryo Cultures and Regeneration of Soybean Plants.

Soybean embryogenic suspension cultures are transformed by the method of particle gun bombardment using procedures known in the art (Klein et al. (1987) Nature (London) 327:70-73; U. S. Patent No. 4,945,050; Hazel et al. (1998) Plant Cell. Rep. 17:765-772; Samoylov et al. (1998) In Vitro Cell Dev. Biol.-Plant 34:8-13). In particle gun bombardment procedures it is possible to use purified 1) entire plasmid DNA or, 2) DNA fragments containing only the recombinant DNA expression cassette(s) of interest.

Stock tissue for transformation experiments are obtained by initiation from soybean immature seeds. Secondary embryos are excised from explants after 6 to 8 weeks on culture initiation medium. The initiation medium is an agar-solidified modified MS (Murashige and Skoog (1962) Physiol. Plant. 15:473-497) medium supplemented with vitamins, 2,4-D and glucose. Secondary embryos are placed in flasks in liquid culture maintenance medium and maintained for 7-9 days on a gyratory shaker at 26 +/- 2°C under ∼80 µEm-2s-1 light intensity. The culture maintenance medium is a modified MS medium supplemented with vitamins, 2,4-D, sucrose and asparagine. Prior to bombardment, clumps of tissue are removed from the flasks and moved to an empty 60X15 mm petri dish for bombardment. Tissue is dried by blotting on Whatman #2 filter paper. Approximately 100-200mg of tissue corresponding to 10-20 clumps (1-5 mm in size each) are used per plate of bombarded tissue.

After bombardment, tissue from each bombarded plate is divided and placed into two flasks of liquid culture maintenance medium per plate of bombarded tissue. Seven days post bombardment, the liquid medium in each flask is replaced with fresh culture maintenance medium supplemented with 100ng/ml selective agent (selection medium). For selection of transformed soybean cells the selective agent used can be a sulfonylurea (SU) compound with the chemical name, 2-chloro-N-((4-methoxy-6 methy-1,3,5-triazine-2-yl)aminocarbonyl) benzenesulfonamide (common names: DPX-W4189 and chlorsulfuron). Chlorsulfuron is the active ingredient in the DuPont sulfonylurea herbicide, GLEAN®. The selection medium containing SU is replaced every week for 6-8 weeks. After the 6-8 week selection period, islands of green, transformed tissue are observed growing from untransformed, necrotic embryogenic clusters. These putative transgenic events are isolated and kept in media with SU at 100 ng/ml for another 2-6 weeks with media changes every 1-2 weeks to generate new, clonally propagated, transformed embryogenic suspension cultures. Embryos spend a total of around 8-12 weeks in contact with SU. Suspension cultures are subcultured and maintained as clusters of immature embryos and also regenerated into whole plants by maturation and germination of individual somatic embryos.

### EXAMPLE 2

### Fatty acid analysis of Soybeans

In order to determine altered fatty acid composition as a result of suppression of the fatty acid desaturase, the relative amounts of the fatty acids, palmitic, stearic, oleic, linoleic and linolenic, can be determined as follows. Fatty acid methyl esters are prepared from single, mature, somatic soybean embryos or soybean seed chips by transesterification. One embryo, or a chip from a seed, is placed in a vial containing 50 µL of trimethylsulfonium hydroxide and incubated for 30 minutes at room temperature while shaking. After 30 minutes 0.5mL of hexane is added, the sample is mixed and allowed to settle for 15 to 30 minutes to allow the fatty acids to partition into the hexane phase. Fatty acid methyl esters (5µL from hexane layer) are injected, separated, and quantified using a Hewlett-Packard 6890 Gas Chromatograph fitted with an Omegawax 320 fused silica capillary column (Supelco Inc., Cat#24152). The oven temperature is programmed to hold at 220°C for 2.7 minutes, increase to 240°C at 20°C per minute, and then hold for an additional 2.3 minutes. Carrier gas is supplied with a Whatman hydrogen generator. Retention times were compared to those for methyl esters of commercially available standards (Nu-Chek Prep, Inc. catalog #U-99-A).

### EXAMPLE 3

### Production of Soybeans with High Levels Oleic Acid and/or High Levels of Stearic Acid and/or Low Levels of Polyunsaturated Fatty Acids by Suppression of Fatty Acid Desaturases

Recombinant DNA fragments were prepared and used in transformation of soybean for the simultaneous suppression of fatty acid desaturases FAD2-1 and FAD2-2 and fatty acid desaturase FAD3. A description of the construction of the recombinant DNA fragments follows.

### A. Recombinant DNA Fragment PHP21676A

Recombinant DNA fragment PHP21676A contains a gene expression silencing cassette designed to silence expression of the FAD2-1 and FAD2-2 genes, and the FAD3 gene, linked in a head to head configuration to the ALS selectable marker recombinant DNA fragment of Example 1D below. The nucleotide sequence of recombinant DNA fragment PHP21676A is shown in SEQ ID NO:1. Recombinant DNA fragment PHP21676A contains in 5' to 3' orientation:
a) the complementary strand of the ALS selectable marker recombinant DNA fragment of Example 1D below,
b) about 2088 nucleotides of the Kti3 promoter,
c) a 74-nucleotide synthetic sequence,
d) an approximately 1500 polynucleotide fragment comprising about 470 nucleotides from the soybean FAD2-2 gene, 420 nucleotides from the soybean FAD2-1 gene, and 643 nucleotides from the soybean FAD3 gene inserted at a unique Not I restriction endonuclease site,
e) an inverted repeat of the 74-nucleotide synthetic sequence in c), and
f) about 202 nucleotides of the Kti3 transcription terminator.

The sequence of the approximately 1500 polynucleotide fragment of item d) above is shown in SEQ ID NO:2. The approximately 1500 polynucleotide fragment comprising about 470 nucleotides from the soybean FAD2-2 gene, about 420 nucleotides from the soybean FAD2-1 gene, about 643 nucleotides from the soybean FAD3 gene was constructed by PCR amplification as follows.

An approximately 0.9 kb DNA fragment, comprising a portion of the soybean FAD2-2 gene and a portion of the soybean FAD2-1 gene, was obtained by PCR amplification using primers BM35 (SEQ ID NO:3) and BM39 (SEQ ID NO:4) and using as a template, recombinant DNA fragment KSFAD2-hybrid, described in Example 1B below.

An approximately 0.65 kb DNA fragment, comprising a portion of a FAD3 gene, was obtained by PCR amplification using primers BM40 (SEQ ID NO:5) and BM41 (SEQ ID NO:6) and using plasmid pXF1 as template. Plasmid pXF1 comprises a polynucleotide encoding a soybean delta-15 desaturase (FAD3) and is described in US Patent No. 5,952,544 issued on September 14, 1999. Plasmid pXF1 was deposited with the American Type Culture Collection (ATCC) of Rockville, MD on December 3, 1991 under the provisions of the Budapest Treaty, and bears Accession Number ATCC 68874.

The approximately 0.9 kb fragment, comprising a portion of the soybean FAD2-2 gene and a portion of the soybean FAD2-1 gene, and the approximately 0.65 kb fragment, comprising a portion of a FAD3 gene, were mixed and used as template for a PCR amplification with BM35 and BM41 as primers to yield an approximately 1533 bp fragment that was cloned into the commercially available plasmid pCR2.1 using the TOPO TA Cloning Kit (Invitrogen). After digestion with NotI the approximately 1500 bp fragment having the nucleotide sequence shown in SEQ ID NO:2 was ligated into the NotI site of plasmid pKS210 (Example 1C below).

### B. Recombinant DNA Fragment KSFAD2-hybrid

Recombinant DNA Fragment KSFAD2-hybrid contains an approximately 890 polynucleotide fragment comprising about 470 nucleotides from the soybean FAD2-2 gene and 420 nucleotides from the soybean FAD2-1 gene. The nucleotide sequence of recombinant DNA fragment KSFAD2-hybrid is shown in SEQ ID NO:7 Recombinant DNA Fragment KSFAD2-hybrid was constructed as follows.

An approximately 0.47 kb DNA fragment comprising a portion of the soybean FAD2-2 gene was obtained by PCR amplification using primers KS1 (SEQ ID NO:8) and KS2 (SEQ ID NO:9) and using genomic DNA purified from leaves of *Glycine max* cv. Jack as a template.

An approximately 0.42 kb DNA fragment comprising a portion of the soybean FAD2-1 gene was obtained by PCR amplification using primers KS3 (SEQ ID NO:10) and KS4 (SEQ ID NO:11) and using genomic DNA purified from leaves of *Glycine max* cv. Jack as a template.

The 0.47 kb fragment comprising a portion of the soybean FAD2-2 gene and the 0.42 kb fragment comprising a portion of the soybean FAD2-1 gene were gel purified using GeneClean (Qbiogene, Irvine, CA), mixed, and used as template for PCR amplification with KS1 and KS4 as primers to yield an approximately 890 bp fragment that was cloned into the commercially available plasmid pGEM-T Easy (Promega, Madison, WI) to create a plasmid comprising recombinant DNA Fragment KSFAD2-hybrid.

### C. Preparation of plasmid pKS210 and plasmid PHP17731

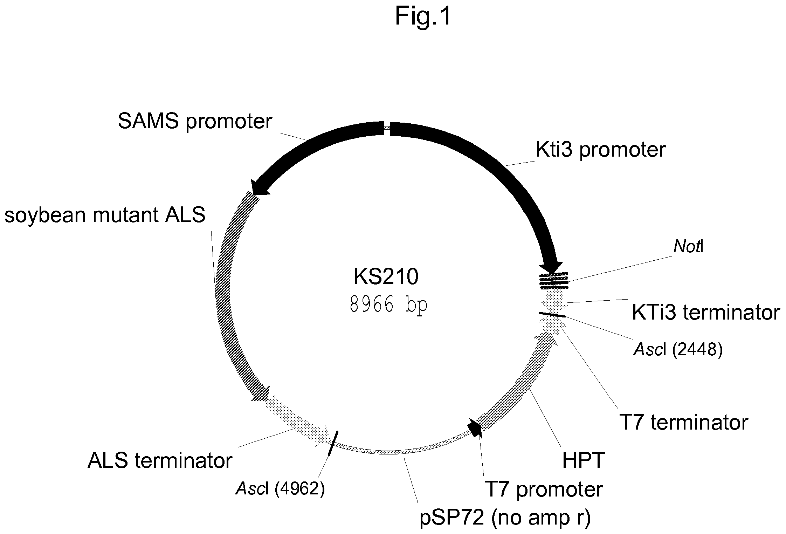

Plasmid pKS210 is derived from the commercially available cloning vector pSP72 (Promega). The beta lactamase coding region has been replaced by a hygromycin phosphotransferase gene for use as a selectable marker in *E. coli.* In addition, a gene expression silencing cassette linked in a head to head configuration to the ALS selectable marker recombinant DNA fragment of Example 1B has been added. The gene expression silencing cassette in plasmid pKS210 comprises the KTi3 promoter, a 74 nucleotide synthetic sequence, a unique NotI restriction endonuclease site, an inverted repeat of the 74 nucleotide synthetic sequence, and the Kti3 terminator region. The gene encoding Kti3 has been described (Jofuku and Goldberg (1989) Plant Cell 1:1079-1093). The 74-nucleotide synthetic sequences of c) and e) (above) promote formation of a stem structure. Insertion of a nucleotide fragment from a desired gene in the unique Not I site has been shown to result in suppression of the desired gene as described in PCT Publication WO 02/00904, published 03 January 2002. The nucleotide sequence of this seed-specific gene expression-silencing cassette from pKS133 is shown in SEQ ID NO:12. A map of plasmid pKS210 is shown in Fig. 1 and its nucleotide sequence is disclosed in SEQ ID NO:13.

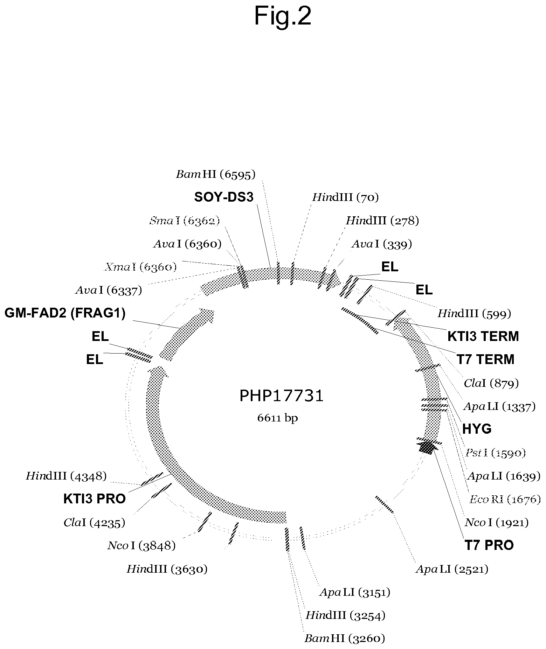

The recombinant plasmid PHP17731, containing gene sequences for the simultaneous silencing of one of the soybean delta-9 desaturase genes and the soybean delta-12 desaturase gene FAD2-1, was prepared. The soybean KTI promoter, terminator regions along with a synthetic inverted repeat sequence were taken from plasmid KS133 (WO 2002016565A2, A3). A fragment of the FAD2-1 gene was amplified by PCR using soybean genomic DNA and the sequence in SEQ ID NO 5 of US 6,372,965 B1 as template to produce the fragment of base pairs 5423 to 6033 of SEQ ID NO:14 (PHP17731). Adjacent to that fragment a portion of the coding sequence of copy 3 of the soybean delta-9 desaturase (sequence 1 of WO 2002016565A2, A3) was placed, which now comprises bases 6054 to 411 of PHP17731. Fragment PHP17731A (SEQ ID NO:15) was removed from cloning vector PHP17731 by digestion with restriction endonuclease AscI and purified as described in section E below. A map of plasmid PHP17731 is shown in Fig. 2.

### D. ALS Selectable Marker Recombinant DNA Fragment

A recombinant DNA fragment comprising a constitutive promoter directing expression of a mutant soybean acetolactate synthase (ALS) gene followed by the soybean ALS 3' transcription terminator was used as a selectable marker for soybean transformation. The constitutive promoter used is a 1.3-Kb DNA fragment that functions as the promoter for a soybean S-adenosylmethionine synthase (SAMS) gene and is described in PCT publication No. WO 00/37662 published 29 June 2000. The nucleotide sequence of this recombinant DNA fragment used as a selectable marker is shown in SEQ ID NO:16. The mutant soybean ALS gene encodes an enzyme that is resistant to inhibitors of ALS, such as sulfonylurea herbicides. The deduced amino acid sequence of the mutant soybean ALS present in the recombinant DNA fragment used as a selectable marker is shown in SEQ ID NO:17.

Mutant plant ALS genes encoding enzymes resistant to sulfonylurea herbicides are described in U. S. Patent No. 5,013,659. One such mutant is the tobacco SURB-Hra gene, which encodes a herbicide-resistant ALS with two substitutions in the amino acid sequence of the protein. This tobacco herbicide-resistant ALS contains alanine instead of proline at position 191 in the conserved "subsequence B" (SEQ ID NO:18) and leucine instead of tryptophan at position 568 in the conserved "subsequence F" (SEQ ID NO:19) (US Patent No. 5,013,659; Lee et al. (1988) EMBO J 7:1241-1248).

The ALS selectable marker recombinant DNA fragment was constructed using a polynucleotide for a soybean ALS to which the two Hra-like mutations were introduced by site directed mutagenesis. Thus, this recombinant DNA fragment will translate to a soybean ALS having alanine instead of proline at position 183 and leucine instead of tryptophan at position 560.

In addition, during construction of the SAMS promoter-mutant ALS expression cassette, the coding region of the soybean ALS gene was extended at the 5'-end by five additional codons, resulting in five amino acids (M-P-H-N-T; SEQ ID NO:20), added to the amino-terminus of the ALS protein. These extra amino acids are adjacent to and presumably removed with the transit peptide during targeting of the mutant soybean ALS protein to the plastid. A DNA fragment comprising a polynucleotide encoding the soybean ALS was digested with KpnI, blunt ended with T4 DNA polymerase, digested with SalI, and inserted into a plasmid containing the SAMS promoter which had been previously digested with NcoI and blunt ended by filling-in with Klenow DNA polymerase.

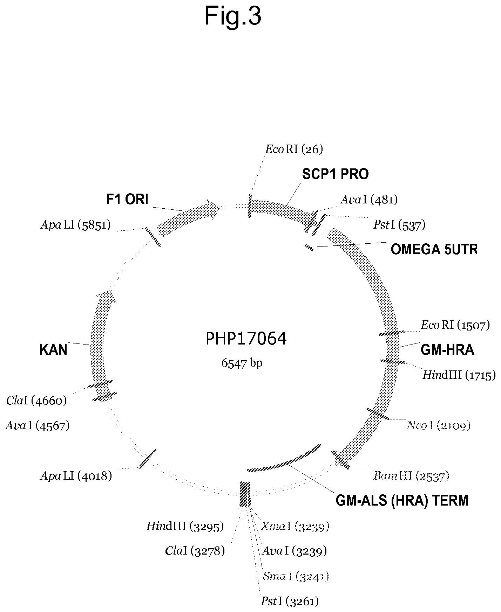

A second selectable marker plasmid and subsequent fragment was prepared by substituting an alternative constitutively expressed plant promoter for the SAMS promoter described above. The synthetic promoter SCP1 (US 6,072,050) was placed in front of the mutant soybean ALS coding sequence to form plasmid PHP17064 (SEQ ID NO: 21 and Fig. 3). For use in soybean transformation fragment PHP17064A (SEQ ID NO:22) was excised from its cloning vector using restriction endonuclease XbaI and purified as described in section E below.

### E. Preparation of Recombinant DNA Fragments, PHP21676A, PHP17731A and PHP17064A, for Soybean Transformation.

For use in plant transformation experiments, the 7993 bp recombinant DNA fragment PHP21676A was removed from its cloning plasmid using restriction endonuclease AscI. Each one of the recombinant DNA fragments PHP21676A, PHP17731A and PHP17064A was separated from the remaining plasmid DNA by agarose gel electrophoresis. Precipitation of the recombinant DNA fragment onto gold particles and soybean transformation was performed as described in Example 1. For every eight bombardment transformations, 30 µl of solution were prepared with 3 mg of 0.6 µm gold particles and 1 to 90 picograms (pg) of DNA fragment per base pair of DNA fragment.

Alternatively, mixtures of fragments PHP17064A and PHP17731A at either equal parts or two parts PHP17731A to PHP17064A were added to gold particles at the same weight per base pair as described above and used in transformation to silence the delta-9 and delta-12 desaturase genes.

### EXAMPLE 4

### Fatty acid analysis of Soybean Transformed with Recombinant DNA Fragments PHP21676A and with PHP17064A and PHP17731A combined.

In a soybean transformation experiment using recombinant DNA fragment PHP21676A as described above, 67 independently transformed embryogenic suspension cultures found to be resistant to sulfonylurea herbicide were obtained. An increase in oleic acid as a percentage of the five major fatty acids, palmitic, stearic, oleic, linoleic and linolenic, is indicative of suppression of the FAD2 genes. Thirteen of the 67 herbicide resistant embryogenic suspension cultures (19%) produced somatic embryos with greater than 25% oleic acid, compared to about 8% oleic acid for untransformed embryos.

Plants were regenerated and T1 seeds were produced from 9 of the 13 events. Seeds were tested for suppression of fatty acid desaturases by measuring fatty acid composition of the seed oil as described in Example 2. Plants derived from 5 transformation events produced seeds exhibiting the high oleic acid-low polyunsaturated fatty acid phenotype.

In a soybean transformation experiment, using the mixture of recombinant DNA fragments PHP17064A and PHP17731A, transformed embryogenic suspension cultures found to be resistant to sulfonylurea herbicide were obtained, screened for the number of copies of the transgene fragments present by southern analysis and then by fatty acid profile of the somatic embryo. A rise in the level of stearic and of oleic acid was taken as indicator of silencing of the seed expressed delta-9 desaturase and delta-12 desaturase. Thirty-three transformed candidate lines were regenerated to mature soybean plants and seed from the initial transformants was analyzed for fatty acid profile. From these lines further selections were made from seed obtained from selfed plants in two additional generations. One candidate line was chosen in which the sum of linoleic and linolenic acid was less than 14% of total fatty acids and in which the stearic acid content was greater than 16% of total fatty acids.

### EXAMPLE 5

### Genetic Material Used to Produce the High Oleic trait (version 1)

High oleic soybeans were prepared by recombinant manipulation of the activity of oleoyl 12-desaturase.

GmFad 2-1 was placed under the control of a strong, seed-specific promoter derived from the α'-subunit of the soybean (*Glycine max*) β-conglycinin gene. This promoter allows high level, seed specific expression of the trait gene. It spans the 606 bp upstream of the start codon of the α' subunit of the *Glycine max* β-congylcinin storage protein. The β-conglycinin promoter sequence represents an allele of the published β-conglycinin gene (Doyle et al., (1986) J. Biol. Chem. 261:9228-9238) having differences at 27 nucleotide positions. It has been shown to maintain seed specific expression patterns in transgenic plants (Barker et al., (1988) Proc. Natl. Acad. Sci. 85:458-462 and Beachy et al., (1985) EMBO J. 4:3047-3053). The reading frame was terminated with a 3' fragment from the phaseolin gene of green bean (*Phaseolus vulgaris*). This is a 1174 bp stretch of sequences 3' of the *Phaseolus vulgaris* phaseolin gene stop codon (originated from clone described in Doyle et al., 1986).

The GmFad 2-1 open reading frame (ORF) was in a sense orientation with respect to the promoter so as to produce a gene silencing of the sense GmFad 2-1 cDNA and the endogenous GmFad 2-1 gene. This phenomenon, known as "sense suppression" is an effective method for deliberately turning off genes in plants and is described in U.S. Patent No. 5,034,323.

For maintenance and replication of the plasmid in *E. coli* the GmFad 2-1 transcriptional unit described above was cloned into plasmid pGEM-9z(-) (Promega Biotech, Madison WI, USA).

For identification of transformed soybean plants the β-glucuronidase gene (GUS) from *E. coli* was used. The cassette used consisted of the three modules; the Cauliflower Mosaic Virus 35S promoter, the β-glucuronidase gene (GUS) from *E. coli* and a 0.77 kb DNA fragment containing the gene terminator from the nopaline synthase (NOS) gene of the Ti-plasmid of *Agrobacterium tumefaciens*. The 35S promoter is a 1.4 kb promoter region from CaMV for constitutive gene expression in most plant tissues (Odell et al. (1985) Nature 303:810-812), the GUS gene a 1.85 kb fragment encoding the enzyme β-glucuronidase (Jefferson et al. (1986) PNAS USA 83:8447-8451) and the NOS terminator a portion of the 3' end of the nopaline synthase coding region (Fraley et al., (1983) PNAS US 80:4803-4807). The GUS cassette was cloned into the GmFad 2-1/pGEM-9z(-) construct and was designated pBS43.

Plasmid pBS43 was transformed into meristems of the elite soybean line A2396, by the method of particle bombardment as described in Example 1. Fertile plants were regenerated using methods well known in the art.

From the initial population of transformed plants, a plant was selected which was expressing GUS activity and which was also positive for the GmFad 2-1 gene (Event 260-05) when evaluated by PCR. Small chips were taken from a number of R1 seeds of plant 260-05 and screened for fatty acid composition. The chipped seed was then planted and germinated. Genomic DNA was extracted from the leaves of the resulting plants and cut with the restriction enzyme *Bam* HI. The blots were probed with a phaseolin probe.

From the DNA hybridization pattern it was clear that in the original transformation event the GmFad 2-1 construct had become integrated at two different loci in the soybean genome. At one locus (Locus A) the GmFad 2-1 construct was causing a silencing of the endogenous GmFad 2-1 gene, resulting in oleic acid contents as shown in Table 3. For comparison elite soybean varieties have an oleic acid content of about 20%. At locus A there were two copies of pBS43. On the DNA hybridization blot this was seen as two cosegregating bands. At the other integration locus (Locus B) the GmFad 2-1 was over-expressing, thus decreasing the oleic acid content to about 4%.

Fourth generation segregant lines (R4 plants), generated from the original transformant, were allowed to grow to maturity. R4 seeds, which contained only the silencing Locus A (e.g., G94-1) did not contain any detectable GmFad 2-1 mRNA (when measured by Northern blotting) in samples recovered 20 days after flowering. GmFad 2-2 mRNA, although reduced somewhat compared with controls, was not suppressed. Thus the GmFad 2-1 sense construct had the desired effect of preventing the expression of the GmFad 2-1 gene and thus increasing the oleic acid content of the seed. All plants homozygous for the GmFad 2-1 silencing locus had an identical Southern blot profile over a number of generations. This indicates that the insert was stable and at the same position in the genome over at least four generations.

### EXAMPLE 6

### Fatty acid analysis High Oleic trait (version 1).

A summary of the oleic acid contents found in the different generations of recombinant soybean plants and seeds is presented in Table 7. The Fatty Acid composition was determined as described in Example 2.

**TABLE 3**

| Plant ID | Generation Planted^{a} | Seed Analyzed^{a} | Bulk Oleic Acid (%) |
|---|---|---|---|
| G253 | R0:1 | R1:2 | 84.1% |
| G276 | R0:1 | R1:2 | 84.2% |
| G296 | R0:1 | R1:2 | 84.1% |
| G313 | R0:1 | R1:2 | 83.8% |
| G328 | R0:1 | R1:2 | 84.0% |
| G168-187 | R1:2 | R2:3 | 84.4% |
| G168-171 | R1:2 | R2:3 | 85.2% |
| G168-59-4 | R2:3 | R3:4 | 84.0% |
| G168-72-1 | R2:3 | R3:4 | 84.1% |
| G168-72-2 | R2:3 | R3:4 | 84.5% |
| G168-72-3 | R2:3 | R3:4 | 84.3% |
| G168-72-4 | R2:3 | R3:4 | 83.3% |

| | | | |
|---|---|---|---|
| ^{a}R0:1 indicates the seed and the plant grown from seed after selfing of the first generation transformant. R1:2 indicates the seed and the plant grown from seed after selfing of the second generation transformant. R2:3 indicates the seed and the plant grown from seed after selfing of the third generation transformant. R3:4 indicates the seed and the plant grown from seed after selfing of the fourth generation transformant. | | | |

### EXAMPLE 7

### Genetic Material Used to Produce the High Oleic trait (version 2)

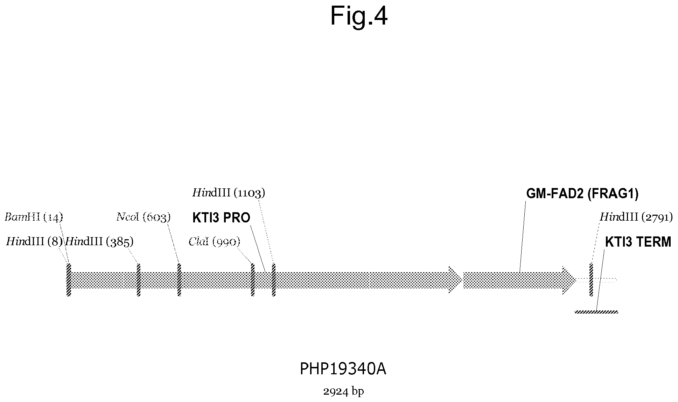

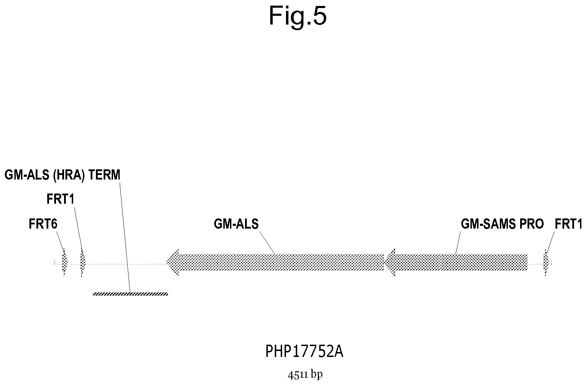

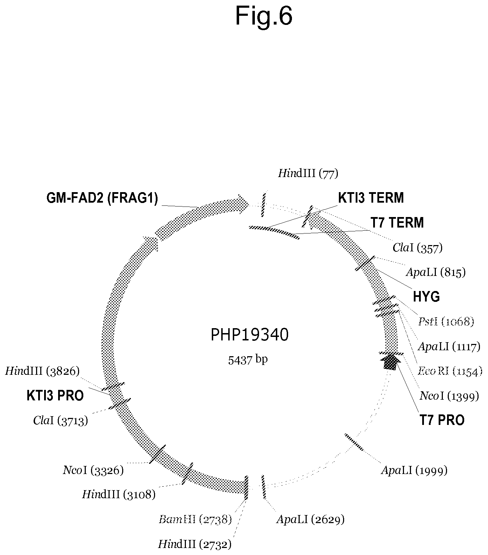

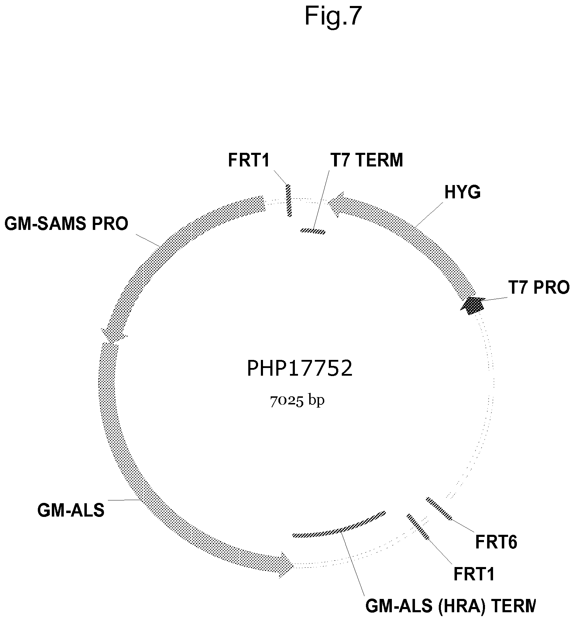

A Soybean (*Glycine max*) event was produced by particle co-bombardment as described in Example 1 with fragments PHP19340A (Fig.4; SEQ ID NO:23) and PHP17752A (Fig. 5; SEQ ID NO:24). These fragments were obtained by *Asc* I digestion from a source plasmid. Fragment PHP19340A was obtained from plasmid PHP19340 (Fig. 6; SEQ ID NO:25) and fragment PHP17752A was obtained from plasmid PHP17752 (Fig. 7; SEQ ID NO:26). The PHP19340A fragment contains a cassette with a 597 bp fragment of the soybean microsomal omega-6 desaturase gene 1 (*gm-fad2-1*) (Heppard et al., 1996, Plant Physiol. 110: 311-319).

The presence of the *gm-fad2-1* fragment in the expression cassette acts to suppress expression of the endogenous omega-6 desaturases, resulting in an increased level of oleic acid and decreased levels of palmitic, linoleic, and linolenic acid levels. Upstream of the *gm-fad2-1* fragment is the promoter region from the Kunitz trypsin inhibitor gene 3 (KTi3) (Jofuku and Goldberg, 1989, Plant Cell 1: 1079-1093; Jofuku et al., 1989, Plant Cell 1: 427-435) regulating expression of the transcript. The KTi3 promoter is highly active in soy embryos and 1000-fold less active in leaf tissue (Jofuku and Goldberg, 1989, Plant Cell 1: 1079-1093). The 3' untranslated region of the KTi3 gene (KTi3 terminator) (Jofuku and Goldberg, 1989, Plant Cell 1: 1079-1093) terminates expression from this cassette.

The PHP17752A fragment contains a cassette with a modified version of the soybean acetolactate synthase gene (*gm-hra*) encoding the GM-HRA protein with two amino acid residues modified from the endogenous enzyme and five additional amino acids at the N-terminal region of the protein derived from the translation of the soybean acetolactate synthase gene 5' untranslated region (Falco and Li, 2003, US Patent Application: 2003/0226166). The *gm-hra* gene encodes a form of acetolactate synthase, which is tolerant to the sulfonylurea class of herbicides. The GM-HRA protein is comprised of 656 amino acids and has a molecular weight of approximately 71 kDa.

The expression of the *gm-hra* gene is controlled by the 5' promoter region of the S-adenosyl-L-methionine synthetase (SAMS) gene from soybean (Falco and Li, 2003, US Patent Application: 2003/0226166). This 5' region consists of a constitutive promoter and an intron that interrupts the SAMS 5' untranslated region (Falco and Li, 2003). The terminator for the *gm-hra* gene is the endogenous soybean acetolactate synthase terminator (*als* terminator) (Falco and Li, 2003, US Patent Application: 2003/0226166).

### EXAMPLE 8

### Transformation and Selection for the Soybean High Oleic Event (version 2)

For transformation of soybean tissue, a linear portion of DNA, containing the *gm-fad2-1* gene sequence and the regulatory components necessary for expression, was excised from the plasmid PHP19340 through the use of the restriction enzyme *Asc* I and purified using agarose gel electrophoresis. A linear portion of DNA, containing the *gm-hra* gene sequences and the regulatory components necessary for expression, was excised from the plasmid PHP17752 through the use of the restriction enzyme *Asc* I and purified using agarose gel electrophoresis. The linear portion of DNA containing the *gm-fad2-1* gene is designated insert PHP19340A and is 2924 bp in size. The linear portion of DNA containing the gm-*hra* gene is designated insert PHP17752A and is 4511 bp in size. The only DNA introduced into transformation event DP-305423-1 was the DNA of the inserts described above.

The transgenic plants from event DP-305423-1 were obtained by microprojectile bombardment as described in Example 1. Embryogenic tissue samples were taken for molecular analysis to confirm the presence of the *gm-fad2-1* and *gm-hra* transgenes by Southern analysis. Plants were regenerated from tissue derived from each unique event and transferred to the greenhouse for seed production.

### EXAMPLE 9

### Southern Analysis of Plants Containing the High Oleic event version 2

*Materials and Methods:* Genomic DNA was extracted from frozen soybean leaf tissue of individual plants of the T4 and T5 generations of DP-305423-1 and of control (variety: Jack) using a standard Urea Extraction Buffer method. Genomic DNA was quantified on a spectrofluorometer using Pico Green® reagent (Molecular Probes, Invitrogen). Approximately 4 µg of DNA per sample was digested with *Hin*d III or *Nco* I. For positive control samples, approximately 3 pg (2 genome copy equivalents) of plasmid PHP19340 or PHP17752 was added to control soybean genomic DNA prior to digestion. Negative control samples consisted of unmodified soybean genomic DNA (variety: Jack). DNA fragments were separated by size using agarose gel electrophoresis.

Following agarose gel electrophoresis, the separated DNA fragments were depurinated, denatured, neutralized *in situ*, and transferred to a nylon membrane in 20x SSC buffer using the method as described for TURBOBLOTTER™ Rapid Downward Transfer System (Schleicher & Schuell). Following transfer to the membrane, the DNA was bound to the membrane by UV crosslinking.

DNA probes for *gm-fad2-1* and *gm-hra* were labeled with digoxigenin (DIG) by PCR using the PCR DIG Probe Synthesis Kit (Roche).

Labeled probes were hybridized to the target DNA on the nylon membranes for detection of the specific fragments using DIG Easy Hyb solution (Roche) essentially as described by manufacturer. Post-hybridization washes were carried out at high stringency. DIG-labeled probes hybridized to the bound fragments were detected using the CDP-Star Chemiluminescent Nucleic Acid Detection System (Roche). Blots were exposed to X-ray film at room temperature for one or more time points to detect hybridizing fragments. The fatty Acid composition of the event was determined as described in Example 2. Oleic acid levels determined in 29 different events (T1 generation) ranged from 61.5-84.6%. Oleic acid level from one event (T4-T5 generation) ranged from 72-82%.

### EXAMPLE 10

### Viscosity Measurements of Soy Protein Solutions Prepared at Bench Scale from Defatted Flakes

Rheological measurements were performed in duplicate on a combination of Anton Paar MCR-300 and MCR-301 rheometers. Each rheometer was equipped with a concentric cylinder geometry (Anton Paar CC27) having an active length of 119.2 mm, a position length of 72.5 mm, and a gap length of 40 mm. Temperature control was achieved by circulating 22 °C water from controlled-temperature baths to a Peltier sample heater that controls the temperature of the measuring cell. All measurements were carried out at 25±0.05 °C. Viscosity curves for each sample were obtained via the following 4-step procedure: (1) A 19 mL sample was loaded into the concentric cylinder cup and pre-sheared for 30 s at a shear rate of 10 s⁻¹ to erase sample loading history. (2) Immediately after the pre-shear step, the sample was allowed to equilibrate at 25 °C. for 10 minutes. (3) The sample was then subjected to a 1-100 s⁻¹ shear rate ramp during which 20 logarithmically-spaced data points were recorded at an interval of 30 s per point. (4) The sample was then immediately exposed to a downward shear rate ramp which had the same characteristics as the upward ramp, but was applied in the opposite direction (100-1 s⁻¹). The resulting viscosity versus shear rate curves were fit to a 2^{nd} log polynomial model: apparent viscosity=*a·*(shear rate )^{[*b*+*c* ln(shear rate)]}, where a, *b*, and c are curve fitting constants. *a* describes the apparent viscosity at a shear rate of1 s⁻¹ and has units of centipoise (cps). The relationship n=1+[*b*+*c* In(shear rate)] describes the non-Newtonian behavior of the material, including shear thinning (pseudoplastic) or shear thickening (dilatent) behavior, in a similar fashion to the flow behavior index of the Herschel-Bulkley and power law (Ostwald) models. The parameter b is unitless and the parameter c has the units [ln(s)]. The shear stress hysteresis area (HA) bounded by the upward and downward shear rate ramps was also recorded during each measurement.

### EXAMPLE 11

### Preparation of High Oleic and Commodity Soy Material Blends with Improved Characteristics

### Modulation of Isolate Viscosity by Mixing Commodity and High Oleic Defatted Flake Prior to First Extraction

### Isolate Prepared from a Mixture of Commodity and High Oleic Soy Flakes

2007 Crop commodity and high oleic soybeans were converted to defatted soy flakes via standard hexane extraction and controlled desolventization, The_defatted soy flakes were used to make the isolates. 10% slurries were made using the defatted flakes for each variety (commodity and high oleic). The slurries were blended to vary the amounts of high oleic and commodity material in the resultant slurry.

The two flake slurries were mixed at commodity: high oleic ratios of 80:20, 60:40 and 40:60. The blended slurries were processed to isolates as shown in the diagram below.

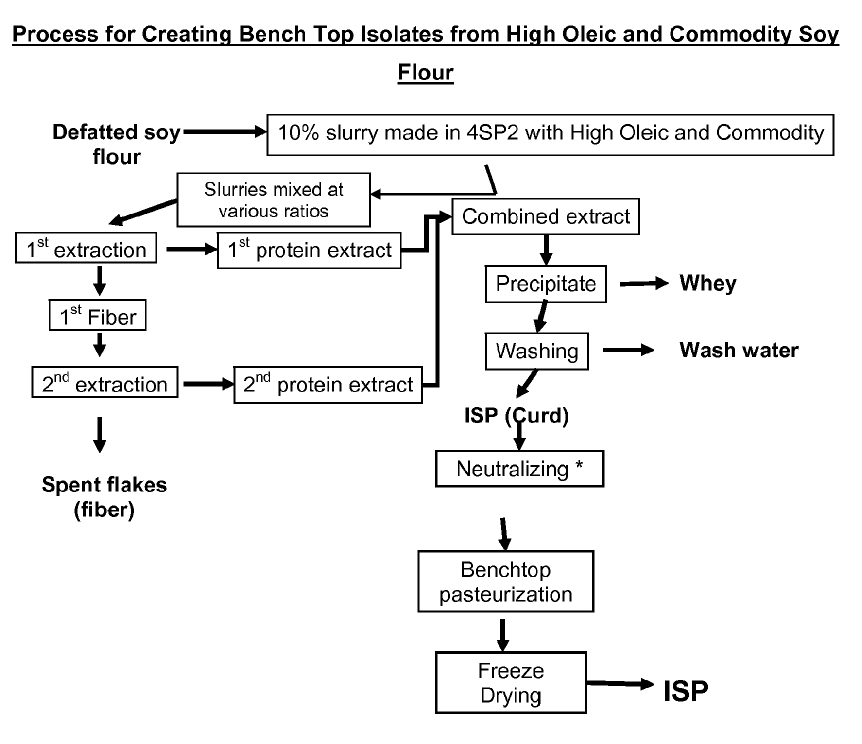

1000g of the flour mixture was dispersed in 10L tap water in a colloid mill for 15min. A defoamer (4g, Ross Chemicals Foamblast) was added to the mixture that was then heated to 90°F and circulated in the colloid mill for 15 min. The slurry (1600ml) was centrifuged at 3000rpm for 15min. The supernatant was retained and the spent flakes were resuspended in 0.96L of tap water at 90°F. The suspension was dispersed with a Silverson L4RT-A mixer at 5500rpm for 60 sec and the pH was then adjusted to 9.00 with 1N NaOH. The solution was then centrifuged at 3000rpm for 15 min. The first supernatant was combined with the second and the pH was adjusted to 4.5 with 2N HCI. The mixture was centrifuged for 15 min at 3000rpm. The supernatant was discarded and the centrifuged curd was slurried with an equal weight of tap water in the centrifuge bottle by manual shaking for two minutes. The mixture was centrifuged for 15min at 3000rpm and the supernatant was discarded.

The centrifuged solids were slurried with a sufficient amount of water to obtain a slurry containing approximately 14% solids. The solids content was determined with a CEM Labwave 9000 moisture analyzer. The mixture was then adjusted to pH 7.0 with 2N NaOH with stirring and the resulting inhomogeneous mixture was dispersed by mixing with the Silverson for 60 sec at 5500rpm. The pH was checked and adjusted to 7.0. The solids were measured and adjusted to 10% with tap water.

Soybeans with an oleic acid content and a PUFA content between that of commodity and high oleic soybeans, described as mid oleic beans, were purchased from Asoya. The beans were converted to defatted soy flake via standard hexane extraction and controlled desolventization. The defatted flake was processed using the same method described above to obtain 10% mid oleic isolate in-process slurry and the freeze-dried isolate.

In-process rheological measurements were done at 10% solids after neutralization. Rheology measurements were made on the sample within 30 min, and the remaining sample was stirred and heated to 85°C in a beaker on hot plate. This temperature was maintained for 7 minutes after which the sample was immediately cooled by immersing it in an ice bath. The sample was then frozen and freeze dried.

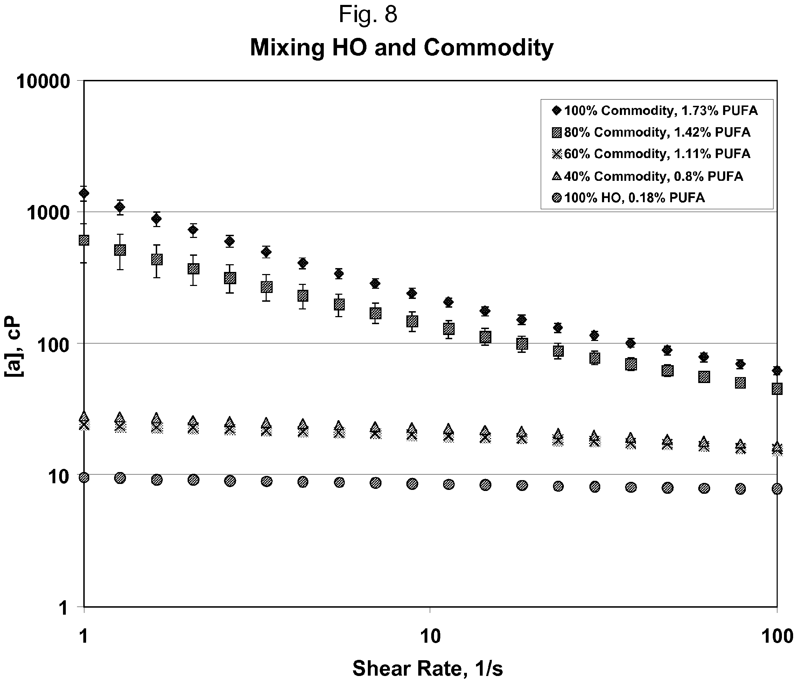

The results are shown in Table 4 and Fig. 8.

**TABLE 4**

| Effect of mixing commodity and high oleic flake on the in process viscosity, ('a' in cP at shear rate of 1/s) of isolate slurry at 10% solids content. | | | | |
|---|---|---|---|---|
| **Sample Description** | **Commodity flake %** | **high oleic flake %** | **%PUFA on flake basis** | **a, cP** |
| Commodity Control | 100% | 0% | 1.73 | 1387 |
| Com:HO::80:20 | 80% | 20% | 1.42 | 614.5 |
| Com:HO::60:40 | 60% | 40% | 1.11 | 24.2 |
| Com:HO::40:60 | 40% | 60% | 0.80 | 28.2 |
| HO control | 0% | 100% | 0.18 | 11.2 |
| Mid Oleic Control | 0% | 0% | 0.73 | 106 |

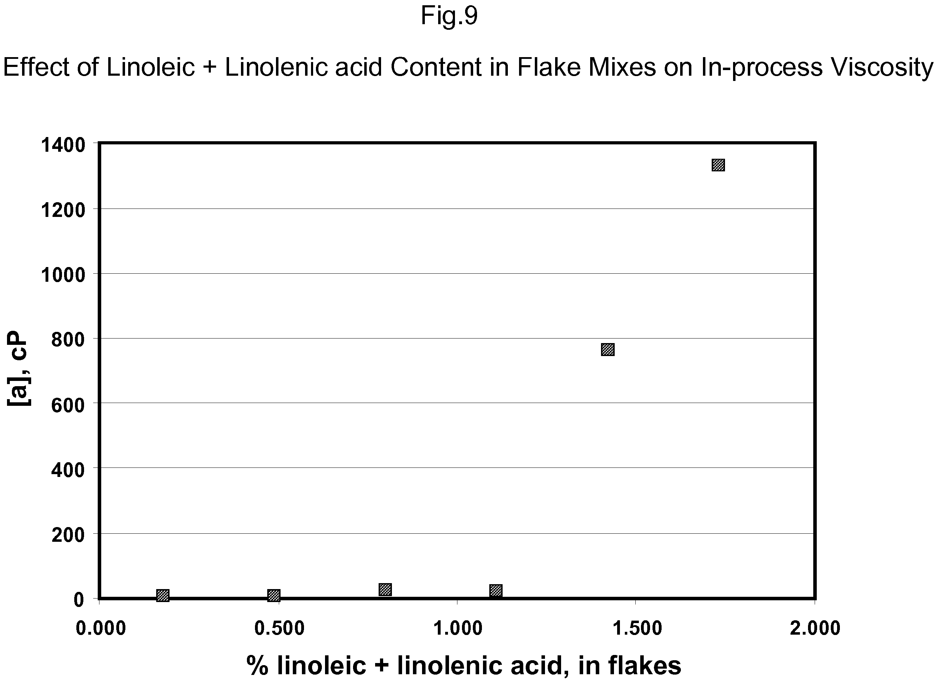

Even with inclusion of only 40% high oleic slurry, the viscosity dropped significantly, approximating the viscosity obtained with 100% high oleic slurries. This makes it possible to dry these blends at higher solids to dryer. Thus it is possible to obtain the functional and processing advantages of the more expensive 100% high oleic isolates when including certain levels of the less expensive commodity isolate. The 'a' values, i.e. viscosity at a shear rates of 1 reciprocal second were plotted as a function of linoleic and linolenic fatty acid content in the flake (calculated for the blends of the original fatty acid contents in the high oleic and commodity soy flake. The results are shown in Fig.9. From the results it can be concluded that viscosity can be modulated by changing the fatty acid profile by blending flake with different fatty acid profiles. The above results show that isolates with varying viscosities can be produced by blending flakes with different polyunsaturated fatty acid profiles without using technologies, such as hydrolysis which add to the manufacturing cost and which produce a bitter flavor of the isolate. The isolates obtained from the blended flakes may be used for various applications ranging from products, such as, but not limited to soy milk, puddings, and gels to meat analogs.

### EXAMPLE 12

### Modification of High Oleic Soy Protein Viscosity by Addition of Linoleic and Linolenic Acid Mixture to Defatted Flake Prior to First Extraction

### SoyProtein Preparation from Commodity Soy Flake

2007 Crop commodity soybeans were converted to defatted soy flakes via standard hexane extraction and controlled desolventization. The flake (1000g) was dispersed in 10L tap water in a colloid mill for 15min. A defoamer (4g, Ross Chemicals Foamblast) was added to the mixture that was then heated to 90°F and circulated in the colloid mill for 15 min. Two liter batches of the extract were centrifuged at 3000rpm for 15 minutes.

The supernatant was retained and the spent flakes were re-suspended in 1.2L of tap water at 90°F. The suspension was dispersed with a Silverson L4RT-A mixer at 5500rpm for 60 sec and the pH was then adjusted to 9.00 with 1N NaOH. The solution was then centrifuged at 3000rpm for 15 min. The first supernatant was combined with the second and the pH was adjusted to 4.5 with 2N HCI. The mixture was centrifuged for 15 min at 3000rpm. The supernatant was discarded and the centrifuged curd was slurried with an equal weight of tap water in the centrifuge bottle by manual shaking for two minutes. The mixture was centrifuged for 15min at 3000rpm and the supernatant was discarded.

The centrifuged solids were slurried with a sufficient amount of water to obtain a slurry containing approximately 14% solids. The solids content was determined with a CEM Labwave 9000 moisture analyzer. The mixture was then adjusted to pH 7.0 with 2N NaOH with stirring and the resulting inhomogeneous mixture was dispersed by mixing with the Silverson for 60 sec at 5500rpm. The pH was checked and adjusted to 7.0. The solids were measured and adjusted to 10% with tap water. Rheology measurements were made on the isolated soy protein within 30 min, and the remaining sample was stirred and heated to 85°C in a beaker on hot plate. This temperature was maintained for 7 minutes after which the sample was immediately cooled by immersing it in an ice bath. The sample was then frozen and freeze dried.

### Soy Protein Preparation from High Oleic Soy Flake

2007 Crop high oleic soy beans were converted to defatted soy flakes via standard hexane extraction and controlled desolventization.. The flakes (1000g) were dispersed in 10L tap water in a colloid mill for 15min. A defoamer (4g, Ross Chemicals Foamblast) was added to the mixture that was then heated to 90°F and circulated in the colloid mill for 15 min.

The slurry (1600ml) was centrifuged at 3000rpm for 15min. The supernatant was retained and the spent flakes were resuspended in 0.96L of tap water at 90°F. The suspension was dispersed with a Silverson L4RT-A mixer at 5500rpm for 60 sec and the pH was then adjusted to 9.00 with 1N NaOH. The solution was then centrifuged at 3000rpm for 15 min. The first supernatant was combined with the second and the pH was adjusted to 4.5 with 2N HCI. The mixture was centrifuged for 15 min at 3000rpm. The supernatant was discarded and the centrifuged curd was slurried with an equal weight of tap water in the centrifuge bottle by manual shaking for two minutes. The mixture was centrifuged for 15min at 3000rpm and the supernatant was discarded.

The centrifuged solids were slurried with a sufficient amount of water to obtain a slurry containing approximately 14% solids. The solids content was determined with a CEM Labwave 9000 moisture analyzer. The mixture was then adjusted to pH 7.0 with 2N NaOH with stirring and the resulting inhomogeneous mixture was dispersed by mixing with the Silverson for 60 sec at 5500rpm. The pH was checked and adjusted to 7.0. The solids were measured and adjusted to 10% with tap water.

Rheology measurements were made on the isolated soy protein within 30 min, and the remaining sample was stirred and heated to 85°C in a beaker on hot plate. This temperature was maintained for 7 minutes after which the sample was immediately cooled by immersing it in an ice bath. The sample was then frozen and freeze dried.

### Soy Protein Prepared from High Oleic Soy Flake Treated with Polyunsaturated Fatty Acids.

The flake (1000g) was dispersed in 10L tap water in a colloid mill for 15min. A defoamer (4g, Ross Chemicals Foamblast) was added to the mixture that was then heated to 90°F and circulated in the colloid mill for 15 min. Four 2L batches of slurry were prepared.

Linoleic acid (3.17ml) and linolenic acid (0.41 ml) were added drop wise to a sample of slurry (200ml) that was taken from one of the 2L batches. The slurry/fatty acid mixture was blended with a Silverson mixer for 60 seconds to disperse the fatty acids. The 200ml mixture was then combined with the other 1.8L and the resulting mixture was homogenized in the colloid mill for 5 min. Two aliquots of 800ml each were taken from the homogenized mixture and placed in 1L vessels that were then placed in a Hanson Dissolution Station SR8Plus. The water bath of the Hanson was set at 50°C and the overhead stirrers were set at 120rpm.

After the slurry was stirred for 30 min at 50°C, it was centrifuged at 3000rpm for 15min. The supernatant was retained and the spent flakes were resuspended in 0.96L of tap water at 90°F. The suspension was dispersed with a Silverson L4RT-A mixer at 5500rpm for 60 sec and the pH was then adjusted to 9.00 with 1N NaOH. The solution was then centrifuged at 3000rpm for 15 min. The first supernatant was combined with the second and the pH was adjusted to 4.5 with 2N HCI. The mixture was centrifuged for 15 min at 3000rpm. The supernatant was discarded and the centrifuged curd was slurried with an equal weight of tap water in the centrifuge bottle by manual shaking for two minutes. The mixture was centrifuged for 15min at 3000rpm and the supernatant was discarded.

The centrifuged solids were slurried with a sufficient amount of water to obtain a slurry containing approximately 14% solids. The solids content was determined with a CEM Labwave 9000 moisture analyzer. The mixture was then adjusted to pH 7.0 with 2N NaOH with stirring and the resulting inhomogeneous mixture was dispersed by mixing with the Silverson for 60 sec at 5500rpm. The pH was checked and adjusted to 7.0. The solids were measured and adjusted to 10% with tap water.

Rheology measurements were made on the 10% in-process protein sample within 30 min, and the remaining sample was stirred and heated to 85°C in a beaker on hot plate. This temperature was maintained for 7 minutes after which the sample was immediately cooled by immersing it in an ice bath. The sample was then frozen and freeze dried. Color measurements were made on the freeze-dried protein isolate.

This experiment was repeated with the following combinations of linoleic + linolenic acid:

| Linoleic acid + | Linolenic acid |
|---|---|
| 8.56ml | 1.11ml |
| 4.28ml | 0.554ml |
| 0.797ml | 0.103ml |
| 0.318ml | 0.041ml |
| 0.159ml | 0.021ml |
| 0.066ml | 0.008ml |
| 0ml | 0ml |

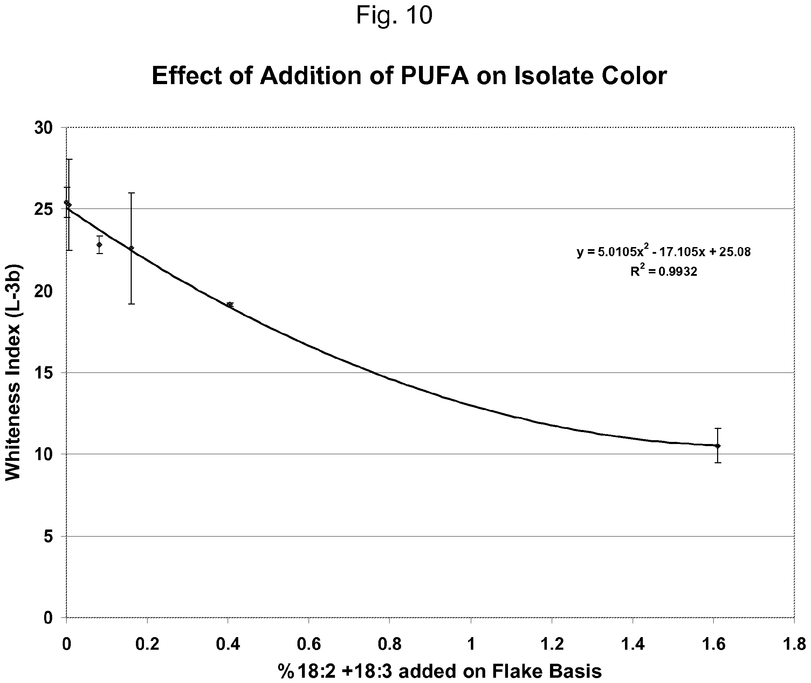

The effect of the added PUFAs on the in-process viscosity and the whiteness index of the dried isolate are shown in the attached Table 5. and Fig 10.

**TABLE 5**

| Flake (2007) | Incubation Time | Added Linoleic acid (g/100g flake) | Added Linolenic acid (g/100g flake) | Total Added Linoleic + Linolen ic Acid (g/100g flake) | Total Linoleic acid in extraction (g/100g flakes) | Total Linolenic acid in extraction (g/100g flake) | Total Linoleic + Linolenic Acid in Extraction (g/100g flake) | FAH % (flake ) | a (cP) |
|---|---|---|---|---|---|---|---|---|---|
| | | | | | | | | | |
| Cm¹ | None | 0.000 | 0.000 | 0.000 | 1.465 | 0.187 | 1.652 | 3.26 | 1903 |
| HO² | None | 0.000 | 0.000 | 0.000 | 0.120 | 0.047 | 0.167 | 2.59 | 12 |
| HO² | 30min | 0.000 | 0.000 | 0.000 | 0.120 | 0.047 | 0.167 | 2.59 | 19 |
| HO² | 30min | 3.350 | 0.500 | 3.850 | 3.470 | 0.547 | 4.017 | 2.59 | 3702 |
| HO² | 30min | 1.925 | 0.250 | 2.175 | 2.045 | 0.297 | 2.342 | 2.59 | 2631 |
| HO² | 30min | 1.430 | 0.185 | 1.615 | 1.550 | 0.232 | 1.782 | 2.59 | 3311 |
| HO² | 30min | 1.073 | 0.139 | 1.211 | 1.193 | 0.186 | 1.378 | 2.59 | 2336 |
| HO² | 30min | 0.358 | 0.046 | 0.404 | 0.478 | 0.093 | 0.571 | 2.59 | 1091 |
| HO² | 30min | 0.143 | 0.018 | 0.161 | 0.263 | 0.065 | 0.328 | 2.59 | 521 |
| HO² | 30min | 0.072 | 0.009 | 0.081 | 0.192 | 0.056 | 0.248 | 2.59 | 30 |
| HO² | 30min | 0.029 | 0.037 | 0.066 | 0.149 | 0.084 | 0.233 | 2.59 | 20 |

| | | | | | | | | | |
|---|---|---|---|---|---|---|---|---|---|
| Cm=commodity ²HO=high oleic | | | | | | | | | |

### EXAMPLE 13

### Effect of Individual Fatty Acids on High Oleic Soy Protein Isolate Viscosity

The experiment was repeated by adding 3.23g of each of the following individual fatty acids to 2L of the first extract as described in Example 12. The stearic acid was dissolved in 20ml ethanol that was added dropwise to 200ml of the slurry being blended with the Silverson. The other acids were added directly as pure liquids.
Stearic acid (3.23g)
Oleic acid (3.23g)
Linoleic acid (3.23g)
Linolenic acid (3.23g)
Linoleic acid (2.86g) + Linolenic acid (0.36g)

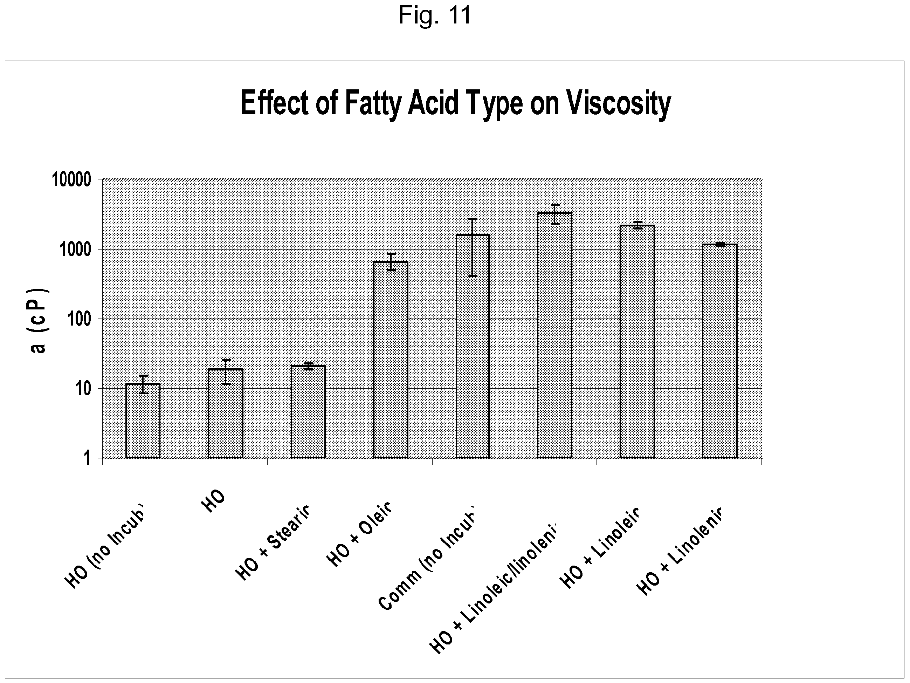

The results are shown in Table 6 and Fig.11. The saturated stearic acid has no effect on the viscosity of the isolated protein, the monounsaturated oleic has some effect and the polyunsaturated linoleic and linolenic acids have a greater effect. The mixture of linoleic and linolenic acids has a greater effect than either linoleic acid or linolenic acid has on its own.

**TABLE 6**

| **Sample** | **Mean a (cP)** | **stdev (cP)** |
|---|---|---|
| 2007 HO (w/o incubation) | 11.9 | 3.3 |
| 2007 HO | 18.8 | 7.0 |
| 2007 HO + Stearic | 20.8 | 2.0 |
| 2007 HO + Oleic | 670 | 174 |
| 2007 Comm (no Incub) | 1576 | 1174 |
| 2007 HO + Linoleic/linolenic | 3310 | 1012 |
| 2007 HO + Linoleic | 2199 | 186 |
| 2007 HO + Linolenic | 1153 | 69 |

### EXAMPLE 14

### Hunter Color Determination of Soybean Protein Products

Color measurements using the Hunter colorimeter can be made on soy protein powders, such as high oleic protein and commodity protein powders and 5% aqueous slurries. Two units of L value differences can be detected visually and one unit of Whiteness index differences can be detected. The whiteness indices between the various protein products can be compared. Whiteness index measurements of for example 5% by weight solids samples of the suspension for the isolates can be determined using a HunterLab Labscan XE colorimeter manufactured by Hunter Associates Laboratory (HunterLab, Reston, VA). For the whiteness index measurement, protein samples are dispersed on a 5% w/w basis: (5.25 g) is added to deionized water (100 mL). The results obtained using the Hunter Colorimeter are reported in units of L, a, and b. Whiteness Index is calculated from the L and b scale values using the following: Whiteness Index = L - 3b.

### EXAMPLE 15

### Gel Strength Measurements of Soy Protein Isolates

Gels can be prepared by the addition of NaCl and mixing for several minutes with additional scraping every 30 seconds. Following preparation, gels are packed into 5 mL glass vials using a disposable cartridge mini gun dispenser. Care has to be taken to eliminate any residual air bubbles. Vials are sealed with tightly crimped septum and cap. Sealed vials are either placed immediately in the refrigerator and stored for 16-24 hours (refrigerated gel) or incubated in an 80°C bath for 30 minutes, cooled for 30 minutes in a 25°C water bath prior to refrigeration for 16-24 hrs (pasteurized gel).

Gel strength is measured either on a texture Analyzer (TAXT.2i, Stable Micro Systems, UK) or an an AR-1000 Rheometer (TA Instruments). When gel strength is measured on the texture Analyzer, gels are removed from the refrigerator and warmed in a 25°C. Decapped sample vials are centered on the loading platform and a 3 mm diameter stainless cylinder punch probe is used for measurement. Gels are penetrated twice in the center of the vial to a depth of 10 mm and the data recorded using the instrument manufacturer's software. The area under the positive portion of the curve is integrated and recorded (labeled area). Gel preparation and measurements are replicated on a second day and the data averaged and recorded.

### EXAMPLE 16

### Improvement of Drying Efficiency by using High Oleic Soybeans

The blended protein products produced by the method of the invention can be fed to a pasteurizer or a dryer at higher feed solids compared to for example commodity soy protein products. This can be explained by the reduced viscosity of some of the blended soy protein products produced by the method of the invention (with 0.17% to about 1.65% PUFAS combined in the final protein product). When protein products are fed to a pasteurizer or a dryer at increased feed solids, less water has to be removed in every pound fed to the dryer resulting in decreased energy costs, and more solids can be dried per hour resulting in better capital utilization as well as higher production quantities.

### EXAMPLE 17

### Fatty Acid Analysis of Soybean Isolates

The relative amounts of the fatty acids of isolated soy protein can be determined as follows. The isolated soy protein can be extracted by the acid hydrolysis fat method (AOAC 922.06). Extracted lipid was saponified with alcoholic sodium hydroxide. The fatty acids was esterified in methanol, with boron trifluoride as a catalyst, taken up in heptane, and injected on an Agilent 5890 Gas Chromatograph equipped with a flame ionization detector and cool on-column injector. Fatty acid methyl esters were separated on a Supelco SP-2560 column (100 m x 0.25 mm ID). The column oven temperature was set to 140°C for 5 minutes, then heated at 4°C per minute to a maximum temperature of 240°C and held at that temperature to the end of the analysis. The percent of individual fatty acid methyl esters were calculated from a set of standards containing known concentrations of prepared methyl esters of selected fatty acids.

## Claims

1. A method of preparing a blended soy protein product, said method comprising blending commodity soybean defatted flakes and high oleic soybean defatted flakes and preparing a first soy protein product therefrom having a combined PUFA content of about 0.17% to about 1.65% by weight, wherein said high oleic soybean flakes are obtained from soybean seeds having an oleic acid content of at least 60% of the fatty acid moieties and wherein a slurry or solution of said first product has reduced viscosity when compared to a slurry or solution of a second soy protein product obtained from the commodity soybean defatted flakes, and prepared using the same process.

2. The method of claim 1 wherein said high oleic soybean defatted flakes comprise at least 40% of the total defatted flakes.

3. The method of claim 1 or claim 2, wherein said first product has improved drying efficiency when compared to a second soy protein product obtained from the commodity soybean defatted flakes.

4. The method of any one of claims 1 to 2, wherein said first product has an improved whiteness index when compared to a second soy protein product obtained from the commodity soybean defatted flakes.

5. The method of any one of claims 1 to 3, wherein the viscosity of the first soy protein product is reduced by at least 25%.

6. The method of any one of claims 1 to 3, wherein the drying efficiency of the first soy protein product is increased by at least 1 %.

7. The method of claim 6 wherein the whiteness index of the first soy protein product is increased by at least 2.5%.

8. The method of any one of claims 1 to 7 wherein said protein product has at least 40%, at least 65% or at least 90% protein (N x 6.25) on a moisture-free basis.

9. The method of any one of claims 1 to 8 wherein said method comprises preparing a product selected from the group consisting of: a soy protein isolate, a soy protein concentrate, soy meal, full fat flour, soymilk powder, defatted flour, soymilk, textured protein, textured flours, textured concentrates and textured isolates.

10. The method of any one of claims 1 to 7 further comprising incorporating said first soy protein product in a food, beverage or animal feed.

## Patentansprüche

1. Verfahren zur Herstellung eines gemischten Sojaproteinprodukts, wobei das Verfahren das Mischen von entfetteten Konsumwaren-Sojabohnenflocken und entfetteten Sojabohnenflocken mit hohem Ölsäuregehalt und das Herstellen eines ersten Sojaproteinprodukts daraus umfasst, das einen kombinierten PUFA-Gehalt von etwa 0,17 bis etwa 1,65 Gew.-% aufweist, wobei die Sojabohnenflocken mit hohem Ölsäuregehalt aus Sojabohnenkörnern erhalten werden, die einen hohen Ölsäuregehalt von mindestens 60 %, auf die Fettsäureanteile bezogen, aufweisen, und wobei eine Aufschlämmung oder Lösung des ersten Produkts eine reduzierte Viskosität im Vergleich mit einer Aufschlämmung oder Lösung eines zweiten Sojaproteinprodukts aufweist, das aus den entfetteten Konsumwaren-Sojabohnenflocken erhalten und unter Anwendung desselben Verfahrens hergestellt worden ist.

2. Verfahren nach Anspruch 1, wobei die entfetteten Sojabohnenflocken mit hohem Ölsäuregehalt mindestens 40 % der gesamten entfetteten Flocken umfassen.

3. Verfahren nach Anspruch 1 oder Anspruch 2, wobei das erste Produkt eine verbesserte Trocknungseffizienz im Vergleich mit einem zweiten Sojaproteinprodukt aufweist, das aus den entfetteten Konsumwaren-Sojabohnenflocken erhalten worden ist.

4. Verfahren nach einem der Ansprüche 1 bis 2, wobei das erste Produkt einen verbesserten Weißheitsindex im Vergleich mit einem zweiten Sojaproteinprodukt aufweist, das aus den entfetteten Konsumwaren-Sojabohnenflocken erhalten worden ist.

5. Verfahren nach einem der Ansprüche 1 bis 3, wobei die Viskosität des ersten Sojaproteinprodukts um mindestens 25 % reduziert ist.

6. Verfahren nach einem der Ansprüche 1 bis 3, wobei die Trocknungseffizienz des ersten Sojaproteinprodukts um mindestens 1 % erhöht ist.

7. Verfahren nach Anspruch 6, wobei der Weißheitsindex des ersten Sojaproteinprodukts um mindestens 2,5 % erhöht ist.

8. Verfahren nach einem der Ansprüche 1 bis 7, wobei das Proteinprodukt mindestens 40 %, mindestens 65 % oder mindestens 90 % Protein (N x 6,25) auf einer feuchtigkeitsfreien Basis aufweist.

9. Verfahren nach einem der Ansprüche 1 bis 8, wobei das Verfahren das Herstellen eines Produkts umfasst ausgewählt aus der Gruppe bestehend aus: einem Sojaproteinisolat, einem Sojaproteinkonzentrat, Sojamehl, Vollfettmehl, Sojamilchpulver, entfettetem Mehl, Sojamilch, texturiertem Protein, texturierten Mehlen, texturierten Konzentraten und texturierten Isolaten.

10. Verfahren nach einem der Ansprüche 1 bis 7, ferner das Integrieren des ersten Sojaproteinprodukts in ein Nahrungsmittel, Getränk oder Tierfutter umfassend.

## Revendications

1. Procédé de préparation d'un produit mélangé de protéine de soja, ledit procédé comprenant le mélange de flocons dégraissés de fèves de soja matières premières et de flocons dégraissés de fèves de soja à teneur élevée en acide oléique et la préparation d'un premier produit de protéine de soja à partir de ceux-ci ayant une teneur combinée en PUFA d'environ 0,17 % à environ 1,65 % en poids, où lesdits flocons de fèves de soja à teneur élevée en acide oléique sont obtenus à partir de fèves de soja ayant une teneur en acide oléique d'au moins 60 % des fractions acides gras et dans lequel une suspension ou une solution dudit premier produit présente une viscosité réduite lorsque comparée à une suspension ou une solution d'un second produit de protéine de soja obtenu à partir des flocons dégraissés de fèves de soja matières premières, et préparée en utilisant le même procédé.

2. Procédé selon la revendication 1 dans lequel lesdits flocons dégraissés de fèves de soja à teneur élevée en acide oléique comprennent au moins 40 % des flocons dégraissés totaux.

3. Procédé selon la revendication 1 ou la revendication 2, dans lequel ledit premier produit présente une efficacité de séchage améliorée lorsque comparé à un second produit de protéine de soja obtenu à partir des flocons dégraissés de fèves de soja matières premières.

4. Procédé selon l'une quelconque des revendications 1 à 2, dans lequel ledit premier produit présente un indice de blancheur amélioré lorsque comparé à un second produit de protéine de soja obtenu à partir des flocons dégraissés de fèves de soja matières premières.

5. Procédé selon l'une quelconque des revendications 1 à 3, dans lequel la viscosité du premier produit de protéine de soja est réduite d'au moins 25 %.

6. Procédé selon l'une quelconque des revendications 1 à 3, dans lequel l'efficacité de séchage du premier produit de protéine de soja est accrue d'au moins 1 %.

7. Procédé selon la revendication 6 dans lequel l'indice de blancheur du premier produit de protéine de soja est accru d'au moins 2,5 %.

8. Procédé selon l'une quelconque des revendications 1 à 7 dans lequel ledit produit protéique présente au moins 40 %, au moins 65 % ou au moins 90 % de protéine (N x 6,25) sur une base sans humidité.

9. Procédé selon l'une quelconque des revendications 1 à 8 dans lequel ledit procédé comprend la préparation d'un produit sélectionné dans le groupe constitué: d'un isolat de protéine de soja, d'un concentré de protéine de soja, d'une moulée de soja, d'une farine de soja à teneur complète en matière grasse, d'une poudre de lait de soja, d'une farine dégraissée, d'un lait de soja, d'une protéine texturée, de farines texturées, de concentrés texturés et d'isolats texturés.

10. Procédé selon l'une quelconque des revendications 1 à 7 comprenant en outre l'incorporation dudit premier produit de protéine de soja à un aliment, une boisson ou un aliment pour animaux.
